# EUROPÄISCHE PATENTSCHRIFT

(11) **EP 0 547 517 B1**
(45) Veröffentlichungstag und Bekanntmachung des Hinweises auf die Patenterteilung: **17.05.1995**
(21) Anmeldenummer: 92121126.4
(22) Anmeldetag: 11.12.1992
(51) Int. Cl.: C07D 213/55, C07D 401/12, C07D 401/06, C07F 7/10, A61K 31/44

(54) **Neue Pyridylderivate, diese Verbindungen enthaltende Arzneimittel und Verfahren zu ihrer Herstellung**
Pyridyl derivatives, medicines containing these compounds and processes for their preparation
Dérivés de pyridines, médicaments contenant ces composés et leurs procédés de préparation

(30) Priorität: 14.12.1991 DE 4141377; 18.05.1992 DE 4216364; 21.05.1992 DE 4216829
(43) Veröffentlichungstag der Anmeldung: 23.06.1993
(73) Patentinhaber: Dr. Karl Thomae GmbH, D-88397 Biberach (DE)
(72) Erfinder: Soyka, Rainer, Dr., Dipl.-Chem., W-7950 Biberach 1 (DE); Müller, Thomas, Dr, Dipl.-Chem., W-7950 Biberach 1 (DE); Weisenberger, Johannes, Dr., Dipl.-Chem., W-7950 Biberach 1 (DE)

(56) Entgegenhaltungen:
- EP-A- 0 397 044
- EP-A- 0 405 391

## Beschreibung

In der EP-A-0 397 044 werden bereits Arylsulfonamide beschrieben, welche antithrombotische sowie durch Thromboxan vermittelte Wirkungen aufweisen und sowohl Thromboxanantagonisten als auch Thromboxansynthesehemmer darstellen, wobei diese Verbindungen auch eine Wirkung auf die PGE₂-Produktion besitzen.

Gegenstand der vorliegenden Erfindung sind neue Pyridylderivate der allgemeinen Formel 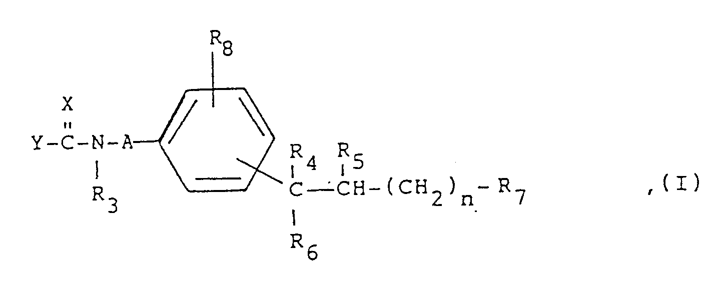
in der
n die Zahl 2, 3, 4 oder 5,
A eine Kohlenstoff-Stickstoff-Bindung oder eine gegebenenfalls durch eine oder zwei Alkylgruppen substituierte geradkettige Alkylengruppe mit 1 bis 4 Kohlenstoffatomen,
X eine Nitromethylengruppe, eine gegebenenfalls durch eine R₉-Gruppe substituierte Cyanomethylengruppe oder eine Gruppe der Formel =N-R₁₀, wobei R₉ mit Ausnahme der Tetrazolylgruppe die für R₇ nachstehend erwähnten Bedeutungen besitzt und R₁₀ eine Cyano-, Alkansulfonyl-, Phenylsulfonyl-, Phenylalkansulfonyl-, Aminosulfonyl-, Alkylaminosulfonyl-, Dialkylaminosulfonyl-, Phenylcarbonyl-, Aminocarbonyl-, Alkylaminocarbonyl- oder Dialkylaminocarbonylgruppe darstellt,
Y eine Alkoxy-, Phenoxy-, Alkylthio- oder Phenylthiogruppe oder eine Gruppe der Formel -R₁NR₂, wobei R₁ ein Wasserstoffatom, eine verzweigte oder unverzweigte Alkylgruppe mit 1 bis 10 Kohlenstoffatomen, die in 2-, 3- oder 4-Stellung durch eine Hydroxy-, Amino-, Alkylamino- oder Dialkylaminogruppe substituiert sein kann, eine Alkylgruppe mit 1 bis 4 Kohlenstoffatomen, die durch eine Phenyl- oder Pyridylgruppe substituiert ist und zusätzlich in 2-, 3- oder 4-Stellung durch eine Hydroxygruppe substituiert sein kann, eine Cycloalkylgruppe mit 3 oder 4 Kohlenstoffatomen, eine Cycloalkylgruppe mit 5 bis 8 Kohlenstoffatomen, in der eine Ethylenbrücke durch eine o-Phenylengruppe ersetzt sein kann, eine gegebenenfalls durch 1, 2 oder 3 Alkylgruppen substituierte Bicycloalkylgruppe mit 6 bis 8 Kohlenstoffatomen, eine Adamantyl-, Alkoxy- oder Trimethylsilylalkylgruppe,
R₂ ein Wasserstoffatom oder eine geradkettige Alkylgruppe oder
R₁ und R₂ zusammen mit dem dazwischenliegenden Stickstoffatom eine cyclische Alkyleniminogruppe mit 4 bis 6 Kohlenstoffatomen, die durch ein oder zwei Alkylgruppen oder durch eine Phenylgruppe substituiert sein kann und in der zusätzlich eine Ethylenbrücke in 3,4-Stellung durch eine o-Phenylengruppe ersetzt sein kann, eine Morpholinogruppe oder eine gegebenenfalls in 4-Stellung durch eine Alkylgruppe mit 1 bis 3 Kohlenstoffatomen oder durch eine Phenylgruppe substituierte Piperazinogruppe,
R₃ ein Wasserstoffatom oder eine Alkylgruppe mit einem bis drei Kohlenstoffatomen,
R₄ und R₅ je ein Wasserstoffatom oder zusammen eine Kohlenstoff-Kohlenstoff-Bindung,
R₆ eine gegebenenfalls in 3- oder 4-Position durch eine Alkylgruppe substituierte Pyridylgruppe,
R₇ eine Cyano-, Tetrazolyl-, Aminocarbonyl-, Alkylaminocarbonyl- oder Dialkylaminocarbonylgruppe, eine in-vivo metabolisch in eine Carboxygruppe überführbare Gruppe oder auch, wenn Y eine R₁NR₂-Gruppe darstellt, eine Carboxygruppe,
R₈ ein Wasserstoff-, Fluor-, Chlor-, Brom- oder Jodatom, eine Alkyl-, Alkoxy- oder Trifluormethylgruppe bedeuten,
wobei alle vorstehend erwähnten Alkyl- und Alkoxyteile, sofern nichts anderes erwähnt wurde, ein bis drei Kohlenstoffatome enthalten können,
sowie alle vorstehend erwähnten Phenylkerne, sofern nichts anderes erwähnt wurde, durch ein Fluor-, Chlor- oder Bromatom, durch eine Alkyl-, Hydroxy-, Alkoxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituiert sein können und die Substituenten gleich oder verschieden sein können,
deren Enantiomere, deren cis- und trans-Isomere, sofern R₄ und R₅ zusammen eine Kohlenstoff-Kohlenstoff-Bindung darstellen, und deren Salze.

Unter dem vorstehend erwähnten Begriff "eine Gruppe, die in-vivo metabolisch in eine Carboxygruppe umgewandelt wird," sind beispielsweise deren Ester der Formeln

- CO - OR',

- CO - O - (HCR'') - O - CO - R''' und

- CO - O - (HCR'') - O - CO - OR'''

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen.

Die Verbindungen der obigen allgemeinen Formel I, in der Y eine Alkoxy-, Phenoxy-, Alkylthio- oder Phenylthiogruppe darstellt, stellen Zwischenprodukte zur Herstellung der Verbindungen der allgemeinen Formel I, in der Y eine R₁NR₂-Gruppe darstellt, und die Verbindungen der obigen allgemeinen Formel I, in der R₇ eine Cyanogruppe darstellt, stellen Zwischenprodukte zur Herstellung von Verbindungen der allgemeinen Formel I, in der R₇ eine Tetrazolylgruppe darstellt, dar.

Die Verbindungen der allgemeinen Formel I, in der Y eine R₁NR₂-Gruppe darstellt, weisen insbesondere antithrombotische Wirkungen auf, außerdem stellen die neuen Verbindungen gleichzeitig Thromboxanantagonisten (TRA) und Thromboxansynthesehemmer (TSH) dar und inhibieren somit auch die durch Thromboxan vermittelten Wirkungen. Ferner weisen diese auch eine Wirkung auf die PGE₂-Produktion in der Lunge und auf die PGD₂-, PGE₂- und PGF_{2α}-Produktion in Humanthrombozyten auf.

Gegenstand der vorliegenden Erfindung sind somit die neuen Zwischenprodukte der obigen allgemeinen Formel I, deren Enantiomere, deren cis- und trans-Isomere, sofern R₄ und R₅ zusammen eine Kohlenstoff-Kohlenstoff-Bindung darstellen, und deren Salze sowie
die neuen Verbindungen der obigen Formel I, welche wertvolle pharmakologische Eigenschaften aufweisen, deren Salze mit anorganischen oder organischen Säuren oder Basen, insbesondere für die pharmazeutische Verwendung deren physiologisch verträgliche Salze, diese Verbindungen enthaltende Arzneimittel und Verfahren zu ihrer Herstellung.

Für die bei der Definition der Reste eingangs erwähnten Bedeutungen kommt beispielsweise für
für A die der Methylen-, Ethylen-, n-Propylen-, n-Butylen-, α-Methyl-ethylen-, α-Methyl-n-propylen-, α-Ethyl-n-propylen-, α-n-Propyl-n-propylen-, α,α-Dimethyl-n-propylen-, α,α-Diethyl-n-propylen-, β-Methyl-n-propylen-, γ-Methyl-n-propylen-, α-Methyl-n-butylen- oder α,α-Dimethyl-n-butylengruppe, wobei die Indices auf die Phenylgruppe bezogen sind,
für R₁ die des Wasserstoffatoms-, der Methyl-, Ethyl-, n-Propyl-, Isopropyl, n-Butyl-, Isobutyl-, tert.Butyl-, 1,1,3,3,-Tetramethylbutyl-, n-Pentyl-, Neopentyl-, n-Hexyl-, n-Heptyl-, n-Octyl-, n-Nonyl-, n-Decyl-, Benzyl-, 2-Phenylethyl-, 3-Phenylpropyl-, Pyridylmethyl-, 2-Pyridylethyl-, 3-Pyridylpropyl-, 2-Hydroxy-2-phenylethyl-, 2-Hydroxy-1-methyl-2-phenylethyl-, 2-Hydroxy-1,1-dimethylethyl-, Cyclopropyl-, Cyclobutyl-, Cyclopentyl-, Cyclohexyl-, Cycloheptyl-, Cyclooctyl-, Indan-1-yl-, Indan-2-yl-, 1,2,3,4-Tetrahydronaphth-1-yl-, 1,2,3,4-Tetrahydronaphth-2-yl-, 2-Hydroxyethyl-, 3-Hydroxy-n-propyl-, 4-Hydroxy-n-butyl-, 2-Hydroxyisopropyl-, Hydroxy-tert.butyl-, Exo-norbornyl-, Endo-norbornyl-, 1-Adamantyl-, 2-Adamantyl-, Methoxy-, Ethoxy-, n-Propoxy-, Isopropoxy-, 2-Amino-ethyl-, 3-Amino-propyl-, 4-Amino-butyl-, 2-Methylamino-ethyl-, 3-Methylamino-propyl-, 4-Methylamino-butyl-, 2-Ethylamino-ethyl-, 3-Ethylamino-propyl-, 4-Ethylamino-butyl-, 2-n-Propylamino-ethyl-, 3-n-Propylamino-propyl-, 4-n-Propylamino-butyl-, 2-Isopropylamino-ethyl-, 3-Isopropylamino-propyl-, 4-Isopropylamino-butyl-, 2-Dimethylamino-ethyl-, 3-Dimethylamino-propyl-, 4-Dimethylamino-butyl-, 2-Diethylamino-ethyl-, 3-Diethylamino-propyl-, 4-Diethylamino-butyl-, 2-Di-n-propylamino-ethyl-, 3-Di-n-propylamino-propyl-, 4-Di-n-propylamino-butyl-, Trimethylsilylmethyl-, 2-Trimethylsilylethyl- oder 3-Trimethylsilylpropylgruppe,
für R₂ die des Wasserstoffs, der Methyl-, Ethyl-, n-Propyl- oder Isopropylgruppe,
für R₁ und R₂ zusammen mit dem dazwischenliegenden Stickstoffatom die der Pyrrolidino-, Piperidino-, Hexamethylenimino-, 3-Methyl-piperidino-, 3,3-Dimethyl-piperidino-, 4-Phenyl-piperidino-, Morpholino-, Piperazino-, N-Methyl-piperazino-, N-Ethyl-piperazino-, N-propyl-piperazino-, N-Phenyl-piperazino-, Isoindolin-2-yl-, 1,2,3,4-Tetrahydroisochinolin-2-yl- oder 1,3,4,5-Tetrahydro-2H-benzazepin-2-yl- oder 1,2,4,5-Tetrahydro-3H-benzazepin-3-yl,
für R₃ die des Wasserstoffatoms, der Methyl-, Ethyl-, n-Propyl- oder Isopropylgruppe,
für R₆ die der 3-Methylpyridyl-(2)-, 3-Ethylpyridyl-(2)-, 3-n-Propylpyridyl-(2)-, 3-Isopropylpyridyl-(2)-, 4-Methylpyridyl-(2)-, 4-Ethylpyridyl-(2)-, 4-n-Propylpyridyl-(2)-, 4-Isopropylpyridyl-(2)-, 5-Methylpyridyl-(2)-, 5-Ethylpyridyl-(2)-, 5-n-Propylpyridyl-(2)-, 5-Isopropylpyridyl-(2)-, 4-Methylpyridyl-(3)-, 4-Ethylpyridyl-(3)-, 4-n-Propylpyridyl-(3)-, 4-Isopropylpyridyl-(3)-, 5-Methylpyridyl-(3)-, 5-Ethylpyridyl-(3)-, 5-n-Propylpyridyl-(3)-, 5-Isopropylpyridyl-(3)-, 3-Methylpyridyl-(4)-, 3-Ethylpyridyl-(4)-, 3-n-Propylpyridyl-(4)-, 3-Isopropylpyridyl-(4)-,
für R₇ die der Cyano-, 1H-Tetrazolyl-, 2H-Tetrazolyl-, Hydroxycarbonyl-, Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, Isoamyloxycarbonyl-, n-Hexyloxycarbonyl-, Cyclopentyloxycarbonyl-, Cyclohexyloxycarbonyl-, Benzyloxycarbonyl-, 1-Phenylethyloxycarbonyl-, 2-Phenylethyloxycarbonyl-, 3-Phenylpropyloxycarbonyl-, Methoxymethoxycarbonyl-, Cinnamyloxycarbonyl-, Acetoxymethoxycarbonyl-, Propionyloxymethoxycarbonyl-, n-Butyryloxymethoxycarbonyl-, Isobutyryloxymethoxycarbonyl-, n-Pentanoyloxymethoxycarbonyl-, Isopentanoyloxymethoxycarbonyl-, Pivaloyloxymethoxycarbonyl-, n-Hexanoyloxymethoxycarbonyl-, Cyclopentanoyloxymethoxycarbonyl-, Cyclohexanoyloxymethoxycarbonyl-, Phenylacetoxymethoxycarbonyl-, 1-Phenylpropionyloxymethoxycarbonyl-, 2-Phenylpropionyloxymethoxycarbonyl-, 3-Phenylbutyryloxymethoxycarbonyl-, Benzoyloxymethoxycarbonyl-, 1-Acetoxyethoxycarbonyl-, 1-Propionyloxyethoxycarbonyl-, 1-n-Butyryloxyethoxycarbonyl-, 1-Isobutyryloxyethoxycarbonyl-, 1-n-Pentanoyloxyethoxycarbonyl-, 1-Isopentanoyloxyethoxycarbonyl-, 1-Pivaloyloxyethoxycarbonyl-, 1-n-Hexanoyloxyethoxycarbonyl-, 1-Cyclopentanoyloxyethoxycarbonyl-, 1-Cyclohexanoyloxyethoxycarbonyl-, 1-Phenylacetoxyethozycarbonyl-, 1-(1-Phenylpropionyloxy)-ethoxycarbonyl-, 1-(2-Phenylpropionyloxy)-ethoxycarbonyl-, 1-(3-Phenylbutyryloxy)-ethoxycarbonyl-, 1-Benzoyloxyethoxycarbonyl-, Methoxycarbonyloxymethoxycarbonyl-, Ethoxycarbonyloxymethoxycarbonyl-, n-Propyloxycarbonyloxymethoxycarbonyl-, Isopropyloxycarbonyloxymethoxycarbonyl-, n-Butyloxycarbonyloxymethoxycarbonyl-, Isobutyloxycarbonyloxymethoxycarbonyl-, tert.Butyloxycarbonyloxymethoxycarbonyl-, n-Pentyloxycarbonyloxymethoxycarbonyl-, Isoamyloxycarbonyloxymethoxycarbonyl-, n-Hexyloxycarbonyloxymethoxycarbonyl-, Cyclopentyloxycarbonyloxymethoxycarbonyl-, Cyclohexyloxycarbonyloxymethoxycarbonyl-, Benzyloxycarbonyloxymethoxycarbonyl-, 1-Phenylethoxycarbonyloxymethoxycarbonyl-, 2-Phenylethoxycarbonyloxymethoxycarbonyl-, 3-Phenylpropyloxycarbonyloxymethoxycarbonyl-, Cinnamyloxycarbonyloxymethoxycarbonyl-, 1-(Methoxycarbonyloxy)-ethoxycarbonyl-, 1-(Ethoxycarbonyloxy)-ethoxycarbonyl-, 1-(n-Propyloxycarbonyloxy)-ethoxycarbonyl-, 1-(Isopropyloxycarbonyloxy)-ethoxycarbonyl-, 1-(n-Butyloxycarbonyloxy)-ethoxycarbonyl-, 1-(Isobutyloxycarbonyloxy)-ethoxycarbonyl-, 1-(tert.Butyloxycarbonyloxy)-ethoxycarbonyl-, 1-(n-Pentyloxycarbonyloxy)-ethoxycarbonyl-, 1-(Isoamyloxycarbonyloxy)-ethoxycarbonyl-, 1-(n-Hexyloxycarbonyloxy)-ethoxycarbonyl-, 1-(Cyclopentyloxycarbonyloxy)-ethoxycarbonyl-, 1-(Cyclohexyloxycarbonyloxy)-ethoxycarbonyl-, Cyclopentylcarbonyloxymethoxycarbonyl-, (1,3-Dioxa-2-oxo-4-methyl-cyclopenten-5-yl)-methoxycarbonyl-, 1-(Benzyloxycarbonyloxy)-ethoxycarbonyl-, 1-(1-Phenylethoxycarbonyloxy)-ethoxycarbonyl-, 1-(2-Phenylethoxycarbonyloxy)-ethoxycarbonyl-, 1-(3-Phenylpropyloxycarbonyloxy)-ethoxycarbonyl-, 1-(Cinnamyloxycarbonyloxy)-ethoxycarbonyl-, Aminocarbonyl-, Methylaminocarbonyl-, Ethylaminocarbonyl-, n-Propylaminocarbonyl-, Isopropylaminocarbonyl-, Dimethylaminocarbonyl-, Diethylaminocarbonyl-, Di-n-propylaminocarbonyl- oder Diisopropylaminocarbonylgruppe,
für R₈ die des Wasserstoff-, Fluor-, Chlor-, Brom- oder Jodatoms, der Methyl-, Ethyl-, n-Propyl-, Isopropyl-, Methoxy-, Ethoxy-, n-Propoxy-, Isopropoxy- oder Trifluormethylgruppe,
für R₉ mit Ausnahme der Tetrazolylgruppe die vorstehend für R₇ erwähnten Bedeutungen und
für R₁₀ die der Cyano-, Methansulfonyl-, Ethansulfonyl-, Propansulfonyl-, Isopropansulfonyl-, Phenylsulfonyl-, Phenylmethansulfonyl-, 2-Phenylethansulfonyl-, 3-Phenylpropansulfonyl-, Aminosulfonyl-, Methylaminosulfonyl-, Ethylaminosulfonyl-, Isopropylaminosulfonyl-, Dimethylaminosulfonyl-, Diethylaminosulfonyl-, Di-n-propylaminosulfonyl-, N-Ethyl-methylaminosulfonyl-, Phenylcarbonyl-, Aminocarbonyl-, Methylaminocarbonyl-, Ethylaminocarbonyl-, Isopropylaminocarbonyl-, Dimethylaminocarbonyl-, Diethylaminocarbonyl-, Di-n-propylaminocarbonyl- oder N-Ethyl-methylaminocarbonylgruppe in Betracht.

Bevorzugte Verbindungen der obigen Formel I sind jedoch diejenigen, in der
n die Zahl 2, 3, 4 oder 5,
A eine Bindung oder eine Ethylengruppe,
X eine Nitromethylengruppe, eine gegebenenfalls durch eine R₉-Gruppe substituierte Cyanomethylengruppe oder eine Gruppe der Formel =N-R₁₀, wobei R₉ mit Ausnahme der Tetrazolylgruppe die für R₇ nachstehend erwähnten Bedeutungen besitzt und R₁₀ eine Cyanogruppe, eine Phenylsulfonyl- oder Alkansulfonylgruppe darstellt,
Y eine Phenoxy- oder Methylthiogruppe oder eine R₁NR₂-Gruppe, wobei R₁ ein Wasserstoffatom, eine geradkettige oder verzweigte Alkylgruppe mit 1 bis 8 Kohlenstoffatomen, die in 2-, 3- oder 4-Stellung durch eine Hydroxy- oder Dimethylaminogruppe substituiert sein kann, eine Alkylgruppe mit 1 bis 4 Kohlenstoffatomen, die durch eine Phenyl- oder Pyridylgruppe substituiert ist und zusätzlich in 2-, 3- oder 4-Stellung durch eine Hydroxygruppe substituiert sein kann, eine Cycloalkylgruppe mit 3 bis 8 Kohlenstoffatomen, eine Methoxy-, Trimethylsilylmethyl-, Indan-2-yl- oder eine gegebenenfalls durch 1, 2 oder 3 Alkylgruppen substituierte Bicycloheptylgruppe und
R₂ ein Wasserstoffatom oder eine Methylgruppe oder
R₁ und R₂ zusammen mit dem dazwischenliegenden Stickstoffatom eine Piperidinogruppe, die durch eine oder zwei Methylgruppen oder durch eine Phenylgruppe substituiert sein kann und in der zusätzlich eine Ethylenbrücke in 3,4-Stellung durch eine o-Phenylengruppe ersetzt sein kann, eine Morpholinogruppe oder eine in 4-Stellung durch eine Phenylgruppe substituierte Piperazinogruppe darstellen,
R₃ ein Wasserstoffatom oder eine Methylgruppe,
R₄ und R₅ jeweils ein Wasserstoffatom oder zusammen eine weitere Kohlenstoff-Kohlenstoff-Bindung,
R₆ eine 3-Pyridyl- oder 4-Pyridylgruppe und
R₇ eine Cyano-, Carboxy-, Tetrazolyl-, Alkoxycarbonyl-, Aminocarbonyl-, Alkylaminocarbonyl- oder Dialkylaminocarbonylgruppe mit jeweils 1 bis 3 Kohlenstoffatomen in den Alkoxy- und Alkylteilen,
R₈ ein Wasserstoff-, Fluor-, Chlor- oder Bromatom, eine Alkyl-, Alkoxy- oder Trifluormethylgruppe bedeuten, wobei alle vorstehend erwähnten Alkyl- und Alkoxyteile, sofern nichts anderes erwähnt wurde, ein bis drei Kohlenstoffatome enthalten können, bedeuten, deren Enantiomere, deren cis- und trans-Isomere, sofern R₄ und R₅ zusammen eine Kohlenstoff-Kohlenstoff-Bindung darstellen, und deren Salze.

Besonders bevorzugt sind jedoch diejenigen Verbindungen der Formel I, in der
n die Zahl 3,
A eine Bindung oder eine Ethylengruppe,
X eine Gruppe der Formel =N-R₁₀, wobei R₁₀ eine Cyano- oder Phenylsulfonylgruppe darstellt, oder eine Dicyanomethylengruppe,
Y eine R₁NR₂-Gruppe, wobei R₁ eine geradkettige oder verzweigte Alkylgruppe mit 1 bis 8 Kohlenstoffatomen, eine Cycloalkylgruppe mit 3 bis 8 Kohlenstoffatomen oder eine Exo-norbornyl-(2)-gruppe und
R₂ ein Wasserstoffatom,
R₃ ein Wasserstoffatom,
R₄ und R₅ jeweils ein Wasserstoffatom oder zusammen eine Kohlenstoff-Kohlenstoff-Bindung,
R₆ eine 3-Pyridylgruppe und
R₇ eine Carboxy- oder Alkoxycarbonylgruppe mit insgesamt 2 bis 4 Kohlenstoffatomen,
R₈ ein Wasserstoff-, Chlor- oder Bromatom, eine Methyl- oder Trifluormethylgruppe bedeuten, deren Enantiomere, deren cis- und trans-Isomere und deren Salze.

Erfindungsgemäß erhält man die neuen Verbindungen nach folgenden Verfahren:
a) Zur Herstellung von Verbindungen der allgemeinen Formel I, in der Y eine Alkoxy-, Phenoxy-, Alkylthio- oder Phenylthiogruppe darstellt:
   Umsetzung einer Verbindung der allgemeinen Formel 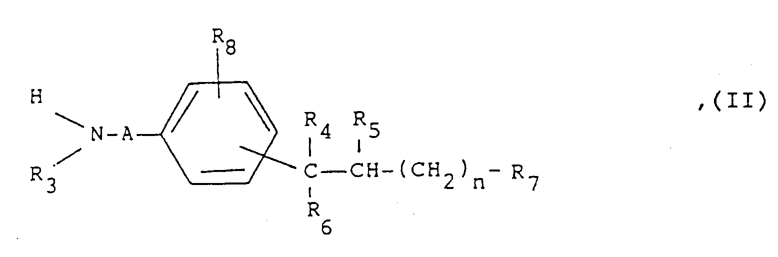 in der
   A, n und R₃ bis R₈ wie eingangs definiert sind, mit einer Verbindung der allgemeinen Formel

   (Y')₂C=X ,(III)

   in der
   X wie eingangs definiert ist und
   Y' eine Alkoxy-, Phenoxy-, Alkylthio- oder Phenylthiogruppe darstellt.
   Die Umsetzung wird vorzugsweise in einem Lösungsmittel wie Methanol, Äthanol, Isopropanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50°C, vorzugsweise jedoch bei Raumtemperatur, durchgeführt.
b) Zur Herstellung von Verbindungen der allgemeinen Formel I, in der Y eine R₁NR₂-Gruppe darstellt:
   Umsetzung einer Verbindung der allgemeinen Formel 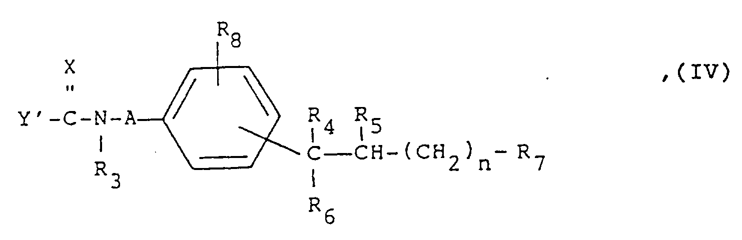 in der
   A, X, n und R₃ bis R₈ wie eingangs definiert sind und
   Y' eine Alkoxy-, Phenoxy-, Alkylthio- oder Phenylthiogruppe darstellt, mit einem Amin der allgemeinen Formel 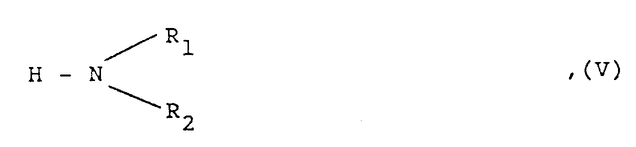 in der
   R₁ und R₂ wie eingangs definiert sind.
   Die Umsetzung wird vorzugsweise in einem Lösungsmittel wie Ethanol, Isopropanol, Tetrahydrofuran, Dioxan oder Benzol oder in einem Überschuß des eingesetzten Amins der allgemeinen Formel V gegebenenfalls in einem Druckgefäß und gegebenenfalls in Gegenwart einer Base wie Natriumcarbonat, Kaliumcarbonat, Triethylamin oder Pyridin bei Temperaturen zwischen 0 und 125°C, vorzugsweise bei Temperaturen zwischen 50 und 100°C, durchgeführt.
c) Zur Herstellung von Verbindungen der allgemeinen Formel I, in der Y eine R₁NR₂-Gruppe und R₇ eine Carboxygruppe darstellt:
   Abspaltung eines Schutzrestes von einer Verbindung der allgemeinen Formel 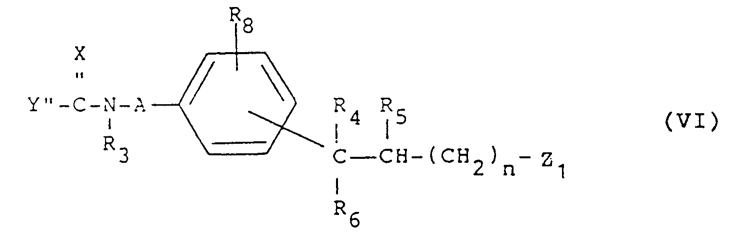 in der
   R₃ bis R₆, R₈, A, X und n wie eingangs definiert sind, Y'' eine R₁NR₂-Gruppe, wobei R₁ und R₂ wie eingangs definiert sind, und
   Z₁ eine mittels Hydrolyse, Thermolyse oder Hydrogenolyse in eine Carboxygruppe überführbare Gruppe darstellt.
   Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, Äthergruppen wie die Methoxy-, Ethoxy-, tert.Butoxy- oder Benzyloxygruppe oder Lactone und
   als thermolytisch abspaltbare Gruppen beispielsweise Ester mit tertiären Alkoholen, z.B. der tert.Butylester, und als hydrogenolytisch abspaltbare Gruppen beispielsweise Aralkylgruppen, z.B. die Benzylgruppe, in Betracht.
   Die Hydrolyse wird zweckmäßigerweise entweder in Gegenwart einer Säure wie Salzsäure, Schwefelsäure, Phosphorsäure oder Trichloresslgsäure oder in Gegenwart einer Base wie Natriumhydroxid oder Kaliumhydroxid in einem geeigneten Lösungsmittel wie Wasser, Wasser/Methanol, Äthanol, Wasser/ Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.
   Enthält beispielsweise eine Verbindung der Formel VI eine Nitril- oder Aminocarbonylgruppe, so können diese Gruppen vorzugsweise mittels 100%iger Phosphorsäure bei Temperaturen zwischen 100 und 180°C, vorzugsweise bei Temperaturen zwischen 120 und 160°C, oder auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.
   Enthält beispielsweise eine Verbindung der Formel VI eine Säureamidgruppe wie die Diethylaminocarbonyl- oder Piperidinocarbonylgruppe, so kann diese Gruppe vorzugsweise hydrolytisch in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, in die Carboxygruppe übergeführt werden.
   Enthält beispielsweise eine Verbindung der Formel VI die tert.Butyloxycarbonylgruppe, so kann die tert.Butylgruppe auch thermisch gegebenenfalls in einem inerten Lösungsmittel wie Methlenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100°C, abgespalten werden.
   Enthält beispielsweise eine Verbindung der Formel VI die Benzyloxy- oder Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur und einem Wasserstoffdruck von 1 bis 5 bar abgespalten werden. Bei der Hydrogenolyse kann gleichzeitig eine halogenhaltige Verbindung enthalogeniert und eine vorhandende Doppelbindung aufhydriert werden.
d) Zur Herstellung von Verbindungen der allgemeinen Formel I, in der R₄ und R₅ jeweils ein Wasserstoffatom darstellen:
   Hydrierung einer Verbindung der allgemeinen Formel 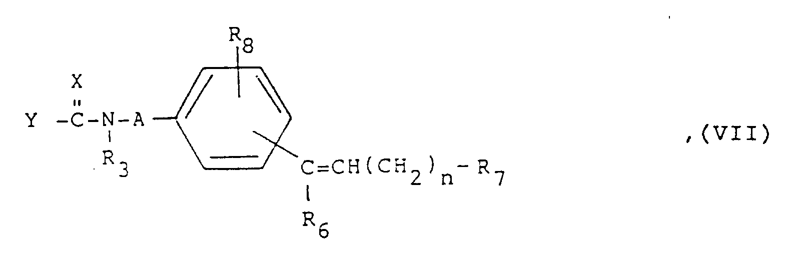 in der
   A, X, Y, n, R₃ und R₆ bis R₈ wie eingangs definiert sind.
   Die Hydrierung wird in einem geeigneten Lösungsmittel wie Methanol, Äthanol, Dioxan, Essigester oder Eisessig mit katalytisch angeregtem Wasserstoff, z.B. mit Wasserstoff in Gegenwart eines Hydrierungskatalysators wie Raney-Nickel, Palladium, Palladium/Kohle, Platin oder Platin/Kohle und einem Wasserstoffdruck von 1 bis 5 bar, oder mit nascierendem Wasserstoff, z.B. in Gegenwart von Eisen/Salzsäure, Zink/Eisessig, Zinn(II)chlorid/Salzsäure oder Eisen(II)sulfat/Schwefelsäure, bei Temperaturen zwischen 0 und 50°C, vorzugsweise bei Raumtemperatur, durchgeführt. Die katalytische Hydrierung kann jedoch auch stereoselektiv in Gegenwart eines geeigneten Katalysators erfolgen.
e) Zur Herstellung einer Verbindung der allgemeinen Formel I, in der X mit Ausnahme der eine Cyanogruppe enthaltenden Reste die für X eingangs erwähnten Bedeutungen besitzt und R₇ eine Tetrazolylgruppe darstellt:
   Umsetzung einer Verbindung der allgemeinen Formel 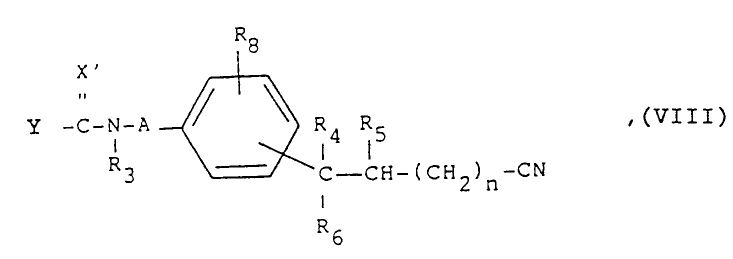 in der
   A, n, Y, R₃ bis R₆ und R₈ wie eingangs definiert sind und
   X' mit Ausnahme der eine Cyanogruppe enthaltenden Reste die für X eingangs erwähnten Bedeutungen besitzt, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150°C, vorzugsweise bei 125°C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

Erhält man erfindungsgemäß eine Verbindung der allgemeinen Formel I, in der X eine Gruppe der Formel = N-CN darstellt, so kann diese mittels Verseifung in eine entsprechende Verbindung der Formel I, in der X eine Gruppe der Formel =N-CONH₂ darstellt, übergeführt werden oder eine Verbindung der Formel I, in der R₇ eine Carboxygruppe darstellt, so kann diese mittels Veresterung oder Amidierung in eine entsprechende Verbindung der Formel I, in der R₇ eine in vivo metabolisch in eine Carboxygruppe überführbare Gruppe, eine Aminocarbonyl-, Alkylaminocarbonyl- oder Dialkylaminocarbonylgruppe darstellt, übergeführt werden oder
eine Verbindung der Formel I, in der R₇ eine Aminocarbonylgruppe darstellt, so kann diese mittels Dehydratisierung in eine entsprechende Verbindung der Formel I, in der R₇ eine Cyanogruppe darstellt, übergeführt werden.

Die nachträgliche Verseifung einer Gruppe der Formel =N-CN wird durch säure- oder basenkatalysierte Hydrolyse, beispielsweise unter Einwirkung von Schwefel- oder Phosphorsäure, Ameisensäure, Salzsäure, Bromwasserstoffsäure, Essigsäure, Bortrifluorid, Titantetrachlorid oder einer Kombination von H₂O₂ mit Natron- oder Kalilauge, bei Temperaturen zwischen 0 und 100°C, vorzugsweise bei 20 bis 50°C, durchgeführt.

Die nachträgliche Veresterung oder Amidierung wird zweckmäßigerweise in einem Lösungsmittel, z.B. in einem Überschuß des eingesetzten Alkohols wie Methanol, Äthanol oder Isopropanol oder des eingesetzten Amins wie Ammoniak, Methylamin, n-Propylamin oder Dimethylamin, in Gegenwart eines die Säure aktivierenden Mittels wie Thionylchlorid oder Chlorwasserstoffgas, Carbonyldiimidazol oder N,N'-Dicyclohexylcarbodiimid bei Temperaturen zwischen -20 und 180°C, vorzugsweise jedoch bei Raumtemperatur, durchgeführt.

Die Überführung einer Carboxylgruppe in eine Gruppe, die in vivo metabolisch in eine Carboxygruppe umgewandelt wird, erfolgt zweckmäßigerweise durch Veresterung mit einem entsprechenden Alkohol oder mit einem entsprechenden reaktionsfähigen Acylderivat zweckmäßigerweise in einem Lösungsmittel oder Lösungsmittelgemisch wie Wasser, Methylenchlorid, Chloroform, Äther, Tetrahydrofuran, Dioxan oder Dimethylformamid oder in einem Überschuß des Acylierungsmittels als Lösungsmittel gegebenenfalls in Gegenwart eines die Säure aktivierenden oder wasserentziehenden Mittels wie Thionylchlorid, mit deren Anhydriden, Estern oder Halogeniden gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

Die nachträgliche Dehydratisierung wird mit einem wasserentziehenden Mittel wie Phosphorpentoxid, Schwefelsäure oder p-Toluolsulfonsäurechlorid gegebenenfalls in einem Lösungsmittel wie Methylenchlorid oder Pyridin bei Temperaturen zwischen 0 und 100°C, vorzugsweise bei Temperaturen zwischen 20 und 80°C, durchgeführt.

Die erhaltenen Verbindungen der Formel I können ferner in ihre Enantiomeren aufgetrennt werden. So lassen sich die erhaltenen Verbindungen der Formel I, welche nur ein optisch aktives Zentrum enthalten, nach an sich bekannten Methoden (sehe Allinger N. L. und Eliel W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Basen, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Basen sind z.B. die D- und L-Formen von α-Phenyl-äthylamin oder Cinchonidin.

Desweiteren lassen sich die erhaltenen Verbindungen der Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält beispielsweise eine Verbindung der Formel I zwei optisch aktive Kohlenstoffatome, so erhält man die entsprechenden (R R', S S')- und (R S', S R')-Formen.

Außerdem lassen sich die so erhaltenen Verbindungen der Formel I, in denen R₄ und R₅ zusammen eine Kohlenstoff-Kohlenstoff-Bindung darstellen, mittels üblichen Methoden beispielsweise durch Chromatographie an einem Träger wie Kieselgel oder durch Kristallisation in ihre cis- und trans-Isomere überführen.

Desweiteren lassen sich die so erhaltenen neuen Verbindungen der Formel I, falls diese eine Carboxygruppe enthalten, gewünschtenfalls anschließend in ihre Additionssalze mit anorganischen oder organischen Basen, oder, falls diese eine basische Gruppe enthalten, gewünschtenfalls anschließend in ihre Salze mit anorganischen oder organischen Säuren, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin, Triäthanolamin, N-Methylglucosamin, Arginin und Lysin und als Säuren beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der Formeln II bis VIII erhält man nach literaturbekannten Verfahren bzw. sind literaturbekannt.

Eine als Ausgangsstoff verwendete Verbindung der Formel II erhält man aus einer entsprechenden N-Acylaminoverbindung durch Acylierung nach Friedel-Craft, anschließende Entacylierung und gegebenenfalls anschließende Reduktion, Hydrolyse und/oder Veresterung oder
durch Umsetzung einer entsprechenden Magnesium- oder Lithiumverbindung mit einer entsprechenden substituierten Pyridinverbindung wie 3-Cyano-pyridin, Pyridin-3-aldehyd oder einem Pyridin-3-carbonsäurederivat und gegebenenfalls anschließende Oxidation.

Eine als Ausgangsstoff verwendete Verbindung der Formel II, in der R₄ und R₅ zusammen eine Kohlenstoff-Kohlenstoff-Bindung darstellen, erhält man durch Umsetzung einer Verbindung der allgemeinen Formel 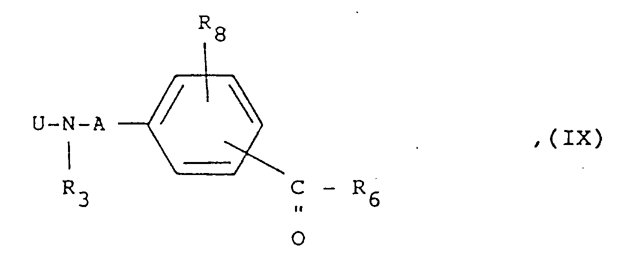
in der
R₃, R₆, R₈ und A wie eingangs definiert sind und
U eine Schutzgruppe für eine Aminogruppe darstellt, mit einer Verbindung der allgemeinen Formel

W - CH₂ - (CH₂)ₙ - R₇ ,(X)

in der
R₇ und n wie eingangs definiert sind und
W einen Triphenylphosphoniumhalogenid-, Dialkylphosphonsäure- oder ein Magnesiumhalogenidrest bedeuten, anschließende Abspaltung des verwendeten Schutzrestes und gegebenenfalls anschließende Dehydratisierung.

Die Umsetzung wird vorzugsweise in einem geeigneten Lösungsmittel wie Diethylether, Tetrahydrofuran, Dioxan oder Dimethylformamid bei Temperaturen zwischen -30 und 100°C, vorzugsweise bei Temperaturen zwischen -20 und 25°C, gegebenenfalls in Gegenwart einer Base, durchgeführt.

Die Umsetzung mit einem Triphenylphosphoniumhalogenid der Formel X wird besonders vorteilhaft jedoch in Gegenwart einer Base wie Kalium-tert.butylat oder Natriumhydrid durchgeführt.

Sollte bei der Umsetzung mit einem Magnesiumhalogenid der Formel X bei dem im Reaktionsgemisch primär entstandenen Carbinol die Hydroxygruppe nicht während der Umsetzung abgespalten werden, so wird diese in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Ethanol, Isopropanol oder Dioxan bei Temperaturen zwischen 0 und 120°C, z.B. bei Temperaturen zwischen Raumtemperatur und der Siedetemperatur des Reaktionsgemisches, abgespalten.

Die nachträgliche Dehydratisierung wird mit einem wasserentziehenden Mittel wie Phosphorpentoxid, Schwefelsäure oder p-Toluolsulfonsäurechlorid gegebenenfalls in einem Lösungsmittel wie Methylenchlorid oder Pyridin bei Temperaturen zwischen 0 und 100°C, vorzugsweise bei Temperaturen zwischen 20 und 80°C, durchgeführt.

Die als Ausgangsstoffe verwendeten Verbindungen der Formel III erhält man nach J. Heteroc. Chem. 19, 1205 (1982) entweder durch Umsetzung von Diphenoxydichlormethan mit einem entsprechend substituierten Amin oder durch Umsetzung von Schwefelkohlenstoff mit einer entsprechend substituierten C-H-aciden Methyl- bzw. Methylenkomponente und anschließende Methylierung (Chem. Ber. 95, 2861 (1962)).

Die als Ausgangsstoffe verwendeten Verbindungen der Formeln VI, VII und VIII erhält man durch Umsetzung einer entsprechenden Aminoverbindung mit einem entsprechenden Kohlensäurederivat.

Die als Ausgangsstoffe verwendeten Verbindungen der Formel IX erhält man durch Friedel-Craft's-Acylierung eines entsprechenden Amins.

Die als Ausgangsstoffe verwendeten Verbindungen der Formel X erhält man durch Umsetzung einer entsprechenden Halogenverbindung mit Triphenylphosphan oder mit einem Trialkylphosphonester.

Wie bereits eingangs erwähnt, weisen die neuen Verbindungen der allgemeinen Formel I, in der Y eine R₁NR₂-Gruppe darstellt, und deren physiologisch verträgliche Salze mit anorganischen oder organischen Basen oder Säuren wertvolle pharmakologische Eigenschaften auf, insbesondere antithrombotische Wirkungen und eine Hemmwirkung auf die Plättchenaggregation. Außerdem stellen sie auch Thromboxanantagonisten und Thromboxansynthesehemmer dar, wobei besonders bemerkenswert ist, daß die Verbindungen der Formel I diese Wirkung gleichzeitig aufweisen. Ferner weisen diese auch eine Wirkung auf die PGE₂-Produktion in der Lunge und auf die PGD₂-, PGE₂-und PGF_{2α}-Produktion in Humanthrombozyten auf.

Beispielsweise werden die neuen Verbindungen
- A =: 5E-6-(3-(2-Cyano-3-cyclopropyl-guanidino)phenyl)-6-(3-pyridyl)hex-5-ensäure,
- B =: 5E-6-(3-(2-Cyano-3-tert.butyl-guanidino)phenyl)-6-(3-pyridyl)hex-5-ensäure,
- C =: 5E-6-(3-(2-Cyano-3-cyclopentyl-guanidino)phenyl)-6-(3-pyridyl)hex-5-ensäure,
- D =: 5E-6-(3-(2-Cyano-3-isopropyl-guanidino)phenyl)-6-(3-pyridyl)hex-5-ensäure,
- E =: 5E-6-(3-(2-Cyano-3-(exo-norborn-2-yl)guanidino)phenyl)-6-(3-pyridyl)hex-5-ensäure,
- F =: 5E-6-(3-(2-Cyano-3-(2-methylpropyl)guanidino)phenyl)-6-(3-pyridyl)hex-5-ensäure,
- G =: 5E-6-(3-(2-Cyano-3-neopentyl-guanidino)phenyl)-6-(3-pyridyl)hex-5-ensäure,
- H =: 5E-6-(3-(2-Cyano-3-pentyl-guanidino)phenyl)-6-(3-pyridyl)hex-5-ensäure,
- I =: 5E-6-(3-(2-Cyano-3-(3-methylbutyl)guanidino)phenyl)-6-(3-pyridyl)hex-5-ensäure,
- J =: 5E-6-(3-(2,2-Dicyano-1-(2-methylpropylamino)ethylenamino)phenyl)-6-(3-pyridyl)hex-5-ensäure,
- K =: 5E-6-(3-(2,2-Dicyano-1-isopropylamino-ethylenamino)phenyl)-6-(3-pyridyl)hex-5-ensäure,
- L =: 5E-6-(3-(2,2-Dicyano-1-(3-methylbutylamino)-ethylenamino)phenyl)-6-(3-pyridyl)hex-5-ensäure,
- M =: 5E-6-(3-(2,2-Dicyano-1-cyclopentylamino-ethylenamino)phenyl)-6-(3-pyridyl)hex-5-ensäure,
- N =: 5E-6-(3-(2,2-Dicyano-1-neopentylamino-ethylenamino)phenyl)-6-(3-pyridyl)hex-5-ensäure,
- O =: 5E-6-(3-(2,2-Dicyano-1-cyclopropylamino-ethylenamino)phenyl)-6-(3-pyridyl)hex-5-ensäure,
- P =: 5E-6-(3-(2,2-Dicyano-1-propylamino-ethylenamino)phenyl)-6-(3-pyridyl)hex-5-ensäure,
- Q =: 5E-6-(3-(2,2-Dicyano-1-tert.butylamino-ethylenamino)phenyl)-6-(3-pyridyl)hex-5-ensäure,
- R =: 5E-6-(4-(2-Cyano-3-cyclohexyl-guanidino)phenyl)-6-(3-pyridyl)hex-5-ensäure,
- S =: 6-(3-(2-Cyano-3-tert.butyl-guanidino)phenyl)-6-(3-pyridyl)hexansäure,
- T =: 5E-6-(3-(1-Neopentylamino-2-nitro-ethylenamino)phenyl)-6-(3-pyridyl)hex-5-ensäure,
- U =: E/Z-6-(4-(2-(2-Cyano-3-tert.butyl-guanidino)ethyl)phenyl)-6-(3-pyridyl)hex-5-ensaure,
- V =: 5E-6-(3-(3-tert.Butyl-2-phenylsulfonyl-guanidino)phenyl)-6-(3-pyridyl)hex-5-ensäure,
- W =: 5E-6-(3-(2-Amidosulfonyl-3-(2-methylpropyl)guanidino)phenyl)-6-(3-pyridyl)hex-5-ensäure,
- X =: 5E-6-(3-(2-Carbamoyl-2-cyano-1-(2-methylpropylamino)ethylenamino)phenyl)-6-(3-pyridyl)hex-5-ensäure und
- Y =: 4E-1-(5-(3-(2-Cyano-3-cyclopentyl-guanidino)phenyl)-5-(3-pyridyl)-4-pentenyl)tetrazol
auf ihre biologischen Eigenschaften wie folgt geprüft:

### 1. Antithrombotische Wirkung

### Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

### Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.
Vor der Collagen-Zugabe wird das Plasma jeweils 10 Minuten mit der Substanz bei 37°C inkubiert.

Aus den erhaltenen Meßzahlen wird graphisch eine EC₅₀ bestimmt, die sich auf eine 50%ige Änderung der "optical density" im Sinne einer Aggregationshemmung bezieht.

### 2. Thromboxanantagonistische Wirkung

Venöses Humanblut wird mit 13 mM Na₃-Citrat antikoaguliert und 10 Minuten bei 170 x g zentrifugiert. Das überstehende plättchenreiche Plasma wird zur Entfernung der Plasmaproteine über eine Sepharose-2B-Säule gegeben. Aliquote der erhaltenen Plättchensuspension werden mit der Testsubstanz, dem Liganden (³H-markiert) und einem Marker (¹⁴C-markiert) 60 Minuten bei Raumtemperatur inkubiert und danach 20 Sekunden bei 10 000 x g abzentrifugiert. Der Überstand wird abgezogen und das Pellet in NaOH gelöst. Die ³H-Aktivität im Überstand entspricht dem freien Liganden, ¹⁴C ergibt die Konzentration des Markers. ³H im Pellet entspricht dem gebundenen Liganden, ¹⁴C wird zur Korrektur für Ligand im Extrazellulärraum verwendet. Aus den Bindungswerten für verschiedene Konzentrationen der Testsubstanz wird nach Iteration die Verdrängungskurve ermittelt und die IC₅₀ bestimmt.

### 3. Bestimmung der Hemmwirkung auf die Thromboxansynthetase

Venöses Humanblut wird mit 13 mM Na₃-Citrat antikoaguliert und 10 Minuten bei 170 x g zentrifugiert. Das überstehende plättchenreiche Plasma wird zur Entfernung der Plasmaproteine über eine Sepharose-2B-Säule gegeben. Aliquots der erhaltenen Plättchensuspension werden mit der Testsubstanz bzw. Lösungsmittel als Kontrolle 10 Minuten bei Raumtemperatur inkubiert und nach Zugabe ¹⁴C-markierter Arachidonsäure die Inkubation weitere 10 Minuten fortgesetzt. Nach Abstoppen mit 50 »l Zitronensäure wird 3 x mit 500 »l Essigester extrahiert und die vereinigten Extrakte mit Stickstoff abgeblasen. Der Rückstand wird in Essigester aufgenommen, auf DC-Folie aufgetragen und mit Chloroform:Methanol:Eisessig:Wasser (90:8:1:0,8, v/v/v/v) getrennt. Die getrockneten DC-Folien werden 3 Tage auf Röntgenfilm gelegt, die Autoradiogramme entwickelt und mit ihrer Hilfe die aktiven Zonen auf der Folie markiert. Nach Ausschneiden wird die Aktivität im Szintillationszähler bestimmt und die Hemmung der TXB2-Bildung berechnet. Die IC₅₀ wird durch lineare Interpolation bestimmt.

Die nachfolgende Tabelle enthält die gefundenen Werte:

| Beispiel | Hemmung der Thromboxansynthetase IC₅₀ | Thromboxanantagonistische Wirkung IC₅₀ | Hemmung der collageninduzierten Aggregation EC₅₀ |
|---|---|---|---|
| A | 0,380 »M/l | 0,017 »M/l | 0,90 »M/l |
| B | 0,004 »M/l | 0,011 »M/l | 0,50 »M/l |
| C | 0,003 »M/l | 0,030 »M/l | 0,53 »M/l |
| D | 0,024 »M/l | 0,018 »M/l | 0,40 »M/l |
| E | 0,004 »M/l | 0,017 »M/l | 0,75 »M/l |
| F | 0,030 »M/l | 0,007 »M/l | 0,35 »M/l |
| G | 0,014 »M/l | 0,022 »M/l | 0,26 »M/l |
| H | 0,005 »M/l | 0,025 »M/l | 0,64 »M/l |
| I | 0,003 »M/l | 0,027 »M/l | 0,69 »M/l |
| J | 0,030 »M/l | 0,002 »M/l | 0,12 »M/l |
| K | 0,045 »M/l | 0,002 »M/l | 0,02 »M/l |
| L | 0,031 »M/l | 0,015 »M/l | 1,20 »M/l |
| M | 0,033 »M/l | 0,001 »M/l | 0,05 »M/l |
| N | 0,044 »M/l | 0,003 »M/l | 0,62 »M/l |
| O | 0,065 »M/l | 0,062 »M/l | 0,04 »M/l |
| P | 0,040 »M/l | 0,037 »M/l | 0,05 »M/l |
| Q | 0,050 »M/l | 0,050 »M/l | 0,12 »M/l |
| R | 0,180 »M/l | 1,300 »M/l | 31,00 »M/l |
| S | 0,037 »M/l | 0,120 »M/l | 1,10 »M/l |
| T | 0,290 »M/l | 0,280 »M/l | 9,50 »M/l |
| U | 0,007 »M/l | 0,050 »M/l | 1,20 »M/l |
| V | 0,004 »M/l | 0,055 »M/l | 3,00 »M/l |
| W | 2,700 »M/l | 0,600 »M/l | 12,00 »M/l |
| X | 0,005 »M/l | 0,380 »M/l | 31,00 »M/l |
| Y | 0,250 »M/l | 0,021 »M/l | 1,20 »M/l |

### 4. Akute Toxizität

Die akute Toxizität der zu untersuchenden Substanzen wurde orientierend an Gruppen von je 10 Mäusen nach oraler Gabe einer Einzeldosis von 250 mg/kg bestimmt (Beobachtungszeit: 14 Tage). Bei dieser Dosis verstarb keines der eingesetzten Tiere.

Aufgrund ihrer pharmakologischen Eigenschaften eignen sich die neuen Verbindungen und deren physiologisch verträgliche Additionssalze zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe sowie zur Behandlung der Ischämie, des Asthmas und von Allergien.

Die neuen Verbindungen und deren physiologisch verträglichen Additionssalze sind auch bei der Behandlung von Erkrankungen nützlich, bei denen eine thromboxanvermittelte Konstriktion oder PGE₂ vermittelte Dilatation der Kapillaren eine Rolle spielen, z.B. bei der pulmonalen Hypertension. Außerdem können diese zur Verminderung des Schweregrades einer Transplantatabstoßung, zur Verminderung der renalen Toxizität von Substanzen wie Cyclosporin, zur Behandlung von Nierenerkrankungen, insbesondere zur Therapie oder Prophylaxe von Nierenveränderungen im Zusammenhang von Hypertension, systemischem Lupus oder Ureterobstruktionen und bei Schockzuständen im Zusammenhang mit Sepsis, Trauma oder Verbrennungen eingesetzt werden.

Die zur Erzielung einer entsprechenden Wirkung erforderliche Dosierung beträgt zweckmäßigerweise zwei- bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Ein weiterer Gegenstand der vorliegenden Erfindung sind neue Arzneimittel, enthaltend eine erfindungsgemäß hergestellte Verbindung der Formel I und einen PDE-Hemmer oder ein Lysemittel.

Als PDE-Hemmer kommt hierbei beispielsweise
2,6-Bis(diäthanolamino)-4,8-dipiperidino-pyrimido[5,4-d]pyrimidin (Dipyridamol),
2,6-Bis(diäthanolamino)-4-piperidino-pyrimido[5,4-d]pyrimidin (Mopidamol),
2-(4-Methoxy-phenyl)-5(6)-(5-methyl-3-oxo-4,5-dihydro-2H-6-pyridazinyl)-benzimidazol (Pimobendan),
2-(4-Hydroxy-phenyl)-5(6)-(5-methyl-3-oxo-4,5-dihydro-2H-6-pyridazinyl)-benzimidazol,
1-(1-Oxido-thiomorpholino)-3-piperazino-5-methyl-isochinolin,
6-[4-(3,4-Dichlorphenylsulfinyl)-butoxy]-3,4-dihydrocarbostyril und
6-[4-(2-Pyridylsulfonyl)-butoxy]carbostyril, wobei die perorale Tagesdosis
für Dipyridamol 2,5 bis 7,5 mg/kg, vorzugsweise 5 mg/kg,
für Mopidamol 15 bis 25 mg/kg, vorzugsweise 20 mg/kg,
für 2-(4-Methoxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 2-(4-Hydroxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 1-(1-Oxido-thiomorpholino)-3-piperazino-5-methyl-isochinolin 0,20 bis 2,00 mg/kg, vorzugsweise 0,40 bis 1,00 mg/kg,
für 6-[4-(3,4-Dichlorophenylsulfinyl)-butoxy]-3,4-dihydrocarbostyril 0,10 bis 1,00 mg/kg, vorzugsweise 0,20 bis 0,50 mg/kg und
für 6-[4-(2-Pyridylsulfonyl)-butoxy]carbostyril 0,10 bis 1,00 mg/kg, vorzugsweise 0,20 bis 0,50 mg/kg, beträgt,
und als Lysemittel Plasminogen-Aktivatoren wie t-PA, rt-PA, Streptokinase, Eminase oder Urokinase in Betracht, wobei die Lysemittel parenteral, vorzugsweise jedoch intravenös, verabreicht werden, z.B. t-PA oder rt-PA in einer Dosierung zwischen 15 und 100 mg pro Patient, Urokinase in einer Dosis zwischen 250 000 und 3 000 000 Einheiten pro Patient, Eminase in einer Dosierung von ungefähr 30 mg pro Patient und Streptokinase in einer Dosis zwischen 5 x 10⁴ bis 3 x 10⁷ IU jeweils innerhalb 5 Minuten und 24 Stunden.

Zur pharmazeutischen Anwendung läßt sich eine neue Kombination, enthaltend 1 bis 500 mg eines PDE-Hemmers, vorzugsweise jedoch 2 bis 75 mg, plus 10 bis 300 mg einer erfindungsgemäß hergestellten Verbindung der Formel I, vorzugsweise jedoch 10 bis 200 mg, sowie deren physiologisch verträgliche Additionssalze zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten. Hierbei erfolgt die Applikation am Erwachsenen zur Erzielung einer entsprechenden Wirkung 2 bis 4 mal täglich, vorzugsweise jedoch 3 bis 4 mal täglich.

Desweiteren läßt sich zur pharmazeutischen Anwendung eine neue Kombination, enthaltend ein Lysemittel in den vorstehend erwähnten Dosierungen plus 10 bis 300 mg einer erfindungsgemäß hergestellten Verbindung der Formel I, vorzugsweise jedoch 10 bis 200 mg, sowie deren physiologisch verträgliche Additionssalze in die üblichen parenteralen, vorzugsweise in die üblichen intravenösen, Applikationsformen wie Ampullen oder Infusionen einarbeiten, wobei die Applikation innerhalb von 5 Minuten und 24 Stunden erfolgen kann.

Selbstverständlich können die einzelnen Wirksubstanzen der vorstehenden Kombinationen gewünschtenfalls appliziert werden.

Die nachfolgenden Beispiele sollen die Erfindung näher erläutern:

### Herstellung der Ausgangsprodukte:

### Beispiel I

### 6-(4-Aminophenyl)-6-(3-pyridyl)hex-5-ensäuremethylester

### a) 4-Acetylaminophenyl-3-pyridyl-keton

980 g Aluminiumtrichlorid werden langsam mit 155 ml Dimethylformamid versetzt. Zu dieser Mischung gibt man bei 90-110°C nacheinander 342 g Nicotinsäurechlorid-hydrochlorid und 206 g N-Acetylanilin. Anschließend wird die Reaktionsmischung mit 600 ml Ethylenchlorid versetzt, danach auf Eis gegeben und unter Kühlung durch Zugabe von 15 N Natronlauge neutralisiert. Die wäßrige Phase wird mit Methylenchlorid extrahiert. Die vereinigten organischen Phasen werden eingeengt und der Rückstand wird aus Methanol umkristallisiert.
Ausbeute: 44 % der Theorie,
Schmelzpunkt: 189-191°C

| C₁₄H₁₂N₂0₃ (240,26) | | | | | | |
|---|---|---|---|---|---|---|
| Ber.: | C | 69,99 | H | 5,03 | N | 11,66 |
| Gef.: | | 69,87 | | 5,14 | | 11,58 |

### b) 6-(4-Acetylaminophenyl)-6-(3-pyridyl)hex-5-ensäure

Zu einer Suspension von 307 g 4-Carboxybutyl-triphenylphosphoniumbromid und 233 g Kalium-tert.butylat in 2,8 l Tetrahydrofuran gibt man bei -40°C 140 g 4-Acetylaminophenyl-3-pyridyl-keton und rührt 2 Stunden. Die Reaktionsmischung wird durch Zugabe von Eiswasser zersetzt und eingeengt. Der Rückstand wird in Wasser aufgenommen und mit Essigsäureethylester gewaschen. Die wäßrige Phase wird auf pH 5-6 angesäuert und mit Essigsäureethylester extrahiert. Die organische Phase wird eingeengt und der Rückstand aus Essigsäureethylester/Diisopropylether umkristallisiert.
Ausbeute: 86 % der Theorie,
Schmelzpunkt: 155-156°C

| C₁₉H₂₀N₂0₃ (324,38) | | | | | | |
|---|---|---|---|---|---|---|
| Ber.: | C | 70,35 | H | 6,21 | N | 8,64 |
| Gef.: | | 70,19 | | 6,27 | | 8,66 |

### c) 6-(4-Aminophenyl)-6-(3-pyridyl)hex-5-ensäuremethylester

65 g 6-(4-Acetylaminophenyl)-6-(3-pyridyl)hex-5-ensäure werden in einer Mischung aus 300 ml Methanol und 150 ml gesättigter methanolischer Salzsäure 2 Stunden unter Rückfluß gekocht. Die Reaktionsmischung wird mit 500 ml Wasser versetzt, durch Zugabe von Natriumcarbonat neutralisiert und mit Essigsäureethylester extrahiert. Die organische Phase wird gewaschen, getrocknet und eingeengt.
Ausbeute: 71 % der Theorie,
Öl, R_{f}-Wert: 0,72 (Kieselgel; Dichlormethan/Aceton = 9:1)

| C₁₈H₂₀N₂0₂ (296,37) | | | | | | |
|---|---|---|---|---|---|---|
| Ber.: | C | 72,95 | H | 6,80 | N | 9,45 |
| Gef.: | | 72,83 | | 6,85 | | 9,23 |

### Analog Beispiel I wird folgende Verbindung erhalten:

6-(4-Methylaminophenyl)-6-(3-pyridyl)hex-5-ensäuremethylester
Öl, R_{f}-Wert: 0,56 (Kieselgel; Dichlormethan/Ethanol = 20:1)

| C₁₉H₂₂N₂0₂ (310,40) | | | | | | |
|---|---|---|---|---|---|---|
| Ber.: | C | 73,52 | H | 7,14 | N | 9,03 |
| Gef.: | | 73,35 | | 7,24 | | 8,91 |

### Beispiel II

### 6-(3-Aminophenyl)-6-(3-pyridyl)hex-5-ensäuremethylester

### a) 3-Acetylaminophenyl-3-pyridylketon

114 g 3-Nitrophenyl-3-pyridylketon werden in 1 000 ml Essigsäure und 35 g Raney-Nickel 2 Stunden bei 50°C und 5 bar hydriert. Der Katalysator wird abfiltriert und das Filtrat mit 80 ml Essigsäureanhydrid versetzt. Nach 30 Minuten bei Raumtemperatur wird eingeengt und der Rückstand in Essigsäureethylester aufgenommen. Die organische Phase wird mit wäßriger Kaliumcarbonatlösung gewaschen und über Natriumsulfat getrocknet. Das Lösungsmittel wird abgezogen und der Rückstand aus Essigsäureethylester/Diisopropylether umkristallisiert.
Ausbeute: 69 % der Theorie,
Schmelzpunkt: 116-117°C

| C₁₄H₁₂N₂0₂ (240,26) | | | | | | |
|---|---|---|---|---|---|---|
| Ber.: | C | 69,99 | H | 5,03 | N | 11,66 |
| Gef.: | | 70,01 | | 5,11 | | 11,81 |

### b) 6-(3-Acetylaminonhenyl)-6-(3-pyridyl)hex-5-ensäure

Zu einer Mischung aus 217 g 4-Carboxybutyl-triphenylphosphoniumbromid und 154 g Kalium-tert.butylat in 1,8 l Tetrahydrofuran gibt man bei -25°C 94 g 3-Acetylaminophenyl-3-pyridylketon. Nach 2 Stunden Rühren bei Raumtemperatur wird die Reaktionsmischung mit 200 ml Wasser versetzt und anschließend weitgehend eingeengt. Der Rückstand wird in 500 ml Wasser aufgenommen und mit Essigsäureethylester gewaschen. Anschließend wird die wäßrige Phase durch Zugabe von Zitronensäure neutralisiert und mit Essigsäureethylester extrahiert. Die organische Phase wird eingeengt und der Rückstand aus Essigsäureethylester/Aceton umkristallisiert.
Ausbeute: 85 % der Theorie,
Schmelzpunkt: 86-89°C

| C₁₉H₂₀N₂0₃ (324,38) | | | | | | |
|---|---|---|---|---|---|---|
| Ber.: | C | 70,35 | H | 6,21 | N | 8,64 |
| Gef.: | | 70,15 | | 6,36 | | 8,50 |

### c) 6-(3-Aminophenyl)-6-(3-pyridyl)hex-5-ensäuremethylester

65 g 6-(3-Acetylaminophenyl)-6-(3-pyridyl)hex-5-ensäure werden in einer Mischung aus 400 ml Methanol und 200 ml methanolischer Salzsäure 4 Stunden unter Rückfluß gekocht. Das Lösungsmittel wird abgezogen und der Rückstand in Wasser aufgenommen. Die wäßrige Phase wird mit Essigsäureethylester gewaschen und durch Zugabe von 4N Natronlauge auf pH 8-9 eingestellt. Die wäßrige Phase wird mit Essigsäureethylester extrahiert. Die organische Phase wird gewaschen, getrocknet und eingeengt.
Ausbeute: 71 % der Theorie,
Öl, R_{f}-Wert: 0,55 (Kieselgel; Dichlormethan/Ethanol = 9:1)

| C₁₈H₂₀N₂0₂ (296,37) | | | | | | |
|---|---|---|---|---|---|---|
| Ber.: | C | 72,95 | H | 6,80 | N | 9,45 |
| Gef.: | | 72,83 | | 6,91 | | 9,18 |

### Analog Beispiel II werden folgende Verbindungen erhalten:

5-(3-Aminophenyl)-5-(3-pyridyl)pent-4-ensäuremethylester
Harz, R_{f}-Wert: 0,58 (Kieselgel; Dichlormethan/Ethanol = 20:1)

| C₁₇H₁₈N₂0₂ (282,34) | | | | | | |
|---|---|---|---|---|---|---|
| Ber.: | C | 72,32 | H | 6,43 | N | 9,92 |
| Gef.: | | 72,29 | | 6,55 | | 9,70 |

7-(3-Aminophenyl)-7-(3-pyridyl)hept-6-ensäuremethylester
Harz, R_{f}-Wert: 0,63 (Kieselgel; Dichlormethan/Ethanol = 20:1)

| C₁₉H₂₂N₂0₂ (310,40) | | | | | | |
|---|---|---|---|---|---|---|
| Ber.: | C | 73,52 | H | 7,14 | N | 9,03 |
| Gef.: | | 73,41 | | 7,18 | | 8,89 |

8-(3-Aminophenyl)-8-(3-pyridyl)oct-7-ensäuremethylester
Öl, R_{f}-Wert: 0,66 (Kieselgel; Dichlormethan/Ethanol = 20:1)

| C₂₀H₂₄N₂0₂ (324,44) | | | | | | |
|---|---|---|---|---|---|---|
| Ber.: | C | 74,05 | H | 7,46 | N | 8,63 |
| Gef.: | | 73,92 | | 7,49 | | 8,48 |

### Beispiel III

### 6-(3-Methylaminophenyl)-6-(3-pyridyl)hex-5-ensäuremethylester

### a) N-Acetyl-3-methylaminophenyl-3-pyridylketon

Zu 84 g 3-Acetylaminophenyl-3-pyridylketon in 600 ml Dimethylformamid gibt man portionsweise unter Kühlung 17 g Natriumhydrid und anschließend 22 ml Methyljodid. Man rührt eine Stunde bei Raumtemperatur und zersetzt die Reaktionsmischung durch Zugabe von 100 ml Wasser. Das Lösungsmittel wird abgezogen und der Rückstand in Essigsäureethylester aufgenommen. Die organische Phase wird gewaschen, getrocknet und eingeengt. Der Rückstand wird über eine Kieselgelsäule mit Dichlormethan/Ethanol (30:1) gereinigt.
Öl, R_{f}-Wert: 0,45 (Kieselgel; Dichlormethan/Ethanol = 30:1)

| C₁₅H₁₄N₂0₂ (254,29) | | | | | | |
|---|---|---|---|---|---|---|
| Ber.: | C | 70,85 | H | 5,55 | N | 11,02 |
| Gef.: | | 70,96 | | 5,65 | | 10,92 |

### b) 6-(3-Methylaminophenyl)-6-(3-pyridyl)hex-5-ensäuremethylester

Hergestellt aus N-Acetyl-3-methylaminophenyl-3-pyridylketon und 4-Carboxybutyl-triphenylphosphoniumbromid analog Beispiel IIb und anschließende Veresterung analog Beispiel IIc.
Öl, R_{f}-Wert: 0,56 (Kieselgel; Dichlormethan/Ethanol = 20:1)

| C₁₉H₂₂N₂0₂ (310,40) | | | | | | |
|---|---|---|---|---|---|---|
| Ber.: | C | 73,52 | H | 7,14 | N | 9,03 |
| Gef.: | | 73,53 | | 7,20 | | 8,84 |

### Beispiel IV

### 6-(3-Amino-4-methylphenyl)-6-(3-pyridyl)hex-5-ensäuremethylester

### a) 4-Methyl-3-nitrophenyl-3-pyridylketon

In eine auf 5-10°C gekühlte Mischung aus 22 ml konz. Schwefelsäure und 16 ml rauchender Salpetersäure werden 16,4 g 4-Methylphenyl-3-pyridylketon portionsweise eingetragen. Anschließend rührt man 2 Stunden bei Raumtemperatur, gibt die Reaktionsmischung auf Eis und stellt durch Zugabe von konz. Ammoniak alkalisch. Die wäßrige Phase wird mit Essigsäureethylester extrahiert. Die organische Phase wird getrocknet, eingeengt und der Rückstand über eine Kieselgelsäule mit Essigsäureethylester/Cyclohexan = 1:1 gereinigt. Die Produktfraktion wird eingeengt und der Rückstand aus Essigsäureethylester/Diisopropylether umkristallisiert.
Ausbeute: 60 % der Theorie,

| C₁₃H₁₀N₂0₃ (242,24) | | | | | | |
|---|---|---|---|---|---|---|
| Ber.: | C | 64,46 | H | 4,16 | N | 11,56 |
| Gef.: | | 64,42 | | 4,19 | | 11,64 |

### b) 3-Acetylamino-4-methylphenyl-3-pyridylketon

12 g 4-Methyl-3-nitrophenyl-3-pyridylketon werden in einer Mischung aus 120 ml Essigsäureethylester und 15 ml Methanol gelöst und nach Zugabe von 2 g Raney-Nickel 3 Stunden bei 50°C und einem Wasserstoffdruck von 3,5 bar hydriert. Der Katalysator wird abfiltriert, das Filtrat eingeengt und der Rückstand in 30 ml Eisessig aufgenommen. Die Lösung wird mit 10 ml Essigsäureanhydrid versetzt und 1 Stunde bei Raumtemperatur gerührt. Die Reaktionsmischung wird eingeengt, der Rückstand in Essigsäureethylester aufgenommen und mit 2N Natriumcarbonatlösung gewaschen. Der organische Extrakt wird getrocknet, eingeengt und der Rückstand aus Essigsäureethylester/Diisopropylether umkristallisiert.
Ausbeute: 77 % der Theorie,
Schmelzpunkt: 92-94°C

| C₁₅H₁₄N₂0₂ (254,29) | | | | | | |
|---|---|---|---|---|---|---|
| Ber.: | C | 70,85 | H | 5,55 | N | 11,02 |
| Gef.: | | 70,77 | | 5,64 | | 10,96 |

### Analog Beispiel IVb wird folgende Verbindung erhalten:

3-Acetylamino-5-trifluormethylphenyl-3-pyridylketon
Schmelzpunkt: 128°C (Essigsäureethylester/Diisopropylether)

| C₁₅H₁₁F₃N₂0₂ (308,26) | | | | | | |
|---|---|---|---|---|---|---|
| Ber.: | C | 58,45 | H | 3,59 | N | 9,09 |
| Gef.: | | 58,42 | | 3,72 | | 9,10 |

### c) 6-(3-Acetylamino-4-methylphenyl)-6-(3-pyridyl)hex-5-ensäure

Herstellung analog Beispiel Ib.
Ausbeute: 70 % der Theorie,
Schmelzpunkt: 177-179°C (Isopropanol/Diisopropylether)

| C₂₀H₂₂N₂0₃ (338,41) | | | | | | |
|---|---|---|---|---|---|---|
| Ber.: | C | 70,99 | H | 6,55 | N | 8,28 |
| Gef.: | | 70,83 | | 6,46 | | 8,19 |

### Analog Beispiel IVc wird folgende Verbindung erhalten:

6-(3-Acetylamino-5-trifluormethylphenyl)-6-(3-pyridyl)hex-5-ensäure
Schmelzpunkt: 164°C (Dichlormethan)

| C₂₀H₁₉F₃N₂0₃ (392,38) | | | | | | |
|---|---|---|---|---|---|---|
| Ber.: | C | 61,22 | H | 4,88 | N | 7,14 |
| Gef.: | | 61,17 | | 4,79 | | 7,05 |

### d) 6-(3-Amino-4-methylphenyl)-6-(3-pyridyl)hex-5-ensäuremethylester

Herstellung analog Beispiel Ic.
Ausbeute: 92 % der Theorie,
Öl, R_{f}-Wert: 0,34 (Kieselgel; Dichlormethan/Aceton = 9:1)

| C₁₉H₂₂N₂0₂ (310,40) | | | | | | |
|---|---|---|---|---|---|---|
| Ber.: | C | 73,52 | H | 7,14 | N | 9,03 |
| Gef.: | | 73,35 | | 7,28 | | 8,86 |

### Analog Beispiel IVd wird folgende Verbindung erhalten:

6-(3-Amino-5-trifluormethylphenyl)-6-(3-pyridyl)hex-5-ensäuremethylester
Öl, R_{f}-Wert: 0,46 (Kieselgel; Dichlormethan/Ethanol = 10:1)

| C₁₉H₁₉F₃N₂0₂ (364,37) | | | | | | |
|---|---|---|---|---|---|---|
| Ber.: | C | 62,63 62,55 | H | 5,26 | N | 7,69 |
| Gef.: | | | | 5,24 | | 7,72 |

### Beispiel V

### 3-Nitro-5-trifluormethylphenyl-3-pyridylketon

Zu 101 g 5-Trifluormethylphenyl-3-pyridylketon gibt man vorsichtig nacheinander 400 ml konz. Schwefelsäure, 200 ml Oleum und 140 ml rauchende Salpetersäure, wobei die Innentemperatur 35°C nicht übersteigen soll. Die Reaktionsmischung wird 12 Stunden bei Raumtemperatur gerührt und anschließend auf 3 kg Eis gegeben. Die Reaktionslösung wird durch Zugabe von 50% Natronlauge neutralisiert, wobei das Nitratsalz ausfällt. Dieses wird abgesaugt und anschließend in 6N Natronlauge gelöst. Die Wasserphase wird mit Essigsäureethylester extrahiert. Der organische Extrakt wird getrocknet und eingeengt, wobei das erhaltene Öl beim Stehenlassen kristallisiert.
Ausbeute: 68 % der Theorie,
Schmelzpunkt: 182-184°C

| C₁₃H₇F₃N₂0₃ (296,20) | | | | | | |
|---|---|---|---|---|---|---|
| Ber.: | C | 52,71 | H | 2,38 | N | 9,46 |
| Gef.: | | 52,56 | | 2,45 | | 9,55 |

### Beispiel VI

### 4E-1-(5-(3-Aminophenyl)-5-(3-pyridyl)pent-4-enyl)tetrazol

### a) 4E-5-(3-Acetylaminophenyl)-5-(3-pyridyl)pent-4-encarbonitril

Zu einer Suspension von 25,5 g 4-Cyanobutyl-triphenylphosphoniumbromid und 14 g Kalium-tert.butylat in 200 ml Tetrahydrofuran gibt man bei -40°C 12 g 3-Acetylaminophenyl-3-pyridylketon. Man rührt 3 Stunden bei Raumtemperatur und zersetzt die Reaktionsmischung durch Zugabe von 50 ml Eiswasser. Die Mischung wird eingeengt, der Rückstand in Wasser aufgenommen und mit Essigsäureethylester extrahiert. Die organische Phase wird eingeengt und der Rückstand über eine Kieselgelsäule mit Dichlormethan/Ethanol = 40:1 gereinigt. Die Produktfraktion wird eingeengt und der Rückstand aus Essigsäureethylester/Diisopropylether umkristallisiert.
Ausbeute: 71 % der Theorie,
Schmelzpunkt: 144-146°C

| C₁₉H₁₉N₃0 (305,38) | | | | | | |
|---|---|---|---|---|---|---|
| Ber.: | C | 74,73 | H | 6,27 | N | 13,76 |
| Gef.: | | 74,57 | | 6,14 | | 13,59 |

### b) 4E-1-(5-(3-Acetylaminophenyl)-5-(3-pyridyl)pent-4-enyl)tetrazol

5,8 g 4E-5-(3-Acetylaminophenyl)-5-(3-pyridyl)pent-4-encarbonitril und 9,96 g Tributylzinnazid werden in 300 ml Toluol 48 Stunden unter Rückfluß gekocht. Die organische Phase wird mit 100 ml 1N Natronlauge extrahiert. Die wäßrige Phase wird mit Essigsäureethylester gewaschen und durch Zugabe von Zitronensäure auf einen pH-Wert von 4-5 eingestellt. Der ausgefallene Niederschlag wird abgesaugt, mit Wasser gewaschen und getrocknet.
Ausbeute: 83 % der Theorie,
Schmelzpunkt: 174-175°C

| C₁₉H₂₀N₆0 (348,41) | | | | | | |
|---|---|---|---|---|---|---|
| Ber.: | C | 65,50 | H | 5,79 | N | 24,12 |
| Gef.: | | 65,39 | | 5,86 | | 23,96 |

### c) 4E-1-(5-(3-Aminophenyl)-5-(3-pyridyl)pent-4-enyl)tetrazol

5,7 g 4E-1-(5-(3-Acetylaminophenyl)-5-(3-pyridyl)pent-4-enyl)tetrazol werden in 60 ml 4N Salzsäure 5 Stunden auf 60°C erwärmt. Die Reaktionslösung wird durch Zugabe von Natriumbicarbonat neutralisiert und anschließend durch Zugabe von Zitronensäure auf einen pH-Wert von 4-5 eingestellt. Die wäßrige Phase wird mit Dichlormethan/Methanol = 4:1 extrahiert. Die organische Phase wird mit gesättigter Natriumchlorid-Lösung gewaschen, getrocknet und eingeengt.
Ausbeute: 91 % der Theorie,
Öl, R_{f}-Wert: 0,49 (Kieselgel RP8; 5%ige Natriumchlorid-Lösung/Methanol = 4:6)

| C₁₇H₁₈N₆ (306,37) | | | | | | |
|---|---|---|---|---|---|---|
| Ber.: | C | 66,65 | H | 5,92 | N | 27,43 |
| Gef.: | | 66,53 | | 6,04 | | 27,21 |

### Beispiel 1

### 5E-6-(4-(2-Cyano-3-cyclohexyl-guanidino)phenyl)-6-(3-pyridyl)hex-5-ensäure

### a) 5E-6-(4-(Cyanimido-phenoxymethylenamino)phenyl)-6-(3-pyridyl)hex-5-ensäuremethylester

11,4 g Diphenoxymethylen-cyanamid (J. Heteroc. Chem. 19, 1205 (1982)) und 15 g 5E-6-(4-(Aminophenyl)-6-(3-pyridyl)hex-5-ensäuremethylester werden in 250 ml Isopropanol gelöst und 6 Stunden bei Raumtemperatur gerührt. Der entstandene Niederschlag wird abgesaugt und mit Diethylether gewaschen.
Ausbeute: 87 % der Theorie,
Schmelzpunkt: 163-165°C

| C₂₆H₂₄N₄O₃ (440,50) | | | | | | |
|---|---|---|---|---|---|---|
| Ber.: | C | 70,89 | H | 5,49 | N | 12,77 |
| Gef.: | | 70,67 | | 5,51 | | 12,50 |

### b) 5E-6-(4-(2-Cyano-3-cyclohexyl-guanidino)phenyl)-6-(3-pyridyl)hex-5-ensäure

2,2 g 5E-6-(4-(Cyanimido-phenoxymethylenamino)phenyl)-6-(3-pyridyl)hex-5-ensäuremethylester und 0,8 g Cyclohexylamin werden in 40 ml Isopropanol 2 Stunden unter Rückfluß erhitzt. Die noch heiße Reaktionsmischung wird filtriert, das Filtrat bei 40-50°C mit 6 ml 2N Natronlauge versetzt und 2 Stunden bei 40-50°C gerührt. Die Reaktionslösung wird eingeengt und der Rückstand in Wasser aufgenommen. Die wäßrige Phase wird mit Essigsäureethylester gewaschen und durch Zugabe von Zitronensäure auf pH 4-5 eingestellt. Die wäßrige Phase wird mit Essigsäureethylester extrahiert. Der organische Extrakt wird mit Wasser gewaschen, getrocknet und eingeengt. Der Rückstand wird aus Essigsäureethylester/Isopropanol umkristallisiert.
Ausbeute: 40 % der Theorie,
Schmelzpunkt: 145-147°C

| C₂₅H₂₉N₅O₂ (431,54) | | | | | | |
|---|---|---|---|---|---|---|
| Ber.: | C | 69,58 | H | 6,77 | N | 16,23 |
| Gef.: | | 69,53 | | 7,01 | | 15,98 |

### Analog Beispiel 1 werden folgende Verbindungen erhalten:

(1) 5E-6-(4-(2-Cyano-3-cyclopropyl-guanidino)phenyl)-6-(3-pyridyl)hex-5-ensäure
Schmelzpunkt: 118°C (Essigsäureethylester/Isopropanol)

| C₂₂H₂₃N₅O₂ (389,46) | | | | | | |
|---|---|---|---|---|---|---|
| Ber.: | C | 67,85 | H | 5,95 | N | 17,98 |
| Gef.: | | 67,61 | | 6,04 | | 17,79 |

(2) E/Z-6-(4-(2-Cyano-3-cyclopropyl-1-methyl-guanidino)phenyl)-6-(3-pyridyl)hex-5-ensäure
Ausgehend von 6-(4-Methylaminophenyl)-6-(3-pyridyl)hex-5-ensäuremethylester; Reinigung des Endproduktes durch Säulenchromatographie an Kieselgel mit Dichlormethan/Ethanol = 20:1.
Schaum, R_{f}-Wert: 0,25 (Kieselgel; Dichlormethan/Ethanol = 20:1).

| C₂₃H₂₅N₅O₂ (403,49) | | | | | | |
|---|---|---|---|---|---|---|
| Ber.: | C | 68,47 | H | 6,25 | N | 17,36 |
| Gef.: | | 68,24 | | 6,40 | | 17,52 |

### Beispiel 2

### 5E-6-(3-(2-Cyano-3-(2-hydroxy-1,1-dimethylethyl)guanidino)phenyl)-6-(3-pyridyl)hex-5-ensäure

### a) 5E-6-(3-(Cyanimido-phenoxymethylenamino)phenyl)-6-(3-pyridyl)hex-5-ensäuremethylester

8,4 g Diphenoxymethylen-cyanamid und 10,5 g 5E-6-(3-Aminophenyl)-6-(3-pyridyl)hex-5-ensäuremethylester werden in 100 ml Isopropanol gelöst und 5 Stunden bei Raumtemperatur gerührt. Anschließend wird die Reaktionsmischung eingeengt und der Rückstand über eine Kieselgelsäule mit Dichlormethan/Ethanol = 40:1 gereinigt.
Ausbeute: 86 % der Theorie,
Harz, R_{f}-Wert: 0,61 (Kieselgel; Dichlormethan/Ethanol = 20:1).

| C₂₆H₂₄N₄O₃ (440,50) | | | | | | |
|---|---|---|---|---|---|---|
| Ber.: | C | 70,89 | H | 5,49 | N | 12,72 |
| Gef.: | | 70,68 | | 5,60 | | 12,79 |

### Analog Beispiel 2a werden folgende Verbindungen erhalten:

(1) 5E-6-(3-(Cyanimido-phenoxy-methylenamino)-4-methylphenyl)-6-(3-pyridyl)hex-5-ensäuremethylester
Schmelzpunkt: 147-149°C (Essigsäureethylester/Diisopropylether)

| C₂₇H₂₆N₄O₃ (454,53) | | | | | | |
|---|---|---|---|---|---|---|
| Ber.: | C | 71,35 | H | 5,77 | N | 12,33 |
| Gef.: | | 71,19 | | 5,92 | | 12,26 |

(2) 5E-6-(3-(Cyanimido-phenoxy-methylenamino)-5-trifluormethylphenyl)-6-(3-pyridyl)hex-5-ensäuremethylester Schmelzpunkt: 148-149°C (Diethylether)

| C₂₇H₂₃F₃N₄O₃ (508,5) | | | | | | |
|---|---|---|---|---|---|---|
| Ber.: | C | 63,78 | H | 4,56 | N | 11,02 |
| Gef.: | | 63,67 | | 4,61 | | 11,11 |

### b) 5E-6-(3-(2-Cyano-3-(2-hydroxy-1,1-dimethylethyl)guanidino)phenyl)-6-(3-pyridyl)hex-5-ensäure

2,2 g 5E-6-(3-(Cyanimido-phenoxymethylenamino)phenyl)-6-(3-pyridyl)hex-5-ensäuremethylester und 2 ml 2-Hydroxy-1,1-dimethylethylamin werden in 22 ml Isopropanol 6 Stunden unter Rückfluß erhitzt. Die noch heiße Reaktionsmischung wird filtriert und bei 50°C mit 15 ml 2N Natronlauge versetzt. Die Reaktionslösung wird eine Stunde bei 50°C gerührt, anschließend eingeengt und der Rückstand in Wasser aufgenommen. Die wäßrige Phase wird mit Essigsäureethylester gewaschen und danach durch Zugabe von Zitronensäure auf pH 4-5 eingestellt. Die wäßrige Phase wird mit Essigsäureethylester extrahiert, der organische Extrakt getrocknet und eingeengt. Der Rückstand wird über eine Kieselgelsäule mit Dichlormethan/Ethanol = 19:1 gereinigt. Die Produktfraktion wird eingeengt und der Rückstand aus Essigsäureethylester/Diisopropylether umkristallisiert.
Ausbeute: 13 % der Theorie,
Schmelzpunkt: 155°C (Zers.)

| C₂₃H₂₇N₅O₃ (421,50) | | | | | | |
|---|---|---|---|---|---|---|
| Ber.: | C | 65,54 | H | 6,46 | N | 16,62 |
| Gef.: | | 65,57 | | 6,36 | | 16,41 |

### Analog Beispiel 2 werden folgende Verbindungen erhalten:

(1) 5E-6-(3-(2-Cyano-3-(2-phenylethyl)guanidino)phenyl-6-(3-pyridyl)hex-5-ensäure
Schmelzpunkt: 164°C (Zers., Wasser/Isopropanol)

| C₂₇H₂₇N₅O₂ (453,50) | | | | | | |
|---|---|---|---|---|---|---|
| Ber.: | C | 71,50 | H | 6,00 | N | 15,44 |
| Gef.: | | 71,34 | | 6,13 | | 15,26 |

(2) 5E-6-(3-(Cyanimido-(4-phenylpiperazin-1-yl)-methylenamino)phenyl)-6-(3-pyridyl)hex-5-ensäure
Schmelzpunkt: 125°C (Zers., Wasser/Isopropanol)

| C₂₉H₃₀N₆O₂ (494,61) | | | | | | |
|---|---|---|---|---|---|---|
| Ber.: | C | 70,42 | H | 6,11 | N | 16,99 |
| Gef.: | | 70,20 | | 5,99 | | 17,19 |

(3) 5E-6-(3-(Cyanimido-(4-phenylpiperidin-1-yl)-methylenamino)phenyl)-6-(3-pyridyl)hex-5-ensäure
Schmelzpunkt: 119°C (Zers., Wasser/Isopropanol)

| C₃₀H₃₁N₅O₂ (493,60) | | | | | | |
|---|---|---|---|---|---|---|
| Ber.: | C | 73,00 | H | 6,33 | N | 14,19 |
| Gef.: | | 72,73 | | 6,25 | | 14,05 |

(4) 5E-6-(3-(Cyanimido-(1,2,3,4-tetrahydroisochinolin-2-yl)methylenamino)phenyl)-6-(3-pyridyl)hex-5-ensäure
Schmelzpunkt: 194°C (Zers., Wasser/Isopropanol)

| C₂₈H₂₇N₅O₂ (465,60) | | | | | | |
|---|---|---|---|---|---|---|
| Ber.: | C | 72,24 | H | 5,85 | N | 15,04 |
| Gef.: | | 72,06 | | 5,97 | | 14,94 |

(5) 5E-6-(3-(2-Cyano-3-(indan-2-yl)guanidino)phenyl-6-(3-pyridyl)hex-5-ensäure
Schmelzpunkt: 132°C (Zers., Essigsäureethylester)

| C₂₈H₂₇N₅O₂ (465,60) | | | | | | |
|---|---|---|---|---|---|---|
| Ber.: | C | 72,24 | H | 5,85 | N | 15,04 |
| Gef.: | | 72,07 | | 5,88 | | 14,82 |

(6) 5E-6-(3-(2-Cyano-3-cyclopropyl-guanidino)phenyl)-6-(3-pyridyl)hex-5-ensäure
Schmelzpunkt: 125°C (Zers.)

| C₂₂H₂₃N₅O₂ (389,50) | | | | | | |
|---|---|---|---|---|---|---|
| Ber.: | C | 67,85 | H | 5,95 | N | 17,98 |
| Gef.: | | 67,62 | | 5,90 | | 17,74 |

(7) 5E-6-(3-(Cyanimido-piperidin-1-yl-methylenamino)phenyl)-6-(3-pyridyl)hex-5-ensäure
Schmelzpunkt: 197°C (Zers., Wasser/Isopropanol)

| C₂₄H₂₇N₅O₂ x 0,5 H₂0 (417,51) | | | | | | |
|---|---|---|---|---|---|---|
| Ber.: | C | 67,58 | H | 6,61 | N | 16,42 |
| Gef.: | | 67,72 | | 6,72 | | 16,21 |

(8) 5E-6-(3-(2-Cyano-3-tert.butyl-guanidino)phenyl)-6-(3-pyridyl)hex-5-ensäure
Schmelzpunkt: 150-151°C (Zers., Ethanol/Diisopropylether)

| C₂₃H₂₇N₅O₂ (405,50) | | | | | | |
|---|---|---|---|---|---|---|
| Ber.: | C | 68,12 | H | 6,71 | N | 17,27 |
| Gef.: | | 67,94 | | 6,78 | | 17,08 |

(9) 5E-6-(3-(2-Cyano-3-cyclohexyl-guanidino)phenyl)-6-(3-pyridyl)hex-5-ensäure
Schmelzpunkt: 156°C (Zers., Essigsäureethylester/Diethylether)

| C₂₅H₂₉N₅O₂ (431,54) | | | | | | |
|---|---|---|---|---|---|---|
| Ber.: | C | 69,58 | H | 6,77 | N | 16,23 |
| Gef.: | | 69,38 | | 6,80 | | 16,06 |

(10) 5E-6-(3-(2-Cyano-3-cyclopentyl-guanidino)phenyl)-6-(3-pyridyl)hex-5-ensäure
Schmelzpunkt: 136°C (Zers., Essigsäureethylester/Diethylether)

| C₂₄H₂₇N₅O₂ (417,51) | | | | | | |
|---|---|---|---|---|---|---|
| Ber.: | C | 69,04 | H | 6,57 | N | 16,77 |
| Gef.: | | 68,96 | | 6,48 | | 16,61 |

(11) 5E-6-(3-(2-Cyano-3-isopropyl-guanidino)phenyl)-6-(3-pyridyl)hex-5-ensäure
Schmelzpunkt: 186°C (Zers., Essigsäureethylester/Diethylether)

| C₂₂H₂₅N₅O₂ (391,47) | | | | | | |
|---|---|---|---|---|---|---|
| Ber.: | C | 67,50 | H | 6,44 | N | 17,89 |
| Gef.: | | 67,31 | | 6,50 | | 17,71 |

(12) 5E-6-(3-(2-Cyano-3-(exo-norborn-2-yl)guanidino)phenyl)-6-(3-pyridyl)hex-5-ensäure
Schmelzpunkt: 172-173°C (Zers., Isopropanol/Diisopropylether)

| C₂₆H₂₉N₅O₂ (443,55) | | | | | | |
|---|---|---|---|---|---|---|
| Ber.: | C | 70,41 | H | 6,60 | N | 15,79 |
| Gef.: | | 70,50 | | 6,63 | | 15,63 |

(13) 5E-6-(3-(Cyanimido-(3,3-dimethylpiperidin-1-yl)methylenamino)phenyl)-6-(3-pyridyl)hex-5-ensäure
Schmelzpunkt: 138°C (Zers., Essigsäureethylester/Diisopropylether)

| C₂₆H₃₁N₅O₂ (445,56) | | | | | | |
|---|---|---|---|---|---|---|
| Ber.: | C | 70,09 | H | 7,01 | N | 15,72 |
| Gef.: | | 69,98 | | 7,10 | | 15,59 |

(14) 5E-6-(3-(2-Cyano-3-trimethylsilylmethyl-guanidino)phenyl)-6-(3-pyridyl)hex-5-ensäure
Schmelzpunkt: 92°C (Zers., Essigsäureethylester/Diethylether)

| C₂₃H₂₉N₅O₂Si (435,60) | | | | | | |
|---|---|---|---|---|---|---|
| Ber.: | C | 63,42 | H | 6,71 | N | 16,08 |
| Gef.: | | 63,23 | | 6,80 | | 15,87 |

(15) 5E-6-(3-(2-Cyano-3-(3-pyridylmethyl)guanidino)phenyl)-6-(3-pyridyl)hex-5-ensäure
Schmelzpunkt: 125-126°C (Essigsäureethylester/Diisopropylether)

| C₂₅H₂₄N₆O₂ (440,51) | | | | | | |
|---|---|---|---|---|---|---|
| Ber.: | C | 68,17 | H | 5,49 | N | 19,08 |
| Gef.: | | 67,96 | | 5,52 | | 18,93 |

(16) 5E-6-(3-(2-Cyano-3-(1,1,3,3-tetramethylbutyl)guanidino)phenyl)-6-(3-pyridyl)hex-5-ensäure
Schmelzpunkt: 170°C (Zers., Essigsäureethylester/Diisopropylether)

| C₂₇H₃₅N₅O₂ (461,61) | | | | | | |
|---|---|---|---|---|---|---|
| Ber.: | C | 70,25 | H | 7,64 | N | 15,17 |
| Gef.: | | 70,25 | | 7,73 | | 15,12 |

(17) 5E-6-(3-(2-Cyano-3-(2-hydroxy-1-methyl-2-phenylethyl)guanidino)phenyl)-6-(3-pyridyl)hex-5-ensäure
Schmelzpunkt: 152-153°C (Essigsäureethylester/Diisopropylether)

| C₂₈H₂₉N₅O₃ (483,57) | | | | | | |
|---|---|---|---|---|---|---|
| Ber.: | C | 69,55 | H | 6,05 | N | 14,49 |
| Gef.: | | 69,43 | | 6,16 | | 14,38 |

(18) 5E-6-(3-(2-Cyano-3-(2-hydroxy-1-phenylethyl)guanidino)phenyl)-6-(3-pyridyl)hex-5-ensäure
Schaum, R_{f}-Wert: 0,33 (Dichlormethan/Ethanol = 19:1)

| C₂₇H₂₇N₅O₃ (469,54) | | | | | | |
|---|---|---|---|---|---|---|
| Ber.: | C | 69,07 | H | 5,80 | N | 14,92 |
| Gef.: | | 68,98 | | 5,87 | | 14,80 |

(19) 5E-6-(3-(2-Cyano-3-methyl-3-isopropyl-guanidino)phenyl)-6-(3-pyridyl)hex-5-ensäure
Schmelzpunkt: 168°C (Essigsäureethylester/Isopropanol)

| C₂₃H₂₇N₅O₂ (405,50) | | | | | | |
|---|---|---|---|---|---|---|
| Ber.: | C | 68,13 | H | 6,71 | N | 17,27 |
| Gef.: | | 68,03 | | 6,74 | | 17,33 |

(20) 5E-6-(3-(2-Cyano-3-benzyl-guanidino)phenyl)-6-(3-pyridyl)hex-5-ensäure
Schmelzpunkt: 138-140°C (Essigsäureethylester/tert.Butylmethylether)

| C₂₆H₂₅N₅O₂ (439,52) | | | | | | |
|---|---|---|---|---|---|---|
| Ber.: | C | 71,05 | H | 5,73 | N | 15,93 |
| Gef.: | | 71,04 | | 5,76 | | 15,94 |

(21) 5E-6-(3-(2-Cyano-3-(2-methylpropyl)guanidino)phenyl)-6-(3-pyridyl)hex-5-ensäure
Schmelzpunkt: 129°C (Zers., Essigsäureethylester/Diisopropylether)

| C₂₃H₂₇N₅O₂ (405,50) | | | | | | |
|---|---|---|---|---|---|---|
| Ber.: | C | 68,13 | H | 6,71 | N | 17,27 |
| Gef.: | | 68,03 | | 6,72 | | 17,08 |

(22) 5E-6-(3-(2-Cyano-3-neopentyl-guanidino)phenyl)-6-(3-pyridyl)hex-5-ensäure
Schmelzpunkt: 115-116°C (Essigsäureethylester/tert.Butylmethylether)

| C₂₄H₂₉N₅O₂ (419,53) | | | | | | |
|---|---|---|---|---|---|---|
| Ber.: | C | 68,71 | H | 6,97 | N | 16,69 |
| Gef.: | | 68,53 | | 7,07 | | 16,53 |

(23) 5E-6-(3-(2-Cyano-3-pentyl-guanidino)phenyl)-6-(3-pyridyl)hex-5-ensäure
Schmelzpunkt: 140-142°C (Essigsäureethylester/Isopropanol)

| C₂₄H₂₉N₅O₂ (419,53) | | | | | | |
|---|---|---|---|---|---|---|
| Ber.: | C | 68,71 | H | 6,97 | N | 16,69 |
| Gef.: | | 68,57 | | 7,11 | | 16,63 |

(24) 5E-6-(3-(2-Cyano-3,3-dimethyl-guanidino)phenyl)-6-(3-pyridyl)hex-5-ensäure
Herstellung analog Beispiel 2b durch Umsetzung mit Dimethylamin im Bombenrohr.
Schmelzpunkt: 194°C (Essigsäureethylester/Diisopropylether)

| C₂₁H₂₃N₅O₂ (377,45) | | | | | | |
|---|---|---|---|---|---|---|
| Ber.: | C | 66,83 | H | 6,14 | N | 18,55 |
| Gef.: | | 66,63 | | 6,25 | | 18,38 |

(25) 5E-6-(3-(2-Cyano-3-methyl-guanidino)phenyl)-6-(3-pyridyl)hex-5-ensäure
Herstellung analog Beispiel 2b durch Umsetzung mit Methylamin im Bombenrohr.
Schmelzpunkt: 120°C (Essigsäureethylester/Isopropanol)

| C₂₀H₂₁N₅O₂ (363,42) | | | | | | |
|---|---|---|---|---|---|---|
| Ber.: | C | 66,10 | H | 5,82 | N | 19,27 |
| Gef.: | | 66,00 | | 5,98 | | 19,35 |

(26) 5E-6-(3-(2-Cyanoguanidino)phenyl)-6-(3-pyridyl)hex-5-ensäure
2,2 g 6-(3-(2-Cyanimido-phenoxymethylenamino)phenyl)-6-(3-pyridyl)hex-5-ensäuremethylester und 4,8 g Ammoniumcarbonat werden in 40 ml Methanol 72 Stunden bei Raumtemperatur gerührt. Die Reaktionsmischung wird eingeengt und der Rückstand in Isopropanol durch Zugabe von Natronlauge analog Beispiel 2b verseift.
Schmelzpunkt: 174°C (Essigsäureethylester/Isopropanol)

| C₁₉H₁₉N₅O₂ (349,39) | | | | | | |
|---|---|---|---|---|---|---|
| Ber.: | C | 65,32 | H | 5,48 | N | 20,04 |
| Gef.: | | 65,17 | | 5,53 | | 19,87 |

(27) 5E-6-(3-(Cyanimido-morpholin-1-yl-methylenamino)phenyl)-6-(3-pyridyl)hex-5-ensäure
Schmelzpunkt: 189°C (Wasser/Isopropanol)

| C₂₃H₂₅N₅O₃ (419,49) | | | | | | |
|---|---|---|---|---|---|---|
| Ber.: | C | 65,86 | H | 6,01 | N | 16,70 |
| Gef.: | | 65,88 | | 5,96 | | 16,53 |

(28) 5E-6-(3-(2-Cyano-3-(2-dimethylaminoethyl)guanidino)phenyl)-6-(3-pyridyl)hex-5-ensäure
Schaum, R_{f}-Wert: 0,28 (Dichlormethan/Methanol = 9:1)

| C₂₃H₂₈N₆O₂ (420,51) | | | | | | |
|---|---|---|---|---|---|---|
| Ber.: | C | 62,94 | H | 6,90 | N | 19,17 |
| Gef.: | | 62,74 | | 6,76 | | 18,96 |

(29) 5E-6-(3-(2-Cyano-3-cyclohexylmethyl-guanidino)phenyl)-6-(3-pyridyl)hex-5-ensäure
Schmelzpunkt: 111°C (Essigsäureethylester/Isopropanol)

| C₂₆H₃₁N₅O₂ x 0,5 Essigsäureethylester (445,57) | | | | | | |
|---|---|---|---|---|---|---|
| Ber.: | C | 68,69 | H | 7,20 | N | 14,30 |
| Gef.: | | 68,55 | | 7,28 | | 14,41 |

(30) 5E-6-(3-(2-Cyano-3-(3-methylbutyl)guanidino)phenyl)-6-(3-pyridyl)hex-5-ensäure
Schmelzpunkt: 108°C (Zers., Essigsäureethylester/tert.Butylmethylether)

| C₂₄H₂₉N₅O₂ (419,53) | | | | | | |
|---|---|---|---|---|---|---|
| Ber.: | C | 68,71 | H | 6,97 | N | 16,69 |
| Gef.: | | 68,67 | | 7,09 | | 16,73 |

(31) 5E-6-(3-(2-Cyano-3-methoxy-guanidino)phenyl)-6-(3-pyridyl)hex-5-ensäure
Schmelzpunkt: 118°C (Zers., Essigsäureethylester/Isopropanol)

| C₂₀H₂₁N₅O₃ (379,42) | | | | | | |
|---|---|---|---|---|---|---|
| Ber.: | C | 63,31 | H | 5,58 | N | 18,46 |
| Gef.: | | 63,19 | | 5,53 | | 18,28 |

(32) 5E-6-(3-(2-Cyano-3-methoxy-3-methyl-guanidino)phenyl)-6-(3-pyridyl)hex-5-ensäure
Schmelzpunkt: 116°C (Essigsäureethylester/Diisopropylether)

| C₂₁H₂₃N₅O₃ (393,45) | | | | | | |
|---|---|---|---|---|---|---|
| Ber.: | C | 64,11 | H | 5,89 | N | 17,80 |
| Gef.: | | 64,21 | | 5,85 | | 17,62 |

(33) E/Z-5-(3-(2-Cyano-3-(2-methylpropyl)guanidino)phenyl)-5-(3-pyridyl)pent-4-ensäure
Schmelzpunkt: 189°C (Essigsäureethylester/Diisopropylether)

| C₂₂H₂₅N₅O₂ (391,47) | | | | | | |
|---|---|---|---|---|---|---|
| Ber.: | C | 67,50 | H | 6,44 | N | 17,89 |
| Gef.: | | 67,31 | | 6,48 | | 17,79 |

(34) E/Z-5-(3-(2-Cyano-3-tert.butyl-guanidino)phenyl)-5-(3-pyridyl)pent-4-ensäure
Schmelzpunkt: 146-148°C (Essigsäureethylester/Isopropanol)

| C₂₂H₂₅N₅O₂ (391,47) | | | | | | |
|---|---|---|---|---|---|---|
| Ber.: | C | 67,50 | H | 6,44 | N | 17,89 |
| Gef.: | | 67,32 | | 6,50 | | 17,68 |

(35) 6E-7-(3-(2-Cyano-3-cyclopropyl-guanidino)phenyl)-7-(3-pyridyl)hept-6-ensäure
Schaum, R_{f}-Wert: 0,24 (Kieselgel; Dichlormethan/Ethanol = 20:1)

| C₂₃H₂₅N₅O₂ (403,49) | | | | | | |
|---|---|---|---|---|---|---|
| Ber.: | C | 68,47 | H | 6,25 | N | 17,36 |
| Gef.: | | 68,40 | | 6,39 | | 17,20 |

(36) 6E-7-(3-(2-Cyano-3-cyclohexyl-guanidino)phenyl)-7-(3-pyridyl)hept-6-ensäure
Schaum, R_{f}-Wert: 0,26 (Kieselgel; Dichlormethan/Ethanol = 20:1)

| C₂₆H₃₁N₅O₂ (445,56) | | | | | | |
|---|---|---|---|---|---|---|
| Ber.: | C | 70,09 | H | 7,01 | N | 15,72 |
| Gef.: | | 70,17 | | 7,05 | | 15,52 |

(37) 6E-7-(3-(2-Cyano-3-tert.butyl-guanidino)phenyl)-7-(3-pyridyl)hept-6-ensäure
Schmelzpunkt: 96°C (Essigsäureethylester/Diisopropylether)

| C₂₄H₂₉N₅O₂ (419,53) | | | | | | |
|---|---|---|---|---|---|---|
| Ber.: | C | 68,71 | H | 6,97 | N | 16,69 |
| Gef.: | | 68,54 | | 7,08 | | 16,44 |

(38) 7E-8-(3-(2-Cyano-3-(2-methylpropyl)guanidino)phenyl)-8-(3-pyridyl)oct-7-ensäure
Schaum, R_{f}-Wert: 0,62 (Kieselgel; Dichlormethan/Ethanol = 9:1)

| C₂₅H₃₁N₅O₂ (433,55) | | | | | | |
|---|---|---|---|---|---|---|
| Ber.: | C | 69,26 | H | 7,21 | N | 16,15 |
| Gef.: | | 69,14 | | 7,30 | | 15,99 |

(39) 7E-8-(3-(2-Cyano-3-tert.butyl-guanidino)phenyl)-8-(3-pyridyl)oct-7-ensäure
Schmelzpunkt: 136-138°C (Essigsäureethylester)

| C₂₅H₃₁N₅O₂ (433,55) | | | | | | |
|---|---|---|---|---|---|---|
| Ber.: | C | 69,26 | H | 7,21 | N | 16,15 |
| Gef.: | | 69,04 | | 7,15 | | 16,17 |

(40) E/Z-6-(3-(2-Cyano-3-cyclopropyl-1-methyl-guanidino)phenyl)-6-(3-pyridyl)hex-5-ensäure
Schmelzpunkt: 158-160°C (Essigsäureethylester/Isopropanol)

| C₂₃H₂₅N₅O₂ (403,49) | | | | | | |
|---|---|---|---|---|---|---|
| Ber.: | C | 68,47 | H | 6,25 | N | 17,36 |
| Gef.: | | 68,34 | | 6,25 | | 17,26 |

(41) 5E-6-(3-(2-Cyano-3-cyclopropyl-guanidino)-4-methylphenyl)-6-(3-pyridyl)hex-5-ensäure
Schmelzpunkt: 156-158°C (Essigsäureethylester/Diisopropylether)

| C₂₅H₂₉N₅O₂ (431,54) | | | | | | |
|---|---|---|---|---|---|---|
| Ber.: | C | 69,58 | H | 6,77 | N | 16,23 |
| Gef.: | | 69,39 | | 6,93 | | 16,29 |

(42) 5E-6-(3-(2-Cyano-3-neopentyl-guanidino)-4-methylphenyl)-6-(3-pyridyl)hex-5-ensäure
Schmelzpunkt: 148-149°C (Essigsäureethylester/Diisopropylether)

| C₂₅H₃₁N₅O₂ (433,55) | | | | | | |
|---|---|---|---|---|---|---|
| Ber.: | C | 69,26 | H | 7,21 | N | 16,15 |
| Gef.: | | 69,08 | | 7,33 | | 16,24 |

(43) 5E-6-(3-(2-Cyano-3-propyl-guanidino)-4-methylphenyl)-6-(3-pyridyl)hex-5-ensäure
Schmelzpunkt: 169-171°C (Essigsäureethylester/Diisopropylether)

| C₂₃H₂₇N₅O₂ (405,50) | | | | | | |
|---|---|---|---|---|---|---|
| Ber.: | C | 68,13 | H | 6,71 | N | 17,27 |
| Gef.: | | 67,95 | | 6,87 | | 17,24 |

(44) 5E-6-(3-(3-tert.Butyl-2-cyano-guanidino)-4-methylphenyl)-6-(3-pyridyl)hex-5-ensäure
Schmelzpunkt: 127-129°C (Essigsäureethylester/Diisopropylether)

| C₂₄H₂₉N₅O₂ (419,53) | | | | | | |
|---|---|---|---|---|---|---|
| Ber.: | C | 68,71 | H | 6,97 | N | 16,69 |
| Gef.: | | 68,56 | | 7,05 | | 16,84 |

(45) 5E-6-(3-(2-Cyano-3-cyclopentyl-guanidino)-5-trifluormethylphenyl)-6-(3-pyridyl)hex-5-ensäure
Schmelzpunkt: 152°C (Essigsäureethylester/Diisopropylether)

| C₂₅H₂₆F₃N₅O₂ (485,51) | | | | | | |
|---|---|---|---|---|---|---|
| Ber.: | C | 61,85 | H | 5,40 | N | 14,42 |
| Gef.: | | 61,73 | | 5,50 | | 14,48 |

(46) 5E-6-(3-(2-Cyano-3-(2-methylpropyl)guanidino)-5-trifluormethylphenyl)-6-(3-pyridyl)hex-5-ensäure
Schmelzpunkt: 164°C (Essigsäureethylester)

| C₂₄H₂₆F₃N₅O₂ (473,50) | | | | | | |
|---|---|---|---|---|---|---|
| Ber.: | C | 60,88 | H | 5,53 | N | 14,79 |
| Gef.: | | 60,69 | | 5,47 | | 14,88 |

(47) 5E-6-(3-(2-Cyano-3-(exo-norborn-2-yl)guanidino)-5-trifluormethylphenyl)-6-(3-pyridyl)hex-5-ensäure
Schmelzpunkt: 144-145°C (Essigsäureethylester)

| C₂₇H₂₈F₃N₅O₂ (511,55) | | | | | | |
|---|---|---|---|---|---|---|
| Ber.: | C | 63,39 | H | 5,42 | N | 13,69 |
| Gef.: | | 63,25 | | 5,49 | | 13,58 |

(48) 5E-6-(3-(2-Cyano-3-cycloheptyl-guanidino)phenyl)-6-(3-pyridyl)hex-5-ensäure
Schmelzpunkt: 178°C (Zers., Essigsäureethylester/Diisopropylether)

| C₂₆H₃₁N₅O₂ (445,57) | | | | | | |
|---|---|---|---|---|---|---|
| Ber.: | C | 70,09 | H | 7,01 | N | 15,72 |
| Gef.: | | 69,96 | | 7,08 | | 15,52 |

(49) 5E-6-(3-(2-Cyano-3-cyclooctyl-guanidino)phenyl)-6-(3-pyridyl)hex-5-ensäure
Schmelzpunkt: 154°C (Zers., Essigsäureethylester/Diisopropylether)

| C₂₇H₃₃N₅O₂ (459,59) | | | | | | |
|---|---|---|---|---|---|---|
| Ber.: | C | 70,56 | H | 7,24 | N | 15,24 |
| Gef.: | | 70,39 | | 7,18 | | 15,14 |

(50) 5E-6-(3-(3-Adamant-1-yl)-2-cyano-guanidino)phenyl)-6-(3-pyridyl)hex-5-ensäure
Schmelzpunkt: 148°C (Zers., Isopropanol/Wasser)

| C₂₉H₃₃N₅O₂ (483,61) | | | | | | |
|---|---|---|---|---|---|---|
| Ber.: | C | 72,02 | H | 6,88 | N | 14,48 |
| Gef.: | | 71,86 | | 6,85 | | 14,45 |

### Beispiel 3

### 6-(3-(2-Cyano-3-isopropyl-guanidino)phenyl)-6-(3-pyridyl)hexansäure

### a) 6-(3-Acetylaminonhenyl)-6-(3-pyridyl)hexansäure

9,75 g 6-(3-Acetylaminophenyl)-6-(3-pyridyl)hex-5-ensäure werden in 33 ml 1N Natronlauge gelöst und nach Zugabe von 1 g 10%igem Palladium Kohle bei Raumtemperatur und 5 bar Wasserstoffdruck eine Stunde hydriert. Anschließend wird vom Katalysator abfiltriert, das Filtrat durch Zugabe von Zitronensäure auf pH 4-5 eingestellt und eingeengt. Der Rückstand wird dreimal mit Methanol/Ethanol (9:1) ausgekocht. Die vereinigten organischen Extrakte werden eingeengt und der Rückstand über eine Kieselgelsäule mit Dichlormethan/Methanol = 19:1 gereinigt.
Ausbeute: 73 % der Theorie,
Schaum, R_{f}-Wert: 0,72 (Kieselgel; Dichlormethan/Methanol = 9:1)

| c₁₉H₂₂N₂O₃ (326,40) | | | | | | |
|---|---|---|---|---|---|---|
| Ber.: | C | 69,92 | H | 6,79 | N | 8,58 |
| Gef.: | | 69,70 | | 6,72 | | 8,43 |

### b) 6-(3-Aminophenyl)-6-(3-pyridyl)hexansäuremethylester

6,9 g 6-(3-Acetylaminophenyl)-6-(3-pyridyl)hexansäure werden in 40 ml 9N methanolischer Salzsäure 24 Stunden bei Raumtemperatur gerührt. Die Reaktionslösung wird eingeengt, der Rückstand in Wasser aufgenommen und durch Zugabe von Natriumcarbonat alkalisch gestellt. Die wäßrige Phase wird mit Essigsäureethylester extrahiert. Der organische Extrakt wird mit Wasser gewaschen, getrocknet und eingeengt.
Ausbeute: 81 % der Theorie,
Öl, R_{f}-Wert: 0,52 (Kieselgel; Dichlormethan/Ethanol = 19:1)

| C₁₈H₂₂N₂O₂ (298,38) | | | | | | |
|---|---|---|---|---|---|---|
| Ber.: | C | 72,46 | H | 7,43 | N | 9,39 |
| Gef.: | | 72,28 | | 7,58 | | 9,23 |

### c) 6-(3-Cyanimido-phenoxymethylenamino)phenyl)-6-(3-pyridyl)hexansäuremethylester

5,1 g 6-(3-Aminophenyl)-6-(3-pyridyl)hexansäuremethylester und 4,1 g Diphenoxymethylencyanamid werden in 130 ml Isopropanol 4 Tage bei Raumtemperatur gerührt. Die Reaktionsmischung wird eingeengt und der Rückstand über eine Kieselgelsäule mit Dichlormethan/Ethanol = 19:1 gereinigt.
Ausbeute: 91 % der Theorie,
Harz, R_{f}-Wert: 0,54 (Kieselgel; Dichlormethan/Ethanol = 19:1)

| C₂₆H₂₆N₄O₃ (442,52) | | | | | | |
|---|---|---|---|---|---|---|
| Ber.: | C | 70,57 | H | 5,92 | N | 15,66 |
| Gef.: | | 70,41 | | 6,03 | | 15,68 |

### d) 6-(3-(2-Cyano-3-isopropyl-guanidino)phenyl)-6-(3-pyridyl)hexansäure

3,1 g 6-(3-Cyanimido-phenoxymethylenamino)phenyl)-6-(3-pyridyl)hexansäuremethylester und 5 ml Isopropylamin werden in 50 ml Isopropanol 3 Stunden unter Rückfluß erhitzt. Bei 50°C werden anschließend 10 ml 2N Natronlauge zugegeben und die Reaktionslösung wird 30 Minuten bei dieser Temperatur gerührt. Danach wird eingeengt, der Rückstand in Wasser aufgenommen und mit Essigsäureethylester gewaschen. Die wäßrige Phase wird durch Zugabe von Zitronensäure auf pH 4-5 eingestellt, der entstandene Niederschlag abgesaugt und aus Essigsäureethylester/Isopropanol umkristallisiert.
Ausbeute: 64 % der Theorie,
Schmelzpunkt: 168-169°C

| C₂₂H₂₇N₅O₂ (393,49) | | | | | | |
|---|---|---|---|---|---|---|
| Ber.: | C | 67,15 | H | 6,92 | N | 17,80 |
| Gef.: | | 67,12 | | 6,95 | | 17,87 |

### Analog Beispiel 3 wird folgende Verbindung erhalten:

6-(3-(2-Cyano-3-tert.butyl-guanidino)phenyl)-6-(3-pyridyl)hexansäure
Schmelzpunkt: 142°C (Essigsäureethylester/Isopropanol)

| C₂₃H₂₉N₅O₂ (407,51) | | | | | | |
|---|---|---|---|---|---|---|
| Ber.: | C | 67,79 | H | 7,17 | N | 17,19 |
| Gef.: | | 67,62 | | 7,25 | | 17,07 |

### Beispiel 4

### 5E-6-(3-(1-Neopentylamino-2-nitro-ethylenamino)phenyl)-6-(3-pyridyl)hex-5-ensäure

### a) 5E-6-(3-(1-Methylthio-2-nitro-ethylenamino)phenyl)-6-(3-pyridyl)hex-5-ensäuremethylester

3 g 5E-6-(3-Aminophenyl)-6-(3-pyridyl)hex-5-ensäuremethylester und 1,65 g 1,1-Bis-(methylthio)-2-nitroethen werden in 50 ml Isopropanol 20 Stunden unter Rückfluß erhitzt. Die Reaktionsmischung wird filtriert, das Filtrat wird eingeengt und der Rückstand über eine Kieselgelsäule mit Dichlormethan/Ethanol = 30:1 gereinigt. Die Produktfraktion wird eingeengt und der Rückstand aus tert.Butylmethylether umkristallisiert.
Ausbeute: 63 % der Theorie,
Schmelzpunkt: 84°C

| C₂₁H₂₃N₃O₄S (413,50) | | | | | | | | |
|---|---|---|---|---|---|---|---|---|
| Ber.: | C | 61,00 | H | 5,61 | N | 10,16 | S | 7,75 |
| Gef.: | | 60,99 | | 5,52 | | 10,23 | | 7,62 |

### b) 5E-6-(3-(1-Neopentylamino-2-nitro-ethylenamino)phenyl)-6-(3-pyridyl)hex-5-ensäure

2,1 g 5E-6-(3-(1-Methylthio-2-nitro-ethylenamino)phenyl)-6-(3-pyridyl)hex-5-ensäuremethylester und 2,4 ml Neopentylamin werden in 20 ml Isopropanol 5 Stunden unter Rückfluß erhitzt. Die Reaktionsmischung wird bei 60°C mit 10 ml 2N Natronlauge versetzt und 30 Minuten bei dieser Temperatur gerührt. Anschließend wird die Reaktionslösung eingeengt, der Rückstand in Wasser aufgenommen und die wäßrige Phase mit Essigsäureethylester gewaschen. Die wäßrige Phase wird durch Zugabe von Zitronensäure auf pH 4-5 eingestellt und mit Essigsäureethylester extrahiert. Der organische Extrakt wird eingeengt und der Rückstand aus Wasser/Isopropanol umkristallisiert.
Ausbeute: 70 % der Theorie,
Schmelzpunkt: 190-191°C

| C₂₄H₃₀N₄O₄ (438,53) | | | | | | |
|---|---|---|---|---|---|---|
| Ber.: | C | 65,73 | H | 6,90 | N | 12,78 |
| Gef.: | | 65,62 | | 6,98 | | 12,61 |

### Analog Beispiel 4 wird folgende Verbindung erhalten:

5E-6-(3-(1-Cyclohexylamino-2-nitro-ethylenamino)phenyl)-6-(3-pyridyl)hex-5-ensäure
Schmelzpunkt: 155-157°C (Essigsäureethylester/Isopropanol)

| C₂₅H₃₀N₄O₄ (450,54) | | | | | | |
|---|---|---|---|---|---|---|
| Ber.: | C | 66,65 | H | 6,71 | N | 12,44 |
| Gef.: | | 66,61 | | 6,71 | | 12,39 |

### Beispiel 5

### 5E-6-(3-(2,2-Dicyano-(2-methylpropylamino)ethylenamino)phenyl)-6-(3-pyridyl)hex-5-ensäure

### a) 5E-6-(3-(2,2-Dicyano-1-methylthio-ethylenamino)phenyl)-6-(3-pyridyl)hex-5-ensäuremethylester

13,4 g 5E-6-(3-(Aminophenyl)-6-(3-pyridyl)hex-5-ensäuremethylester und 7,7 g 2,2-Dicyano-1,1-bis(methylthio)ethen werden in 130 ml Isopropanol 6 Stunden unter Rückfluß erhitzt. Die Reaktionsmischung wird eingeengt und der Rückstand aus Essigsäureethylester/Diisopropylether umkristallisiert.
Ausbeute: 45 % der Theorie,
Schmelzpunkt: 125-127°C

| C₂₃H₂₂N₄O₂S (418,51) | | | | | | | | |
|---|---|---|---|---|---|---|---|---|
| Ber.: | C | 66,01 | H | 5,30 | N | 13,39 | S | 7,66 |
| Gef.: | | 65,97 | | 5,27 | | 13,49 | | 7,66 |

### b) 5E-6-(3-(2,2-Dicyano-1-(2-methylpropylamino)ethylenamino)phenyl)-6-(3-pyridyl)hex-5-ensäure

4 g 5E-6-(3-(2,2-Dicyano-1-methylthio-ethylenamino)phenyl)-6-(3-pyridyl)hex-5-ensäuremethylester und 10 ml 2-Methylpropylamin werden in 80 ml Isopropanol 4 Stunden unter Rückfluß erhitzt. Die Reaktionsmischung wird bei 40°C mit 10 ml 2N Natronlauge versetzt und eine Stunde bei dieser Temperatur gerührt. Anschließend wird eingeengt, der Rückstand in Wasser aufgenommen und mit Essigsäureethylester gewaschen. Die wäßrige Phase wird durch Zugabe von Zitronensäure auf pH 4-5 eingestellt und mit Essigsäureethylester extrahiert. Der organische Extrakt wird eingeengt und der Rückstand über eine Kieselgelsäule mit Dichlormethan/Ethanol = 29:1 gereinigt.
Ausbeute: 68 % der Theorie,
Schaum, R_{f}-Wert: 0,48 (Kieselgel; Dichlormethan/Ethanol = 19:1)

| C₂₅H₂₇N₅O₂ (429,52) | | | | | | |
|---|---|---|---|---|---|---|
| Ber.: | C | 69,91 | H | 6,34 | N | 16,30 |
| Gef.: | | 69,70 | | 6,23 | | 16,16 |

### Analog Beispiel 5 werden folgende Verbindungen erhalten:

(1) 5E-6-(3-(2,2-Dicyano-1-isopropylamino-ethylenamino)phenyl)-6-(3-pyridyl)hex-5-ensäure
Schmelzpunkt: 164-165°C (Essigsäureethylester/Diethylether)

| C₂₄H₂₅N₅O₂ (415,49) | | | | | | |
|---|---|---|---|---|---|---|
| Ber.: | C | 69,38 | H | 6,06 | N | 16,86 |
| Gef.: | | 69,22 | | 6,11 | | 16,68 |

(2) 5E-6-(3-(2,2-Dicyano-1-(3-methylbutylamino)-ethylenamino)phenyl)-6-(3-pyridyl)hex-5-ensäure
Schmelzpunkt: 139°C (Essigsäureethylester/Diisopropylether)

| C₂₆H₂₉N₅O₂ (443,55) | | | | | | |
|---|---|---|---|---|---|---|
| Ber.: | C | 70,41 | H | 6,59 | N | 15,79 |
| Gef.: | | 70,47 | | 6,72 | | 15,61 |

(3) 5E-6-(3-(2,2-Dicyano-1-cyclopentylamino-ethylenamino)phenyl)-6-(3-pyridyl)hex-5-ensäure
Schmelzpunkt: 150°C (Essigsäureethylester/Diisopropylether)

| C₂₆H₂₇N₅O₂ (441,53) | | | | | | |
|---|---|---|---|---|---|---|
| Ber.: | C | 70,73 | H | 6,16 | N | 15,86 |
| Gef.: | | 70,55 | | 6,27 | | 15,90 |

(4) 5E-6-(3-(2,2-Dicyano-1-neopentylamino-ethylenamino)phenyl)-6-(3-pyridyl)hex-5-ensäure
Schmelzpunkt: 116°C (Essigsäureethylester/Diisopropylether)

| C₂₆H₂₉N₅O₂ (443,55) | | | | | | |
|---|---|---|---|---|---|---|
| Ber.: | C | 70,41 | H | 6,59 | N | 15,79 |
| Gef.: | | 70,29 | | 6,63 | | 15,65 |

(5) 5E-6-(3-(2,2-Dicyano-1-cyclopropylamino-ethylenamino)phenyl)-6-(3-pyridyl)hex-5-ensäure
Schmelzpunkt: 173°C (Essigsäureethylester/tert.butylmethylether)

| C₂₄H₂₃N₅O₂ (413,48) | | | | | | |
|---|---|---|---|---|---|---|
| Ber.: | C | 69,72 | H | 5,61 | N | 16,94 |
| Gef.: | | 69,57 | | 5,77 | | 17,05 |

(6) 5E-6-(3-(2,2-Dicyano-1-benzylamino-ethylenamino)phenyl)-6-(3-pyridyl)hex-5-ensäure
Schmelzpunkt: 154°C (Isopropanol/Wasser)

| C₂₈H₂₅N₅O₂ (463,54) | | | | | | |
|---|---|---|---|---|---|---|
| Ber.: | C | 72,55 | H | 5,44 | N | 15,11 |
| Gef.: | | 72,42 | | 5,59 | | 15,02 |

(7) 5E-6-(3-(2,2-Dicyano-1-propylamino-ethylenamino)phenyl)-6-(3-pyridyl)hex-5-ensäure
Schmelzpunkt: 116°C (Essigsäureethylester/Diisopropylether)

| C₂₄H₂₅N₅O₂ (415,49) | | | | | | |
|---|---|---|---|---|---|---|
| Ber.: | C | 69,38 | H | 6,06 | N | 16,86 |
| Gef.: | | 69,25 | | 6,13 | | 16,77 |

(8) 5E-6-(3-(2,2-Dicyano-1-methylamino-ethylenamino)phenyl)-6-(3-pyridyl)hex-5-ensäure
Herstellung analog Beispiel 5b durch Umsetzung mit Methylamin im Bombenrohr.
Schmelzpunkt: 178°C (Essigsäureethylester)

| C₂₂H₂₁N₅O₂ (387,44) | | | | | | |
|---|---|---|---|---|---|---|
| Ber.: | C | 68,20 | H | 5,46 | N | 18,08 |
| Gef.: | | 68,10 | | 5,58 | | 18,12 |

(9) 5E-6-(3-(2,2-Dicyano-1-dimethylamino-ethylenamino)phenyl)-6-(3-pyridyl)hex-5-ensäure
Herstellung analog Beispiel 5b durch Umsetzung mit Dimethylamin im Bombenrohr.
Schmelzpunkt: 147°C (Essigsäureethylester)

| C₂₃H₂₃N₅O₂ (401,47) | | | | | | |
|---|---|---|---|---|---|---|
| Ber.: | C | 68,81 | H | 5,77 | N | 17,44 |
| Gef.: | | 68,75 | | 5,83 | | 17,28 |

(10) 5E-6-(3-(2,2-Dicyano-1-(exo-norborn-2-ylamino)ethylenamino)phenyl)-6-(3-pyridyl)hex-5-ensäure
Schmelzpunkt: 154-156°C (tert.Butylmethylether)

| C₂₈H₂₉N₅O₂ (467,57) | | | | | | |
|---|---|---|---|---|---|---|
| Ber.: | C | 71,93 | H | 6,25 | N | 14,98 |
| Gef.: | | 71,81 | | 6,31 | | 14,87 |

(11) 5E-6-(3-(2,2-Dicyano-1-cyclooctylamino-ethylenamino)phenyl)-6-(3-pyridyl)hex-5-ensäure
Schmelzpunkt: 167-169°C (Essigsäureethylester)

| C₂₉H₃₃N₅O₂ (483,61) | | | | | | |
|---|---|---|---|---|---|---|
| Ber.: | C | 72,02 | H | 6,88 | N | 14,48 |
| Gef.: | | 72,00 | | 6,94 | | 14,40 |

(12) 5E-6-(3-(2,2-Dicyano-1-cycloheptylamino-ethylenamino)phenyl)-6-(3-pyridyl)hex-5-ensäure
Schmelzpunkt: 140-142°C (Essigsäureethylester)

| C₂₈H₃₁N₅O₂ (469,59) | | | | | | |
|---|---|---|---|---|---|---|
| Ber.: | C | 71,62 | H | 7,07 | N | 14,91 |
| Gef.: | | 71,49 | | 7,16 | | 14,84 |

(13) 5E-6-(3-(2,2-Dicyano-1-cyclopentylamino-ethylenamino)-5-trifluormethyl-phenyl)-6-(3-pyridyl)hex-5-ensäure
Schmelzpunkt: 118°C (Essigsäureethylester/Diisopropylether)

| C₂₇H₂₈F₃N₅O₂ (509,54) | | | | | | |
|---|---|---|---|---|---|---|
| Ber.: | C | 63,65 | H | 5,14 | N | 13,74 |
| Gef.: | | 63,50 | | 5,25 | | 13,57 |

### Beispiel 6

### 5E-6-(3-(2,2-Dicyano-1-tert.butylamino-ethylenamino)phenyl)-6-(3-pyridyl)hex-5-ensäure

2,1 g 5E-6-(3-(2,2-Dicyano-1-methylthio-ethylenamino)phenyl)-6-(3-pyridyl)hex-5-ensäuremethylester werden in 100 ml Dichlormethan gelöst. Dazu gibt man 1,9 g 3-Chlorperbenzoesäure und rührt eine Stunde bei Raumtemperatur. Anschließend wird die Reaktionsmischung mit 10 ml tert.Butylamin versetzt und 12 Stunden bei Raumtemperatur gerührt. Die Reaktionsmischung wird mit Wasser gewaschen, eingeengt, der Rückstand in 20 ml Ethanol und 20 ml 1N Natronlauge aufgenommen und 3 Stunden auf 50°C erwärmt. Die Reaktionslösung wird eingeengt, der Rückstand in Wasser aufgenommen und mit Essigsäureethylester gewaschen. Die wäßrige Phase wird durch Zugabe von Zitronensäure auf pH 4-5 eingestellt und mit Essigsäureethylester extrahiert. Die organische Phase wird eingeengt und der Rückstand über eine Kieselgelsäule mit Dichlormethan/Ethanol = 20:1 gereinigt. Die Produktfraktion wird eingeengt und der Rückstand aus Essigsäureethylester/Diisopropylether umkristallisiert.
Ausbeute: 43 % der Theorie,
Schmelzpunkt: 188-189°C

| C₂₅H₂₇N₅O₂ (429,52) | | | | | | |
|---|---|---|---|---|---|---|
| Ber.: | C | 69,91 | H | 6,34 | N | 16,30 |
| Gef.: | | 69,71 | | 6,37 | | 16,18 |

### Analog Beispiel 6 wird folgende Verbindung erhalten:

(1) 5E-6-(3-(1-Adamant-1-ylamino-2,2-dicyano-ethylenamino)phenyl-6-(3-pyridyl)hex-5-ensäure
Schmelzpunkt: 142°C (Zers., Isopropanol/Diisopropylether)

| C₃₁H₃₃N₅O₂ (507,64) | | | | | | |
|---|---|---|---|---|---|---|
| Ber.: | C | 73,35 | H | 6,55 | N | 13,80 |
| Gef.: | | 73,22 | | 6,61 | | 13,71 |

(2) 5E-6-(3-(2,2-Dicyano-1-diethylamino-ethylenamino)phenyl-6-(3-pyridyl)hex-5-ensäure
Schmelzpunkt: 167-168°C (Essigsäureethylester/Diisopropylether)

| C₂₅H₂₇N₅O₂ (429,52) | | | | | | |
|---|---|---|---|---|---|---|
| Ber.: | C | 69,91 | H | 6,34 | N | 16,30 |
| Gef.: | | 69,73 | | 6,46 | | 16,24 |

### Beispiel 7

### E/Z-6-(4-(2-(2-Cyano-3-tert.butyl-guanidino)ethyl)phenyl)-6-(3-pyridyl)hex-5-ensäure

### a) E/Z-6-(4-(2-Cyanimido-phenoxymethylenamino)ethyl)phenyl)-6-(3-pyridyl)hex-5-ensäuremethylester

26,5 g E/Z-6-(4-(2-(Acetylamino)ethyl)phenyl)-6-(3-pyridyl)hex-5-ensäure werden in 200 ml 6N Salzsäure 12 Stunden unter Rückfluß erhitzt. Die Reaktionslösung wird eingeengt, der Rückstand in 200 ml 3N methanolischer Salzsäure aufgenommen und 12 Stunden bei Raumtemperatur gerührt. Die Reaktionslösung wird eingeengt, der Rückstand in Wasser gelöst und mit Essigsäureethylester gewaschen. Die wäßrige Phase wird bei 0°C durch Zugabe von konz. Ammoniak alkalisch gestellt und mit Dichlormethan extrahiert. Der organische Extrakt wird mit Wasser gewaschen, getrocknet und eingeengt. Der Rückstand wird zusammen mit 14,9 g Diphenoxymethylencyanamid in 300 ml Isopropanol 14 Stunden bei Raumtemperatur gerührt. Das Lösungsmittel wird abgezogen und der Rückstand über eine Kieselgelsäule mit Dichlormethan/Ethanol = 30:1 gereinigt.
Ausbeute: 80 % der Theorie,
Öl, R_{f}-Wert: 0,72 (Kieselgel; Dichlormethan/Ethanol = 20:1)

| C₂₈H₂₈N₄O₃ (468,56) | | | | | | |
|---|---|---|---|---|---|---|
| Ber.: | C | 71,78 | H | 6,02 | N | 11,96 |
| Gef.: | | 71,64 | | 6,08 | | 11,85 |

### b) E/Z-6-(4-(2-(2-Cyano-3-tert.butyl-guanidino)ethyl)phenyl)-6-(3-pyridyl)hex-5-ensäure

2,35 g E/Z-6-(4-(2-Cyanimido-phenoxymethylenamino)ethyl)phenyl)-6-(3-pyridyl)hex-5-ensäuremethylester und 3 ml tert.Butylamin werden in 40 ml Isopropanol 3 Stunden unter Rückfluß gekocht. Anschließend wird die Reaktionsmischung bei 50°C mit 10 ml 2N Natronlauge versetzt und 30 Minuten bei dieser Temperatur gerührt. Die Reaktionsmischung wird eingeengt, der Rückstand in Wasser aufgenommen und mit Essigsäureethylester gewaschen. Die wäßrige Phase wird durch Zugabe von Zitronensäure auf pH 4-5 eingestellt und mit Essigsäureethylester extrahiert. Der organische Extrakt wird mit Wasser gewaschen, eingeengt und der Rückstand über eine Kieselgelsäule mit Dichlormethan/Ethanol = 20:1 gereinigt.
Ausbeute: 55 % der Theorie,
Schaum, R_{f}-Wert: 0,64 (Dichlormethan/Ethanol = 9:1)

| C₂₅H₃₁N₅O₂ (433,55) | | | | | | |
|---|---|---|---|---|---|---|
| Ber.: | C | 69,26 | H | 7,21 | N | 16,15 |
| Gef.: | | 69,27 | | 7,29 | | 15,95 |

### Analog Beispiel 7 wird folgende Verbindung erhalten:

E/Z-6-(4-(2-(2-Cyano-3-(2-methylpropyl)guanidino)ethyl)phenyl)-6-(3-pyridyl)hex-5-ensäure
Harz, R_{f}-Wert: 0,48 (Dichlormethan/Ethanol = 9:1)

| C₂₅H₃₁N₅O₂ (433,55) | | | | | | |
|---|---|---|---|---|---|---|
| Ber.: | C | 69,26 | H | 7,21 | N | 16,15 |
| Gef.: | | 69,17 | | 7,26 | | 16,17 |

### Beispiel 8

### 5E-6-(3-(2,2-Dicyano-1-(2-methylpropylamino)-ethylenamino)phenyl)-6-(3-pyridyl)hex-5-ensäuremethylamid

1,1 g 5E-6-(3-(2,2-Dicyano-1-(2-methylpropylamino)-ethylenamino)phenyl)-6-(3-pyridyl)hex-5-ensäure werden in 20 ml absolutem Tetrahydrofuran gelöst. Dazu gibt man 0,5 g Carbonyldiimidazol, rührt solange bis die Gasentwicklung abgeklungen ist und fügt anschließend 0,09 g Methylamin zu. Die Reaktionsmischung wird 12 Stunden bei Raumtemperatur gerührt, danach mit 2 ml Wasser versetzt und eingeengt. Der Rückstand wird in Essigsäureethylester aufgenommen. Der organische Extrakt wird mit Wasser gewaschen, getrocknet, eingeengt und der Rückstand über eine Kieselgelsäule mit Dichlormethan/Ethanol = 14:1 gereinigt.
Ausbeute: 60 % der Theorie,
Schaum, R_{f}-Wert: 0,61 (Kieselgel; Dichlormethan/Ethanol = 9:1)

| C₂₆H₃₀N₆O (442,56) | | | | | | |
|---|---|---|---|---|---|---|
| Ber.: | C | 70,56 | H | 6,83 | N | 18,99 |
| Gef.: | | 70,39 | | 6,86 | | 18,82 |

### Analog Beispiel 8 werden folgende Verbindungen erhalten:

(1) 5E-6-(3-(2,2-Dicyano-1-(2-methylpropylamino)-ethylenamino)phenyl)-6-(3-pyridyl)hex-5-ensäuredimethylamid
Schaum, R_{f}-Wert: 0,63 (Kieselgel; Dichlormethan/Ethanol = 9:1)

| C₂₇H₃₂N₆O (456,59) | | | | | | |
|---|---|---|---|---|---|---|
| Ber.: | C | 71,03 | H | 7,06 | N | 18,41 |
| Gef.: | | 70,83 | | 7,17 | | 18,43 |

(2) 5E-6-(3-(2,2-Dicyano-1-(2-methylpropylamino)-ethylenamino)phenyl)-6-(3-pyridyl)hex-5-ensäuremethylester
Schmelzpunkt: 127-129°C (tert.Butylmethylether)

| C₂₆H₂₉N₅O₂ (443,55) | | | | | | |
|---|---|---|---|---|---|---|
| Ber.: | C | 70,41 | H | 6,59 | N | 15,79 |
| Gef.: | | 70,45 | | 6,62 | | 15,83 |

(3) 5E-6-(3-(2,2-Dicyano-1-isopropylamino-ethylenamino)phenyl)-6-(3-pyridyl)hex-5-ensäuremethylester
Schmelzpunkt: 159°C (Diisopropylether)

| C₂₅H₂₇N₅O₂ (429,52) | | | | | | |
|---|---|---|---|---|---|---|
| Ber.: | C | 69,91 | H | 6,34 | N | 16,31 |
| Gef.: | | 69,87 | | 6,41 | | 16,48 |

(4) 5E-6-(3-(2,2-Dicyano-1-isopropylamino-ethylenamino)phenyl)-6-(3-pyridyl)hex-5-ensäure-(2-methylpropyl)ester
Öl, R_{f}-Wert: 0,53 (Kieselgel; Dichlormethan/Ethanol = 20:1)

| C₂₈H₃₃N₅O₂ (471,61) | | | | | | |
|---|---|---|---|---|---|---|
| Ber.: | C | 71,31 | H | 7,05 | N | 14,85 |
| Gef.: | | 71,10 | | 7,16 | | 15,09 |

(5) 5E-6-(3-(2,2-Dicyano-1-isopropylamino-ethylenamino)phenyl)-6-(3-pyridyl)hex-5-ensäureisopropylester
Schmelzpunkt: 119°C (Essigsäureethylester/Diisopropylether)

| C₂₇H₃₁N₅O₂ (457,58) | | | | | | |
|---|---|---|---|---|---|---|
| Ber.: | C | 70,87 | H | 6,83 | N | 15,31 |
| Gef.: | | 70,65 | | 6,99 | | 15,35 |

(6) 5E-6-(3-(2-Cyano-3-(2-methylpropyl)guanidino)phenyl)-6-(3-pyridyl)hex-5-ensäureisopropylester
Öl, R_{f}-Wert: 0,35 (Kieselgel; Dichlormethan/Ethanol = 20:1)

| C₂₄H₂₉N₅O₂ (419,53) | | | | | | |
|---|---|---|---|---|---|---|
| Ber.: | C | 68,71 | H | 6,97 | N | 16,69 |
| Gef.: | | 68,52 | | 7,12 | | 16,50 |

(7) 5E-6-(3-(2-Cyano-3-(2-methylpropyl)guanidino)phenyl)-6-(3-pyridyl)hex-5-ensäure-(2-methylpropyl)ester
Öl, R_{f}-Wert: 0,38 (Kieselgel; Dichlormethan/Ethanol = 20:1)

| C₂₇H₃₅N₅O₂ (461,61) | | | | | | |
|---|---|---|---|---|---|---|
| Ber.: | C | 70,25 | H | 7,64 | N | 15,17 |
| Gef.: | | 70,04 | | 7,78 | | 14,98 |

(8) 5E-6-(3-(2-Cyano-3-(2-methylpropyl)guanidino)phenyl)-6-(3-pyridyl)hex-5-ensäureisopropylester
Öl, R_{f}-Wert: 0,37 (Kieselgel; Dichlormethan/Ethanol = 20:1)

| C₂₆H₃₃N₅O₂ (447,58) | | | | | | |
|---|---|---|---|---|---|---|
| Ber.: | C | 69,77 | H | 7,43 | N | 15,65 |
| Gef.: | | 69,65 | | 7,55 | | 15,51 |

(9) 5E-6-(3-(2,2-Dicyano-1-isopropylamino-ethylenamino)phenyl)-6-(3-pyridyl)hex-5-ensäureamid
Schmelzpunkt: 123°C (Essigsäureethylester/Diisopropylether)

| C₂₄H₂₆N₆O (414,50) | | | | | | |
|---|---|---|---|---|---|---|
| Ber.: | C | 69,55 | H | 6,32 | N | 20,28 |
| Gef.: | | 69,46 | | 6,43 | | 20,42 |

(10) 5E-6-(3-(2-Cyano-3-(2-methylpropyl)guanidino)phenyl)-6-(3-pyridyl)hex-5-ensäurecyclohexylester
Öl, R_{f}-Wert: 0,42 (Kieselgel; Dichlormethan/Ethanol = 20:1)

| C₂₉H₃₇N₅O₂ (487,65) | | | | | | |
|---|---|---|---|---|---|---|
| Ber.: | C | 71,43 | H | 7,65 | N | 14,36 |
| Gef.: | | 71,38 | | 7,68 | | 14,18 |

### Beispiel 9

### 5E-6-(3-(3-Neopentyl-2-phenylsulfonyl-guanidino)phenyl)-6-(3-pyridyl)hex-5-ensäure

### a) Diphenoxymethylen-phenylsulfonamid

13,5 g Dichlor-diphenoxymethan und 17,3 g Phenylsulfonamid werden in 160 ml Essigsäureethylester 48 Stunden unter Rückfluß erhitzt. Die Reaktionsmischung wird eingeengt, der Rückstand in Wasser aufgenommen und durch Zugabe von Natriumhydrogencarbonat auf pH 8 eingestellt. Die wäßrige Phase wird mit Essigsäureethylester extrahiert. Der organische Extrakt wird eingeengt, der Rückstand in Dichlormethan erhitzt und filtriert. Das Filtrat wird eingeengt und der Rückstand über eine Kieselgelsäule mit Dichlormethan gereinigt. Die Produktfraktion wird eingeengt und der Rückstand aus Essigsäureethylester/Diisopropylether umkristallisiert.
Ausbeute: 26 % der Theorie,
Schmelzpunkt: 121-122°C

| C₁₉H₁₅NO₄S (353,40) | | | | | | | | |
|---|---|---|---|---|---|---|---|---|
| Ber.: | C | 64,58 | H | 4,28 | N | 3,96 | S | 9,07 |
| Gef.: | | 64,60 | | 4,41 | | 3,94 | | 8,94 |

### Analog Beispiel 9a wird folgende Verbindung erhalten:

Diphenoxymethylen-methylsulfonamid
Schmelzpunkt: 124°C (Essigsäureethylester/Diisopropylether)

| C₁₄H₁₃NO₄S (291,33) | | | | | | | | |
|---|---|---|---|---|---|---|---|---|
| Ber.: | C | 57,72 | H | 4,50 | N | 4,81 | S | 11,01 |
| Gef.: | | 57,62 | | 4,58 | | 4,87 | | 11,10 |

### b) 5E-6-(3-(Phenylsulfonimido-phenoxymethylenamino)phenyl)-6-(3-pyridyl)hex-5-ensäuremethylester

3,5 g Diphenoxymethylen-phenylsulfonamid und 3 g 5E-6-(3-Aminophenyl)-6-(3-pyridyl)hex-5-ensäuremethylester werden in 60 ml Isopropanol 48 Stunden bei Raumtemperatur gerührt. Die Reaktionsmischung wird eingeengt und der Rückstand über eine Kieselgelsäule mit Dichlormethan gereinigt. Die Produktfraktion wird eingeengt und der Rückstand aus Diethylether/Petrolether umkristallisiert.
Ausbeute: 86 % der Theorie,
Schmelzpunkt: 90-92°C

| C₃₁H₂₉N₃O₅S (555,65) | | | | | | | | |
|---|---|---|---|---|---|---|---|---|
| Ber.: | C | 67,01 | H | 5,26 | N | 7,56 | S | 5,77 |
| Gef.: | | 66,82 | | 5,35 | | 7,63 | | 5,81 |

### Analog Beispiel 9b wurde folgende Verbindung erhalten:

5E-6-(3-(Methylsulfonimido-phenoxymethylenamino)phenyl)-6-(3-pyridyl)hex-5-ensäuremethylester
Schmelzpunkt: 101-102°C (Essigsäureethylester/Petrolether)

| C₂₆H₂₇N₃O₅S (493,58) | | | | | | | | |
|---|---|---|---|---|---|---|---|---|
| Ber.: | C | 63,27 | H | 5,51 | N | 8,51 | S | 6,50 |
| Gef.: | | 63,39 | | 5,58 | | 8,56 | | 6,59 |

### c) 5E-6-(3-(3-Neopentyl-2-phenylsulfonyl-guanidino)phenyl)-6-(3-pyridyl)hex-5-ensäure

2.4 g 5E-6-(3-Phenylsulfonimido-phenoxymethylenamino)phenyl)-6-(3-pyridyl)hex-5-ensäuremethylester und 2,4 ml Neopentylamin werden in 40 ml Isopropanol eine Stunde unter Rückfluß erhitzt. Man läßt auf 50°C abkühlen, versetzt die Reaktionsmischung mit 10 ml 2N Natronlauge und rührt 30 Minuten bei 50°C . Die Reaktionsmischung wird eingeengt, der Rückstand in Wasser aufgenommen und mit Essigsäureethylester extrahiert. Die wäßrige Phase wird durch Zugabe von Zitronensäure auf pH 4-5 eingestellt und mit Essigsäureethylester extrahiert. Der organische Extrakt wird eingeengt und der Rückstand aus Essigsäureethylester/Isopropanol umkristallisiert.
Ausbeute: 91 % der Theorie,
Schmelzpunkt: 159-160°C

| C₂₉H₃₄N₄O₄S (534,68) | | | | | | | | |
|---|---|---|---|---|---|---|---|---|
| Ber.: | C | 65,15 | H | 6,41 | N | 10,48 | S | 6,00 |
| Gef.: | | 65,05 | | 6,52 | | 10,48 | | 6,04 |

### Analog Beispiel 9c werden folgende Verbindungen erhalten:

(1) 5E-6-(3-(3-tert.Butyl-2-phenylsulfonyl-guanidino)phenyl)-6-(3-pyridyl)hex-5-ensäure
Schaum, R_{f}-Wert: 0,20 (Dichlormethan/Ethanol = 30:1)

| C₂₈H₃₂N₄O₄S (520,65) | | | | | | | | |
|---|---|---|---|---|---|---|---|---|
| Ber.: | C | 64,59 | H | 6,19 | N | 10,76 | S | 6,16 |
| Gef.: | | 64,39 | | 6,27 | | 10,61 | | 6,13 |

(2) 5E-6-(3-(3-(2-Methylpropyl)-2-phenylsulfonyl-guanidino)phenyl)-6-(3-pyridyl)hex-5-ensäure
Schmelzpunkt: 124-125°C (Essigsäureethylester)

| C₂₈H₃₂N₄O₄S (520,65) | | | | | | | | |
|---|---|---|---|---|---|---|---|---|
| Ber.: | C | 64,59 | H | 6,19 | N | 10,76 | S | 6,16 |
| Gef.: | | 64,45 | | 6,24 | | 10,59 | | 6,11 |

(3) 5E-6-(3-(3-Neopentyl-2-methylsulfonyl-guanidino)phenyl)-6-(3-pyridyl)hex-5-ensäure
Schmelzpunkt: 143-144°C (Essigsäureethylester/Isopropanol)

| C₂₄H₃₂N₄O₄S (472,61) | | | | | | | | |
|---|---|---|---|---|---|---|---|---|
| Ber.: | C | 60,99 | H | 6,83 | N | 11,86 | S | 6,78 |
| Gef.: | | 60,87 | | 6,95 | | 11,79 | | 6,73 |

(4) 5E-6-(3-(3-(2-Methylpropyl)-2-methylsulfonyl-guanidino)phenyl)-6-(3-pyridyl)hex-5-ensäure
Schmelzpunkt: 158°C (Essigsäureethylester/Isopropanol)

| C₂₃H₃₀N₄O₄S (458,58) | | | | | | | | |
|---|---|---|---|---|---|---|---|---|
| Ber.: | C | 60,24 | H | 6,59 | N | 12,22 | S | 6,99 |
| Gef.: | | 60,15 | | 6,65 | | 12,08 | | 6,92 |

(5) 5E-6-(3-(3-Cyclopentyl-2-methylsulfonyl-guanidino)phenyl)-6-(3-pyridyl)hex-5-ensäure
Schmelzpunkt: 169-170°C (Essigsäureethylester/Isopropanol)

| C₂₄H₃₀N₄O₄S (470,59) | | | | | | | | |
|---|---|---|---|---|---|---|---|---|
| Ber.: | C | 61,26 | H | 6,43 | N | 11,91 | S | 6,81 |
| Gef.: | | 61,41 | | 6,45 | | 12,10 | | 6,90 |

(6) 5E-6-(3-(3-tert.Butyl-2-methylsulfonyl-guanidino)phenyl)-6-(3-pyridyl)hex-5-ensäure
Schmelzpunkt:150-151°C(Essigsäureethylester/Diisopropylether)

| C₂₃H₃₀N₄O₄S (458,58) | | | | | | | | |
|---|---|---|---|---|---|---|---|---|
| Ber.: | C | 60,24 | H | 6,59 | N | 12,22 | S | 6,99 |
| Gef.: | | 60,33 | | 6,62 | | 12,35 | | 7,04 |

(7) 5E-6-(3-(3-Cyclopentyl-2-phenylsulfonyl-guanidino)phenyl)-6-(3-pyridyl)hex-5-ensäure
Schmelzpunkt: 116-117°C (Essigsäureethylester/Diisopropylether)

| C₂₉H₃₂N₄O₄S (532,66) | | | | | | | | |
|---|---|---|---|---|---|---|---|---|
| Ber.: | C | 65,39 | H | 6,06 | N | 10,52 | S | 6,02 |
| Gef.: | | 65,20 | | 6,08 | | 10,39 | | 6,19 |

### Beispiel 10

### 5E-6-(3-(2-Amidosulfonyl-3-(2-methylpropyl)guanidino)phenyl)-6-(3-pyridyl)hex-5-ensäure

### a) 5E-6-(3-(Sulfamoylimido-phenoxymethylenamino)phenyl)-6-(3-pyridyl)hex-5-ensäuremethylester

2,4 g N-Sulfamoyl-diphenyl-imidocarbonat und 3 g 5E-6-(3-Aminophenyl)-6-(3-pyridyl)hex-5-ensäuremethylester werden in 60 ml Isopropanol gelöst und 36 Stunden bei Raumtemperatur gerührt. Das Lösungsmittel wird abgezogen und der Rückstand über eine Kieselgelsäule mit Dichlormethan/Ethanol = 40:1 gereinigt.
Ausbeute: 89 % der Theorie,
Öl, R_{f}-Wert: 0,59 (Kieselgel; Dichlormethan/Ethanol = 20:1)

| C₂₅H₂₆N₄O₅S (494,57) | | | | | | | | |
|---|---|---|---|---|---|---|---|---|
| Ber.: | C | 60,71 | H | 5,30 | N | 11,33 | S | 6,48 |
| Gef.: | | 60,55 | | 5,43 | | 11,18 | | 6,39 |

### b) 5E-6-(3-(2-Amidosulfonyl-3-(2-methylpropyl)guanidino)phenyl)-6-(3-pyridyl)hex-5-ensäure

2,2 g 5E-6-(3-(Sulfamoylimido-phenoxymethylenamino)phenyl)-6-(3-pyridyl)hex-5-ensäuremethylester und 5 ml 2-Methylpropylamin werden in 50 ml Isopropanol 4 Stunden unter Rückfluß gekocht. Man läßt auf 50°C abkühlen, versetzt die Reaktionsmischung mit 10 ml 2N Natronlauge und rührt 60 Minuten bei 50°C. Die Reaktionsmischung wird eingeengt, der Rückstand in Wasser aufgenommen und mit Essigsäureethylester extrahiert. Die wäßrige Phase wird durch Zugabe von Zitronensäure auf pH 4-5 eingestellt und mit Essigsäureethylester extrahiert. Der organische Extrakt wird eingeengt und der Rückstand über eine Kieselgelsäule mit Dichlormethan/Ethanol = 30:1 gereinigt. Die Produktfraktion wird eingeengt und der Rückstand aus Essigsäureethylester/Diisopropylether umkristallisiert.
Ausbeute: 38 % der Theorie,
Schmelzpunkt: 98°C (Zers.)

| C₂₂H₂₉N₅O₄S (459,57) | | | | | | | | |
|---|---|---|---|---|---|---|---|---|
| Ber.: | C | 57,50 | H | 6,36 | N | 15,24 | S | 6,98 |
| Gef.: | | 57,62 | | 6,51 | | 15,38 | | 7,05 |

### Analog Beispiel 10b werden folgende Verbindungen erhalten:

(1) 5E-6-(3-(2-Amidosulfonyl-3-neopentyl-guanidino)phenyl-6-(3-pyridyl)hex-5-ensäure
Schmelzpunkt: 136-140°C (Essigsäureethylester/Diisopropylether)

| C₂₃H₃₁N₅O₄S (473,60) | | | | | | | | |
|---|---|---|---|---|---|---|---|---|
| Ber.: | C | 58,33 | H | 6,60 | N | 14,79 | S | 6,77 |
| Gef.: | | 58,28 | | 6,73 | | 14,53 | | 6,50 |

(2) 5E-6-(3-(2-Amidosulfonyl-3-cyclopentyl-guanidino)phenyl-6-(3-pyridyl)hex-5-ensäure
Schmelzpunkt: 95°C (Zers., Essigsäureethylester/Diisopropylether)

| C₂₃H₂₉N₅O₄S (471,58) | | | | | | | | |
|---|---|---|---|---|---|---|---|---|
| Ber.: | C | 58,58 | H | 6,20 | N | 14,85 | S | 6,80 |
| Gef.: | | 58,29 | | 6,33 | | 14,65 | | 6,68 |

### Beispiel 11

### 5E-6-(3-(2-Carbamoyl-2-cyano-1-(2-methylpropylamino)ethylenamino)phenyl)-6-(3-pyridyl)hex-5-ensäure

1,5 g 5E-6-(3-Aminophenyl)-6-(3-pyridyl)hex-5-ensäuremethylester und 0,95 g 1-Carbamoyl-1-cyano-2,2-bis(methylthio)ethen werden in 25 ml Isopropanol 20 Stunden unter Rückfluß gekocht. Die Reaktionsmischung wird eingeengt, der ölige Rückstand in 40 ml Isopropanol aufgenommen und mit 5 ml 2-Methylbutylamin versetzt. Die Reaktionsmischung wird 4 Stunden unter Rückfluß gekocht und anschließend bei 50°C mit 10 ml 2N Natronlauge versetzt. Die Reaktionslösung wird 30 Minuten bei 50°C gerührt und danach eingeengt. Der Rückstand wird in Wasser aufgenommen und mit Essigsäureethylester gewaschen. Die wäßrige Phase wird durch Zugabe von Zitronensäure auf pH 4-5 eingestellt und mit Essigsäureethylester extrahiert. Der organische Extrakt wird getrocknet eingeengt und der Rückstand über eine Kieselgelsäule mit Dichlormethan/Ethanol = 30:1 gereinigt.
Ausbeute: 40 % der Theorie,
Schaum, R_{f}-Wert: 0,38 (Kieselgel; Dichlormethan/Ethanol = 20:1)

| C₂₅H₂₉N₅O₃ (447,54) | | | | | | |
|---|---|---|---|---|---|---|
| Ber.: | C | 67,09 | H | 6,53 | N | 15,65 |
| Gef.: | | 66,94 | | 6,63 | | 15,69 |

### Analog Beispiel 11 werden folgende Verbindungen erhalten:

(1) 5E-6-(3-(2-Carbamoyl-2-cyano-1-cyclopentylamino-ethylenamino)phenyl-6-(3-pyridyl)hex-5-ensäure
Schaum, R_{f}-Wert: 0,35 (Kieselgel; Dichlormethan/Ethanol = 20:1)

| C₂₆H₂₉N₅O₃ (459,55) | | | | | | |
|---|---|---|---|---|---|---|
| Ber.: | C | 67,95 | H | 6,36 | N | 15,24 |
| Gef.: | | 67,99 | | 6,48 | | 15,06 |

(2) 5E-6-(3-(2-Carbamoyl-2-cyano-1-propylamino-ethylenamino)phenyl-6-(3-pyridyl)hex-5-ensäure
Schmelzpunkt: 161-163°C (Essigsäureethylester/Isopropanol)

| C₂₄H₂₇N₅O₃ (433,51) | | | | | | |
|---|---|---|---|---|---|---|
| Ber.: | C | 66,50 | H | 6,28 | N | 16,16 |
| Gef.: | | 66,37 | | 6,42 | | 16,05 |

(3) 5E-6-(3-(2-Carbamoyl-2-cyano-1-dimethylamino-ethylenamino)phenyl-6-(3-pyridyl)hex-5-ensäure
Schaum, R_{f}-Wert: 0,34 (Kieselgel; Dichlormethan/Ethanol = 10:1)

| C₂₃H₂₅N₅O₃ (419,50) | | | | | | |
|---|---|---|---|---|---|---|
| Ber.: | C | 65,85 | H | 6,01 | N | 16,69 |
| Gef.: | | 65,69 | | 6,10 | | 16,54 |

(4) 5E-6-(3-(2-Cyano-2-methoxycarbonyl-1-(2-methylpropylamino)ethylenamino)phenyl)-6-(3-pyridyl)hex-5-ensäure
Herstellung analog Beispiel 11 durch Umsetzung mit 1-Cyano-1-methoxycarbonyl-2,2-bis(methylthio)ethen.
Schmelzpunkt: 134-135°C (Essigsäureethylester/Diisopropylether)

| C₂₆H₃₀N₄O₄ (462,55) | | | | | | |
|---|---|---|---|---|---|---|
| Ber.: | C | 67,51 | H | 6,54 | N | 12,11 |
| Gef.: | | 67,33 | | 6,48 | | 12,28 |

(5) 5E-6-(3-(2-Cyano-1-cyclopentylamino-2-methoxycarbonylethylenamino)phenyl)-6-(3-pyridyl)hex-5-ensäure
Herstellung analog Beispiel 11 durch Umsetzung mit 1-Cyano-1-methoxycarbonyl-2,2-bis(methylthio)ethen.
Schmelzpunkt: 150-151°C (Essigsäureethylester/Diisopropylether)

| C₂₇H₃₀N₄O₄ (474,56) | | | | | | |
|---|---|---|---|---|---|---|
| Ber.: | C | 68,34 | H | 6,37 | N | 11,81 |
| Gef.: | | 68,31 | | 6,46 | | 11,88 |

### Beispiel 12

### 4E-1-(5-(3-(2-Cyano-3-(2-methylpropyl)guanidino)phenyl-5-(3-pyridyl)pent-4-enyl)tetrazol

2,25 g 4E-1-(5-(3-Aminophenyl)-5-(3-pyridyl)pent-4-enyl)-tetrazol und 2,4 g Diphenoxymethylen-cyanamid werden in 50 ml Isopropanol 20 Stunden bei Raumtemperatur gerührt. Die Reaktionsmischung wird filtriert, das Filtrat mit 5 ml 2-Methylpropylamin versetzt und 13 Stunden unter Rückfluß erhitzt. Das Lösungsmittel wird abgezogen und der Rückstand über eine Kieselgelsäule mit Dichlormethan/Methanol/Essigsäure = 96:4:3 gereinigt.
Ausbeute: 66 % der Theorie,
Schaum, R_{f}-Wert: 0,27 (Kieselgel; Dichlormethan/Methanol/Essigsäure = 90:10:3)

| C₂₃H₂₇N₉ x 0,5 CH₃COOH (459,56) | | | | | | |
|---|---|---|---|---|---|---|
| Ber.: | C | 62,73 | H | 6,36 | N | 27,43 |
| Gef.: | | 62,61 | | 6,52 | | 27,64 |

### Analog Beispiel 12 wird folgende Verbindung erhalten:

(1) 4E-1-(5-(3-(2-Cyano-3-cyclopentyl-guanidino)phenyl)-5-(3-pyridyl)pent-4-enyl)tetrazol
Schaum, R_{f}-Wert: 0,07 (Kieselgel; Dichlormethan/Ethanol = 10:1)

| C₂₄H₂₇N₉ (441,54) | | | | | | |
|---|---|---|---|---|---|---|
| Ber.: | C | 65,29 | H | 6,16 | N | 28,55 |
| Gef.: | | 65,03 | | 6,28 | | 28,42 |

### Beispiel 13

### 4E-1-(5-(3-(2,2-Dicyano-1-cyclopentylamino-ethylenamino)phenyl)-5-(3-pyridyl)pent-4-enyl)tetrazol

2,25 g 4E-1-(5-(3-Aminophenyl)-5-(3-pyridyl)pent-4-enyl)tetrazol und 2,15 g 2,2-Dicyano-1,1-bis(methylthio)ethen werden in 50 ml Isopropanol 48 Stunden unter Rückfluß erhitzt. Die Reaktionsmischung wird mit 3 ml Cyclopentylamin versetzt und weitere 26 Stunden unter Rückfluß gekocht. Das Lösungsmittel wird abgezogen und der Rückstand über eine Kieselgelsäule mit Dichlormethan/Methanol/Essigsäure = 96:4:3 gereinigt.
Ausbeute: 58 % der Theorie,
Schaum, R_{f}-Wert: 0,35 (Kieselgel RP8; 5%ige Natriumchlorid-Lösung/Methanol = 4:6)

| C₂₆H₂₇N₉ x 0,5 CH₃COOH (495,59) | | | | | | |
|---|---|---|---|---|---|---|
| Ber.: | C | 65,44 | H | 5,90 | N | 25,44 |
| Gef.: | | 65,26 | | 6,07 | | 25,35 |

### Beispiel 14

### 5E-6-(3-(2-Benzoyl-3-(2-methylpropyl)guanidino)phenyl)-6-(3-pyridyl)hex-5-ensäure

### a) 5E-6-(3-(Benzoylimino-phenoxy-methylenamino)phenyl)-6-(3-pyridyl)hex-5-ensäuremethylester

5 g N-Benzoyl-diphenylimidocarbonat und 4,14 g 5E-6-(3-Aminophenyl)-6-(3-pyridyl)hex-5-ensäuremethylester werden in 200 ml Isopropanol gelöst und 4 Stunden bei Raumtemperatur gerührt. Der entstandene Niederschlag wird abgesaugt, mit Isopropanol gewaschen und getrocknet.
Ausbeute: 79 % der Theorie,
Schmelzpunkt: 112°C

| C₃₂H₂₉N₃O₄ (519,60) | | | | | | |
|---|---|---|---|---|---|---|
| Ber.: | C | 73,97 | H | 5,63 | N | 8,09 |
| Gef.: | | 73,94 | | 5,68 | | 8,10 |

### b) 5E-6-(3-(2-Benzoyl-3-(2-methylpropyl)guanidino)phenyl)-6-(3-pyridyl)hex-5-ensäure

2,6 g 5E-6-(3-(Benzoylimino-phenoxy-methylenamino)phenyl-6-(3-pyridyl)hex-5-ensäuremethylester und 5 ml 2-Methylpropylamin werden in 50 ml Isopropanol 1 Stunde unter Rückfluß erhitzt. Die Reaktionsmischung läßt man auf 50°C abkühlen, versetzt mit 15 ml 1N Natronlauge und rührt 30 Minuten bei dieser Temperatur. Das Lösungsmittel wird abgezogen, der Rückstand in Wasser aufgenommen und mit Essigsäureethylester gewaschen. Die wäßrige Phase wird durch Zugabe von Zitronensäure auf einen pH-Wert von 4-5 eingestellt, der entstandene Niederschlag abgesaugt und mit Wasser gewaschen. Der Filterkuchen wird aus Essigsäureethylester umkristallisiert.
Ausbeute: 74 % der Theorie,
Schmelzpunkt: 159-160°C

| C₂₉H₃₂N₄O₃ (484,60) | | | | | | |
|---|---|---|---|---|---|---|
| Ber.: | C | 71,88 | H | 6,66 | N | 11,56 |
| Gef.: | | 71,92 | | 6,70 | | 11,73 |

### Analog Beispiel 14 wird folgende Verbindung enthalten:

(1) 5E-6-(3-(2-Benzoyl-3-cyclopentyl-guanidino)phenyl)-6-(3-pyridyl)hex-5-ensäure
Schmelzpunkt: 171°C (Isopropanol/Essigsäureethylester)

| C₃₀H₃₂N₄O₃ (496,61) | | | | | | |
|---|---|---|---|---|---|---|
| Ber.: | C | 72,56 | H | 6,50 | N | 11,28 |
| Gef.: | | 72,42 | | 6,55 | | 11,15 |

### Beispiel 15

### 5E-6-(3-(2-Carbamoyl-3-(2-methylpropyl)guanidino)phenyl)-6-(3-pyridyl)hex-5-ensäure

1,34 g 5E-6-(3-(2-Cyano-3-(2-methylpropyl)guanidino)phenyl)-6-(3-pyridyl)hex-5-ensäure werden in 50 ml 4N Salzsäure gelöst und 48 Stunden bei Raumtemperatur gerührt. Die Lösung wird durch Zugabe von Natriumacetat auf pH 5-6 eingestellt und mit Essigsäureethylester extrahiert. Der organische Extrakt wird eingeengt und der Rückstand über eine Kieselgelsäule mit Dichlormethan/Ethanol = 19:1 gereinigt.
Ausbeute: 54 % der Theorie,
Schaum, R_{f}-Wert: 0,20 (Kieselgel; Dichlormethan/Ethanol = 19:1)

| C₂₃H₂₉N₅O₃ (423,52) | | | | | | | |
|---|---|---|---|---|---|---|---|
| Ber.: | C | 65,22 | H | 6,90 | N | 16,54 | |
| Gef.: | | 65,30 | | 7,05 | | | 16,37 |

### Beispiel 16

### Tabletten mit 100 mg 5E-6-(3-(2,2-Dicyano-1-cyclopentylamino-ethylenamino)phenyl)-6-(3-pyridyl)hex-5-ensäure

### Zusammensetzung:

| 1 Tablette enthält: | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 2̅2̅0̅,̅0̅ m̅g̅ |

### Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Massen (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht | 220 mg |
| Durchmesser | 9 mm, biplan mit beidseitiger Facette und einseitiger Teilkerbe. |

### Beispiel 17

### Hartgelatine-Kapseln mit 150 mg 5E-6-(3-(2,2-Dicyano-1-cyclopentylamino-ethylenamino)phenyl)-6-(3-pyridyl)hex-5-ensäure

| 1 Kapsel enthält: | |
|---|---|
| Wirkstoff | 150,0 mg |
| Maisstärke getr. ca. | 180,0 mg |
| Milchzucker pulv. ca. | 87,0 mg |
| Magnesiumstearat | 3,0 mg |
| | ca. 4̅2̅0̅,̅0̅ m̅g̅ |

### Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung | ca. 420 mg |
| Kapselhülle | Hartgelatine-Kapsel Größe 1. |

### Beispiel 18

### Suppositorien mit 150 mg 5E-6-(3-(2,2-Dicyano-1-cyclopentylamino-ethylenamino)phenyl)-6-(3-pyridyl)hex-5-ensäure

| 1 Zäpfchen enthält: | |
|---|---|
| Wirkstoff | 150,0 mg |
| Polyäthylenglykol (M.G. 1500) | 550,0 mg |
| Polyäthylenglykol (M.G. 6000) | 460,0 mg |
| Polyoxyäthylensorbitanmonostearat | 840,0 mg |
| | 2̅ 0̅0̅0̅,̅0̅ m̅g̅ |

### Herstellung:

Nach dem Aufschmelzen der Suppositorienmassen wird der Wirkstoff darin homogen verteilt und die Schmelze in vorgekühlte Formen gegossen.

### Beispiel 19

### Suspensionen mit 50 mg 5E-6-(3-(2,2-Dicyano-1-cyclopentylamino-ethylenamino)phenyl)-6-(3-pyridyl)hex-5-ensäure

| 100 ml Suspension enthalten: | |
|---|---|
| Wirkstoff | 1,0 g |
| Carboxymethylcellulose-Na-Salz | 0,2 g |
| p-Hydroxybenzoesäuremethylester | 0,05 g |
| p-Hydroxybenzoesäurepropylester | 0,01 g |
| Glycerin | 5,0 g |
| Sorbitlösung 70%ig | 50,0 g |
| Aroma | 0,3 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe der Sorbitlösung und des Aromas wird die Suspension zur Entlüftung unter Rühren evakuiert.
5 ml Suspension enthalten 50 mg Wirkstoff.

### Beispiel 20

### Tabletten mit 150 mg 5E-6-(3-(2,2-Dicyano-1-cyclopentylamino-ethylenamino)phenyl)-6-(3-pyridyl)hex-5-ensäure

### Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloidale Kieselsäure | 10,0 mg |
| Polyvinylpyrrolidon | 10,0 mg |
| Magnesiumstearat | 1,0 mg |
| | 3̅0̅0̅,̅0̅ m̅g̅ |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht | 300 mg |
| Stempel | 10 mm, flach |

### Beispiel 21

### Filmtabletten mit 75 mg 5E-6-(3-(2,2-Dicyano-1-cyclopentylamino-ethylenamino)phenyl)-6-(3-pyridyl)hex-5-ensäure

| 1 Tablettenkern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 2̅3̅0̅,̅0̅ m̅g̅ |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht | 230 mg |
| Stempel | 9 mm, gewölbt |

Die so hergestellten Tablettenkerne werden mit einem Film überzogen, der im wesentlichen aus Hydroxypropylmethylcellulose besteht. Die fertigen Filmtabletten werden mit Bienenwachs geglänzt.

| | |
|---|---|
| Filmtablettengewicht | 245 mg |

Selbstverständlich können alle übrigen Verbindungen der allgemeinen Formel I als Wirkstoffe in den vorstehenden galenischen Zubereitungen eingesetzt werden.

### Beispiel 22

### Filmtabletten, enthaltend 75 mg 5E-6-(3-(2,2-Dicyano-1-cyclopentylamino-ethylenamino)phenyl)-6-(3-pyridyl)hex-5-ensäure (Substanz B) + 75 mg PDE-Hemmer

Eine Pulvermischung aus

| | |
|---|---|
| Dipyridamol | 25 % |
| Substanz B | 25 % |
| Fumarsäure | 15 % |
| Cellulose | 20 % |
| Maisstärke | 8 % |
| Polyvinylpyrrolidon | 6 % |

wird in einem Mischgerät mit Wasser befeuchtet und durch ein Sieb mit der Maschenweite 1.5 mm granuliert. Nach Trocknung und erneuter Siebung mischt man 1 % Magnesiumstearat zu und stellt 10 mm bikonvexe Tabletten von 300 mg her. Diese Tabletten werden solange mit Hydroxypropylmethylcelluloselack besprüht bis sie 312 mg wiegen.

### Beispiel 23

### Hartgelatinekapseln, enthaltend 200 mg 5E-6-(3-(2,2-Dicyano-1-cyclopentylamino-ethylenamino)phenyl)-6-(3-pyridyl)hex-5-ensäure (Substanz B) + 50 mg PDE-Hemmer

10 kg Dipyridamol, 20 kg Fumarsäure, 11,5 kg Polyvinylpyrrolidon, 40 kg Substanz B, 1,5 kg Siliciumdioxid und 0,8 kg Magnesiumstearat mischt man 15 Minuten in einem Kubusmischer. Diese Mischung gibt man über einen Walzenkompaktor, dem ein Trockengranuliergerät mit Siebeinrichtung nachgeschaltet ist. Verwendung findet die Fraktion 0.25-1,0 mm. Die Kapselfüllmaschine wird so eingestellt, daß jede Kapsel der Größe 0 die 50 mg PDE-Hemmer und 200 mg (Substanz B) entsprechende Menge Granulat enthält.

### Beispiel 24

### Hartgelatinekapseln enthaltend 100 mg 5E-6-(3-(2,2-Dicyano-1-cyclopentylamino-ethylenamino)phenyl)-6-(3-pyridyl)hex-5-ensäure (Substanz B) + 250 mg PDE-Hemmer

### a) Granulat

125 kg Mopidamol, 50 kg Fumarsäure, 13,5 kg Milchzucker werden gemischt und mit einer Lösung aus Wasser/Polyäthylenglykol 6000 befeuchtet. Nach Granulierung durch ein Sieb mit der Maschenweite 1,0 mm und Trocknung bei 45°C mischt man 1,4 kg Stearinsäure zu.

### b) Dragée

100 kg Substanz B, 7,5 kg Hydroxypropylmethylcellulose, 2,5 kg Siliciumdioxid und 15 kg Carboxymethylcellulose werden mit Äthanol befeuchtet und durch ein Sieb mit der Maschenweite 1,5 mm granuliert. Nach Trocknung mischt man 1 kg Magnesiumstearat zu und verpreßt das Granulat zu 126 mg schweren bikonvexen Tabletten mit einem Durchmesser von 5,5 mm.

Diese Kerne überzieht man in mehreren Schritten mit einer Dragiersuspension, bestehend aus 5,6 kg Saccharose, 0,5 kg Gummi-arabicum und 3,8 kg Talcum, bis die Tabletten ein Gewicht von 135 mg haben.

### c) Abfüllung

Auf einer Spezial-Kapselmaschine füllt man in eine Hartgelatine-Kapsel der Größe 0 long die 250 mg PDE-Hemmer entsprechende Menge Granulat ein und legt das 100 mg Substanz B enthaltende Dragée obenauf.

### Beispiel 25

### Suspension, enthaltend 10 mg 5E-6-(3-(2,2-Dicyano-1-cyclopentylamino-ethylenamino)phenyl)-6-(3-pyridyl)hex-5-ensäure (Substanz B) + 100 mg Dipyridamol pro 5 g.

Die Suspension hat folgende Zusammensetzung:

| | |
|---|---|
| (1) Dipyridamol | 2,0 % |
| (2) Substanz B | 0,2 % |
| (3) Sorbit | 20,8 % |
| (4) Cellulose | 7,5 % |
| (5) Natriumcarboxymethylcellulose | 2,5 % |
| (6) Geschmackskorrigentien/Konservierungsstoffe | 1,8 % |
| (7) Wasser | 65,2 % |

In heißes Wasser wird unter hoher Scherung (3) - (6) eingerührt. Nach Abkühlung werden in die viskose Suspension (1), (2) und (7) eingearbeitet.

### Beispiel 26

### Depot-Form, enthaltend 50 mg 5E-6-(3-(2,2-Dicyano-1-cyclopentylamino-ethylenamino)phenyl)-6-(3-pyridyl)hex-5-ensäure (Substanz B) + 200 mg Dipyridamol

### a) Pellet I

Eine Mischung aus

| | |
|---|---|
| Substanz B | 50,0 kg |
| Lysin | 12,5 kg |
| Hydroxypropylcellulose, hochpolymer | 52,5 kg |
| Triacetin | 4,0 kg |
| Äthylcellulose | 2,5 kg |
| Magnesiumstearat | 3,5 kg |

wird auf einem Spezialextruder mit Äthanol geknetet und in Form von Spaghetti (Durchmesser 1 mm) extrudiert, die in einem Sphäronizer zu Pellets gerundet werden. Danach trocknet man sie gründlich.

### b) Pellet II

300 kg ausgemischte Weinsäure-Starterpellets werden in einem Spezialkessel mit einer Suspension bestehend aus Isopropanol, Dipyridamol und Polyvinylpyrrolidon solange besprüht, bis die entstehenden Wirkstoffpellets ca. 45 % Dipyridamol enthalten.

Diese Pellets besprüht man mit einem Lack, der aus Methacrylsäure/Methylmethacrylat-Copolymer (Handelsnahme Eudragit S) und Hydroxypropylmethylcellulosephthalat (Handelsname HP 55) im Gewichtsverhältnis 85:15 bis 50:50 besteht. Die organischen Lacklösungen enthalten noch Weichmacher und Talkum. Es werden zwei Pelletkomponenten mit 5 und 7 % Hüllenmittel und unterschiedlichem Verhältnis der Lackkomponenten in den genannten Grenzen gesprüht. Die beiden Komponenten werden so gemischt, daß sie nachfolgende in vitro-Freigabe ergeben:
Bedingungen (entsprechend USPXXI, Basket-Methode, 100 Umdrehungen/Min.,
1. Stunde künstlicher Magensaft, pH 1,2, 2. bis 6. Stunde künstlicher Darmsaft (Phosphatpuffer), pH 5.5):
Wirkstoff-Freigabe pro Stunde:

| | |
|---|---|
| 1. Stunde | ca. 30 % |
| 2. Stunde | ca. 25 % |
| 3. Stunde | ca. 18 % |
| 4. Stunde | ca. 12 % |

nach der 6. Stunde mehr als 90 % Dipyridamol-Freigabe.

### c) Abfüllung

Entsprechend dem Wirkstoffgehalt der Pelletkomponenten I und II und der gewünschten Dosierung werden die Pellets miteinander vermischt und auf einer Kapselmaschine in Kapseln der Größe 0 long abgefüllt.

### Beispiel 27

### Ampullen, enthaltend 5 mg 5E-6-(3-(2,2-Dicyano-1-cyclopentylamino-ethylenamino)phenyl)-6-(3-pyridyl)hex-5-ensäure (Substanz B) + 10 mg Dipyridamol per 5 ml

### Zusammensetzung:

| | |
|---|---|
| (1) Dipyridamol | 10 mg |
| (2) Substanz B | 5 mg |
| (3) Propylenglykol | 50 mg |
| (4) Polyethylenglykol | 5 mg |
| (5) Ethanol | 10 mg |
| (6) Wasser für Injektionszwecke ad | 5 ml |
| (7) 1N HCl ad | pH 3 |

Die Wirkstoffe werden unter Erwärmung in der Lösung aus (3) - (7) gelöst. Nach pH-Kontrolle und Sterilfiltration füllt man in geeignete Ampullen und sterilisiert.

## Patentansprüche

1. Pyridylderivate der allgemeinen Formel 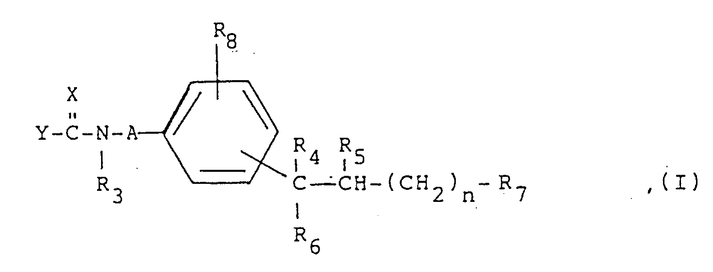 in der
n die Zahl 2, 3, 4 oder 5,
A eine Kohlenstoff-Stickstoff-Bindung oder eine gegebenenfalls durch eine oder zwei Alkylgruppen substituierte geradkettige Alkylengruppe mit 1 bis 4 Kohlenstoffatomen,
X eine Nitromethylengruppe, eine gegebenenfalls durch eine R₉-Gruppe substituierte Cyanomethylengruppe oder eine Gruppe der Formel =N-R₁₀, wobei R₉ mit Ausnahme der Tetrazolylgruppe die für R₇ nachstehend erwähnten Bedeutungen besitzt und R₁₀ eine Cyano-, Alkansulfonyl-, Phenylsulfonyl-, Phenylalkansulfonyl-, Aminosulfonyl-, Alkylaminosulfonyl-, Dialkylaminosulfonyl-, Phenylcarbonyl-, Aminocarbonyl-, Alkylaminocarbonyl- oder Dialkylaminocarbonylgruppe darstellt,
Y eine Alkoxy-, Phenoxy-, Alkylthio- oder Phenylthiogruppe oder eine Gruppe der Formel -R₁NR₂, wobei R₁ ein Wasserstoffatom, eine verzweigte oder unverzweigte Alkylgruppe mit 1 bis 10 Kohlenstoffatomen, die in 2-, 3- oder 4-Stellung durch eine Hydroxy-, Amino-, Alkylamino- oder Dialkylaminogruppe substituiert sein kann, eine Alkylgruppe mit 1 bis 4 Kohlenstoffatomen, die durch eine Phenyl- oder Pyridylgruppe substituiert ist und zusätzlich in 2-, 3- oder 4-Stellung durch eine Hydroxygruppe substituiert sein kann, eine Cycloalkylgruppe mit 3 oder 4 Kohlenstoffatomen, eine Cycloalkylgruppe mit 5 bis 8 Kohlenstoffatomen, in der eine Ethylenbrücke durch eine o-Phenylengruppe ersetzt sein kann, eine gegebenenfalls durch 1, 2 oder 3 Alkylgruppen substituierte Bicycloalkylgruppe mit 6 bis 8 Kohlenstoffatomen, eine Adamantyl-, Alkoxy- oder Trimethylsilylalkylgruppe,
R₂ ein Wasserstoffatom oder eine geradkettige Alkylgruppe oder
R₁ und R₂ zusammen mit dem dazwischenliegenden Stickstoffatom eine cyclische Alkyleniminogruppe mit 4 bis 6 Kohlenstoffatomen, die durch ein oder zwei Alkylgruppen oder durch eine Phenylgruppe substituiert sein kann und in der zusätzlich eine Ethylenbrücke in 3,4-Stellung durch eine o-Phenylengruppe ersetzt sein kann, eine Morpholinogruppe oder eine gegebenenfalls in 4-Stellung durch eine Alkylgruppe mit 1 bis 3 Kohlenstoffatomen oder durch eine Phenylgruppe substituierte Piperazinogruppe,
R₃ ein Wasserstoffatom oder eine Alkylgruppe mit einem bis drei Kohlenstoffatomen,
R₄ und R₅ je ein Wasserstoffatom oder zusammen eine Kohlenstoff-Kohlenstoff-Bindung,
R₆ eine gegebenenfalls in 3- oder 4-Position durch eine Alkylgruppe substituierte Pyridylgruppe,
R₇ eine Cyano-, Tetrazolyl-, Aminocarbonyl-, Alkylaminocarbonyl- oder Dialkylaminocarbonylgruppe, eine in-vivo metabolisch in eine Carboxygruppe überführbare Gruppe oder auch, wenn Y eine R₁NR₂-Gruppe darstellt, eine Carboxygruppe,
R₈ ein Wasserstoff-, Fluor-, Chlor-, Brom- oder Jodatom, eine Alkyl-, Alkoxy- oder Trifluormethylgruppe bedeuten,
wobei alle vorstehend erwähnten Alkyl- und Alkoxyteile, sofern nichts anderes erwähnt wurde, ein bis drei Kohlenstoffatome enthalten können,
sowie alle vorstehend erwähnten Phenylkerne, sofern nichts anderes erwähnt wurde, durch ein Fluor-, Chlor- oder Bromatom, durch eine Alkyl-, Hydroxy-, Alkoxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituiert sein können und die Substituenten gleich oder verschieden sein können,
deren Enantiomere, deren cis- und trans-Isomere, sofern R₄ und R₅ zusammen eine Kohlenstoff-Kohlenstoff-Bindung darstellen, und deren Salze.

2. Pyridylderivate der allgemeinen Formel I gemäß Anspruch 1, in der
n, A, X, Y, R₃ bis R₆ und R₈ wie im Anspruch 1 definiert sind und
R₇ eine Tetrazolylgruppe oder eine Gruppe der Formeln
- CO - OR',
- CO - O - (HCR'') - O - CO - R''' und
- CO - O - (HCR'') - O - CO - OR''',
in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten,
oder auch, wenn Y eine R₁NR₂-Gruppe darstellt, eine Carboxygruppe darstellt,
deren Enantiomere, deren cis- und trans-Isomere, sofern R₄ und R₅ zusammen eine Kohlenstoff-Kohlenstoff-Bindung darstellen, und deren Salze.

3. Pyridylderivate der allgemeinen Formel I gemäß Anspruch 1, in der
n die Zahl 2, 3, 4 oder 5,
A eine Bindung oder eine Ethylengruppe,
X eine Nitromethylengruppe, eine gegebenenfalls durch eine R₉-Gruppe substituierte Cyanomethylengruppe oder eine Gruppe der Formel =N-R₁₀, wobei R₉ mit Ausnahme der Tetrazolylgruppe die für R₇ nachstehend erwähnten Bedeutungen besitzt und R₁₀ eine Cyanogruppe, eine Phenylsulfonyl- oder Alkansulfonylgruppe darstellt,
Y eine Phenoxy- oder Methylthiogruppe oder eine R₁NR₂-Gruppe, wobei R₁ ein Wasserstoffatom, eine geradkettige oder verzweigte Alkylgruppe mit 1 bis 8 Kohlenstoffatomen, die in 2-, 3- oder 4-Stellung durch eine Hydroxy- oder Dimethylaminogruppe substituiert sein kann, eine Alkylgruppe mit 1 bis 4 Kohlenstoffatomen, die durch eine Phenyl- oder Pyridylgruppe substituiert ist und zusätzlich in 2-, 3- oder 4-Stellung durch eine Hydroxygruppe substituiert sein kann, eine Cycloalkylgruppe mit 3 bis 8 Kohlenstoffatomen, eine Methoxy-, Trimethylsilylmethyl-, Indan-2-yl- oder eine gegebenenfalls durch 1, 2 oder 3 Alkylgruppen substituierte Bicycloheptylgruppe und
R₂ ein Wasserstoffatom oder eine Methylgruppe oder
R₁ und R₂ zusammen mit dem dazwischenliegenden Stickstoffatom eine Piperidinogruppe, die durch eine oder zwei Methylgruppen oder durch eine Phenylgruppe substituiert sein kann und in der zusätzlich eine Ethylenbrücke in 3,4-Stellung durch eine o-Phenylengruppe ersetzt sein kann, eine Morpholinogruppe oder eine in 4-Stellung durch eine Phenylgruppe substituierte Piperazinogruppe darstellen,
R₃ ein Wasserstoffatom oder eine Methylgruppe,
R₄ und R₅ jeweils ein Wasserstoffatom oder zusammen eine weitere Kohlenstoff-Kohlenstoff-Bindung,
R₆ eine 3-Pyridyl- oder 4-Pyridylgruppe und
R₇ eine Cyano-, Tetrazolyl-, Alkoxycarbonyl-, Aminocarbonyl-, Alkylaminocarbonyl- oder Dialkylaminocarbonylgruppe mit jeweils 1 bis 3 Kohlenstoffatomen in den Alkoxy- und Alkylteilen, oder auch, wenn Y eine R₁NR₂-Gruppe darstellt, eine Carboxygruppe,
R₈ ein Wasserstoff-, Fluor-, Chlor- oder Bromatom, eine Alkyl-, Alkoxy- oder Trifluormethylgruppe bedeuten, wobei alle vorstehend erwähnten Alkyl- und Alkoxyteile, sofern nichts anderes erwähnt wurde, ein bis drei Kohlenstoffatome enthalten können, bedeuten, deren Enantiomere, deren cis- und trans-Isomere, sofern R₄ und R₅ zusammen eine Kohlenstoff-Kohlenstoff-Bindung darstellen, und deren Salze.

4. Pyridylderivate der allgemeinen Formel I gemäß Anspruch 1, in der
n die Zahl 3,
A eine Bindung oder eine Ethylengruppe,
X eine Gruppe der Formel =N-R₁₀, wobei R₁₀ eine Cyano- oder Phenylsulfonylgruppe darstellt, oder eine Dicyanomethylengruppe,
Y eine R₁NR₂-Gruppe, wobei R₁ eine geradkettige oder verzweigte Alkylgruppe mit 1 bis 8 Kohlenstoffatomen, eine Cycloalkylgruppe mit 3 bis 8 Kohlenstoffatomen oder eine Exo-norbornyl-(2)-gruppe und
R₂ ein Wasserstoffatom,
R₃ ein Wasserstoffatom,
R₄ und R₅ jeweils ein Wasserstoffatom oder zusammen eine Kohlenstoff-Kohlenstoff-Bindung,
R₆ eine 3-Pyridylgruppe und
R₇ eine Carboxy- oder Alkoxycarbonylgruppe mit insgesamt 2 bis 4 Kohlenstoffatomen,
R₈ ein Wasserstoff-, Chlor- oder Bromatom, eine Methyl- oder Trifluormethylgruppe bedeuten, deren Enantiomere, deren cis- und trans-Isomere und deren Salze.

5. Verbindungen der Formel I gemäß Anspruch 1:
(a) 5E-6-(3-(2-Cyano-3-cyclopropyl-guanidino)phenyl)-6-(3-pyridyl)hex-5-ensäure,
(b) 5E-6-(3-(2-Cyano-3-tert.butyl-guanidino)phenyl)-6-(3-pyridyl)hex-5-ensäure,
(c) 5E-6-(3-(2-Cyano-3-cyclopentyl-guanidino)phenyl)-6-(3-pyridyl)hex-5-ensäure,
(d) 5E-6-(3-(2-Cyano-3-isopropyl-guanidino)phenyl)-6-(3-pyridyl)hex-5-ensäure,
(e) 5E-6-(3-(2-Cyano-3-(exo-norborn-2-yl)guanidino)phenyl)-6-(3-pyridyl)hex-5-ensäure,
(f) 5E-6-(3-(2-Cyano-3-(2-methylpropyl)guanidino)phenyl)-6-(3-pyridyl)hex-5-ensäure,
(g) 5E-6-(3-(2-Cyano-3-neopentyl-guanidino)phenyl)-6-(3-pyridyl)hex-5-ensäure,
(h) 5E-6-(3-(2-Cyano-3-pentyl-guanidino)phenyl)-6-(3-pyridyl)hex-5-ensäure,
(i) 5E-6-(3-(2-Cyano-3-(3-methylbutyl)guanidino)phenyl)-6-(3-pyridyl)hex-5-ensäure,
(j) 5E-6-(3-(2,2-Dicyano-1-(2-methylpropylamino)ethylenamino)phenyl)-6-(3-pyridyl)hex-5-ensäure,
(k) 5E-6-(3-(2,2-Dicyano-1-isopropylamino-ethylenamino)phenyl)-6-(3-pyridyl)hex-5-ensäure,
(l) 5E-6-(3-(2,2-Dicyano-1-(3-methylbutylamino)-ethylenamino)phenyl)-6-(3-pyridyl)hex-5-ensäure,
(m) 5E-6-(3-(2,2-Dicyano-1-cyclopentylamino-ethylenamino)phenyl)-6-(3-pyridyl)hex-5-ensäure,
(n) 5E-6-(3-(2,2-Dicyano-1-neopentylamino-ethylenamino)phenyl)-6-(3-pyridyl)hex-5-ensäure,
(o) 5E-6-(3-(2,2-Dicyano-1-cyclopentylamino-ethylenamino)phenyl)-6-(3-pyridyl)hex-5-ensäure,
(p) 5E-6-(3-(2,2-Dicyano-1-propylamino-ethylenamino)phenyl)-6-(3-pyridyl)hex-5-ensäure und
(q) 5E-6-(3-(2,2-Dicyano-1-tert.butylamino-ethylenamino)phenyl)-6-(3-pyridyl)hex-5-ensäure,
(r) 5E-6-(4-(2-Cyano-3-cyclohexyl-guanidino)phenyl)-6-(3-pyridyl)hex-5-ensäure,
(s) 6-(3-(2-Cyano-3-tert.butyl-guanidino)phenyl)-6-(3-pyridyl)hexansäure,
(t) 5E-6-(3-(1-Neopentylamino-2-nitro-ethylenamino)phenyl)-6-(3-pyridyl)hex-5-ensäure,
(u) E/Z-6-(4-(2-(2-Cyano-3-tert.butyl-guanidino)ethyl)phenyl)-6-(3-pyridyl)hex-5-ensäure,
(v) 5E-6-(3-(3-tert.Butyl-2-phenylsulfonyl-guanidino)phenyl)6-(3-pyridyl)hex-5-ensäure,
(w) 5E-6-(3-(2-Amidosulfonyl-3-(2-methylproppyl)guanidino)phenyl)-6-(3-pyridyl)hex-5-ensäure,
(x) 5E-6-(3-(2-Carbamoyl-2-cyano-1-(2-methylpropylamino)ethylenamino)phenyl)-6-(3-pyridyl)hex-5-ensäure und
(y) 4E-1-(5-(3-(2-Cyano-3-cyclopentyl-guanidino)phenyl)-5-(3-pyridyl)-4-pentenyl)tetrazol,
deren cis- und trans-Isomere und deren Salze.

6. 5E-6-(3-(2,2-Dicyano-1-cyclopentylamino-ethylenamino)phenyl)-6-(3-pyridyl)hex-5-ensäure,
deren cis- und trans-Isomere und deren Salze.

7. Physiologisch verträgliche Salze der Verbindungen gemäß den Ansprüchen 1 bis 6 mit anorganischen oder organischen Säuren oder Basen.

8. Arzneimittel, enthaltend als Wirkstoff eine Verbindung gemäß den Ansprüchen 1 bis 6 oder dessen physiologisch verträgliches Salz gemäß Anspruch 7 neben gegebenenfalls einem oder mehreren inerten Trägerstoffen und/oder Verdünnungsmitteln.

9. Arzneimittel gemäß Anspruch 8, geeignet zur Behandlung und zur Prophylaxe thrombo-embolischer Erkrankungen, zur Prophylaxe der Arteriosklerose und zur Metastasenprophylaxe sowie zur Behandlung der Ischämie, des Asthmas und von Allergien.

10. Arzneimittel gemäß Anspruch 8, geeignet zur Behandlung und zur Prophylaxe von Erkrankungen, bei denen eine thromboxanvermittelte Konstriktion oder PGE₂-vermittelte Dilatation der Kapillaren eine Rolle spielen, zur Verminderung des Schweregrades einer Transplantatabstoßung, zur Verminderung der renalen Toxizität von Substanzen wie Cyclosporin, zur Behandlung von Nierenerkrankungen und zur Behandlung von Schockzuständen.

11. Arzneimittel gemäß Anspruch 8, 9 oder 10, dadurch gekennzeichnet, daß dieses zusätzlich als Wirkstoff einen PDE-Hemmer oder ein Lysemittel enthält.

12. Verfahren zur Herstellung eines Arzneimittels gemäß den Ansprüchen 8 bis 11, dadurch gekennzeichnet, daß auf nichtchemischem Wege eine Verbindung gemäß den Ansprüchen 1 bis 6 oder deren physiologisch verträgliches Additionssalz gemäß Anspruch 7, gegebenenfalls in Kombination mit einem PDE-Hemmer oder einem Lysemittel, in ein oder in mehrere inerte übliche Trägerstoffe und/oder Verdünnungsmittel eingearbeitet wird.

13. Verfahren zur Herstellung der Verbindungen gemäß den Ansprüchen 1 bis 7, dadurch gekennzeichnet, daß
a) zur Herstellung von Verbindungen der allgemeinen Formel I, in der Y eine Alkoxy-, Phenoxy-, Alkylthio- oder Phenylthiogruppe darstellt, eine Verbindung der allgemeinen Formel 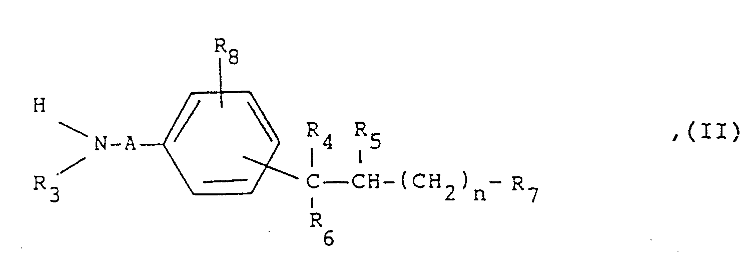 in der
A, n und R₃ bis R₈ wie in den Ansprüchen 1 bis 6 definiert sind, mit einer Verbindung der allgemeinen Formel
(Y')₂C=X ,(III)
in der
X wie in den Ansprüchen 1 bis 6 definiert ist und
Y' eine Alkoxy-, Phenoxy-, Alkylthio- oder Phenylthiogruppe darstellt, umgesetzt wird oder
b) zur Herstellung von Verbindungen der allgemeinen Formel I, in der Y eine R₁NR₂-Gruppe darstellt, eine Verbindung der allgemeinen Formel 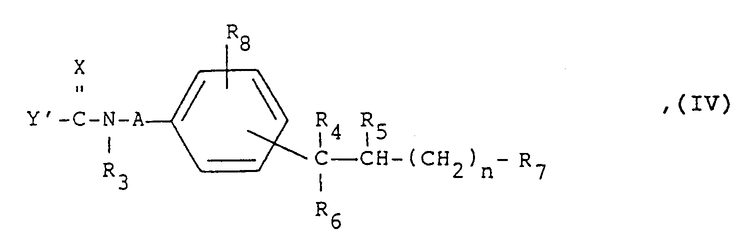 in der
A, X, n und R₃ bis R₈ wie in den Ansprüchen 1 bis 6 definiert sind und
Y' eine Alkoxy-, Phenoxy-, Alkylthio- oder Phenylthiogruppe darstellt, mit einem Amin der allgemeinen Formel 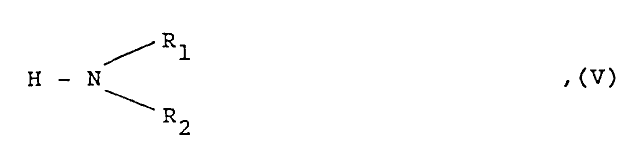 in der
R₁ und R₂ wie in den Ansprüchen 1 bis 6 definiert sind, umgesetzt wird oder
c) zur Herstellung von Verbindungen der allgemeinen Formel I, in der Y eine R₁NR₂-Gruppe und R₇ eine Carboxygruppe darstellt, ein Schutzrest von einer Verbindung der allgemeinen Formel 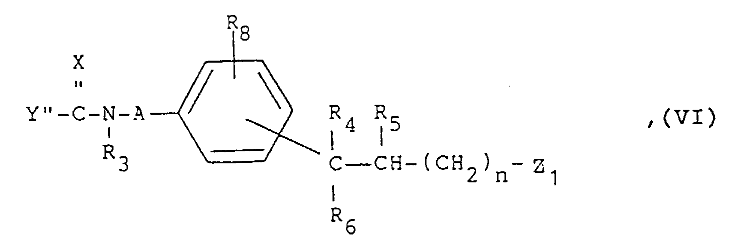 in der
R₃ bis R₆, R₈, A, X und n wie in den Ansprüchen 1 bis 6 definiert sind,
Y'' eine R₁NR₂-Gruppe, wobei R₁ und R₂ wie in den Ansprüchen 1 bis 6 definiert sind, und
Z₁ eine mittels Hydrolyse, Thermolyse oder Hydrogenolyse in eine Carboxygruppe überführbare Gruppe darstellt, abgespalten wird oder
d) zur Herstellung von Verbindungen der allgemeinen Formel I, in der R₄ und R₅ jeweils ein Wasserstoffatom darstellen, eine Verbindung der allgemeinen Formel 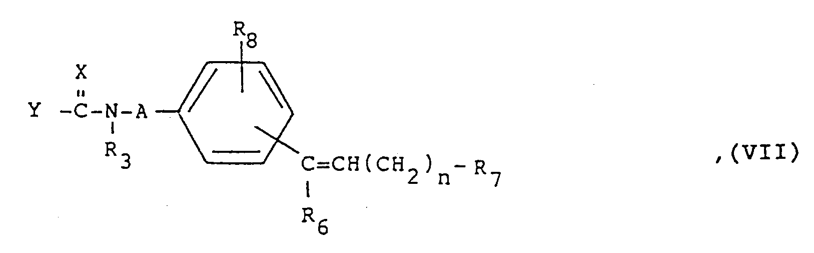 in der
A, X, Y, n, R₃ und R₆ bis R₈ wie in den Ansprüchen 1 bis 6 definiert sind, hydriert wird oder
e) zur Herstellung einer Verbindung der allgemeinen Formel I, in der X mit Ausnahme der eine Cyanogruppe enthaltenden Reste die für X in den Ansprüchen 1 bis 6 erwähnten Bedeutungen besitzt und R₇ eine Tetrazolylgruppe darstellt, eine Verbindung der allgemeinen Formel 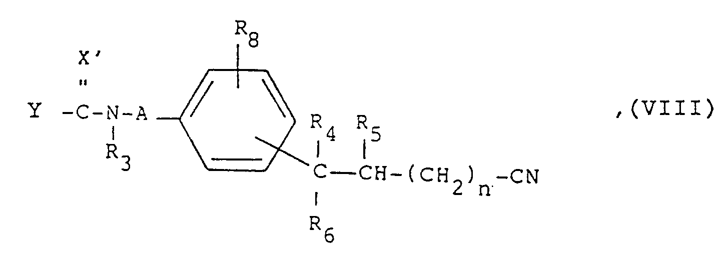 in der
A, n, Y, R₃ bis R₆ und R₈ wie in den Ansprüchen 1 bis 6 definiert sind und
X' mit Ausnahme der eine Cyanogruppe enthaltenden Reste die für X wie in den Ansprüchen 1 bis 6 erwähnten Bedeutungen besitzt, mit Stickstoffwasserstoffsäure oder deren Salzen umgesetzt wird und
gewünschtenfalls eine so erhaltene Verbindung der allgemeinen Formel I, in der X eine Gruppe der Formel = N-CN darstellt, mittels Verseifung in eine entsprechende Verbindung der Formel I, in der X eine Gruppe der Formel =N-CONH₂ darstellt, übergeführt wird oder
gewünschtenfalls anschließend eine so erhaltene Verbindung der Formel I, in der R₇ eine Carboxygruppe darstellt, mittels Veresterung oder Amidierung in eine entsprechende Verbindung der Formel I, in der R₇ eine in vivo metabolisch in eine Carboxygruppe überführbare Gruppe, eine Aminocarbonyl-, Alkylaminocarbonyl- oder Dialkylaminocarbonylgruppe darstellt, übergeführt wird oder
eine so erhaltene Verbindung der Formel I, in der R₇ eine Aminocarbonylgruppe darstellt, mittels Dehydratisierung in eine entsprechende Verbindung der Formel I, in der R₇ eine Cyanogruppe darstellt, übergeführt wird oder
eine so erhaltene Verbindung der Formel I, in der R₄ und R₅ zusammen eine Kohlenstoff-Kohlenstoff-Bindung darstellen, in ihre cis- und trans-Isomere aufgetrennt wird oder
eine so erhaltene Verbindung der Formel I in ihre Enantiomeren aufgetrennt wird oder
eine so erhaltene Verbindung der Formel I in ihre Salze, insbesondere in ihre physiologisch verträglichen Salze mit anorganischen oder organischen Säuren oder Basen übergeführt wird.

## Claims

1. Pyridyl derivatives of general formula 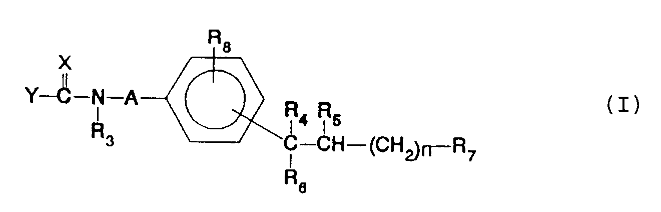 wherein
n represents the number 2, 3, 4 or 5,
A denotes a carbon-nitrogen bond or a straight-chained C₁₋₄-alkylene group optionally substituted by one or two alkyl groups,
X denotes a nitromethylene group, a cyanomethylene group optionally substituted by an R₉ group, or a group of the formula =N-R₁₀, wherein R₉ has the meanings given for R₇ hereinafter with the exception of the tetrazolyl group and R₁₀ denotes a cyano, alkanesulphonyl, phenylsulphonyl, phenylalkanesulphonyl, aminosulphonyl, alkylaminosulphonyl, dialkylaminosulphonyl, phenylcarbonyl, aminocarbonyl, alkylaminocarbonyl or dialkylaminocarbonyl group,
Y denotes an alkoxy, phenoxy, alkylthio or phenylthio group or a group of the formula -R₁NR₂ (wherein R₁ denotes a hydrogen atom, a straight-chained or branched C₁₋₁₀-alkyl group which may be substituted in the 2-, 3-or 4-position by a hydroxy, amino, alkylamino or dialkylamino group, a C₁₋₄-alkyl group which is substituted by a phenyl or pyridyl group and which may additionally be substituted in the 2-, 3- or 4-position by a hydroxy group, a C₃₋₄-cycloalkyl group, a C₅₋₈-cycloalkyl group in which an ethylene bridge may be replaced by an o-phenylene group, a C₆₋₈-bicycloalkyl group optionally substituted by 1, 2 or 3 alkyl groups, or an adamantyl, alkoxy or trimethylsilylalkyl group,
R₂ denotes a hydrogen atom or a straight-chained alkyl group or
R₁ and R₂ together with the nitrogen atom between them denote a cyclic C₄₋₆-alkyleneimino group which may be substituted by one or two alkyl groups or by a phenyl group and wherein additionally an ethylene bridge in the 3,4-position may be replaced by an o-phenylene group, or they denote a morpholino group or a piperazino group optionally substituted in the 4-position by a C₁₋₃-alkyl group or by a phenyl group),
R₃ denotes a hydrogen atom or a C₁₋₃-alkyl group,
R₄ and R₅ each denote a hydrogen atom or together represent a carbon-carbon bond,
R₆ denotes a pyridyl group optionally substituted in the 3- or 4-position by an alkyl group,
R₇ denotes a cyano, tetrazolyl, aminocarbonyl, alkylaminocarbonyl or dialkylaminocarbonyl group, a group which may be metabolically converted into a carboxy group in vivo or, if Y denotes an R₁NR₂- group, R₇ may represent a carboxy group,
R₈ denotes a hydrogen, fluorine, chlorine, bromine or iodine atom or an alkyl, alkoxy or trifluoromethyl group,
whilst all the above-mentioned alkyl and alkoxy moieties, unless otherwise stated, may contain one to three carbon atoms,
and all the above-mentioned phenyl nuclei, unless otherwise stated, may be mono- or disubstituted by fluorine, chlorine or bromine atoms or by alkyl, hydroxy, alkoxy, phenyl, nitro, amino, alkylamino, dialkylamino, alkanoylamino, cyano, carboxy, alkoxycarbonyl, aminocarbonyl, alkylaminocarbonyl, dialkylaminocarbonyl, trifluoromethyl, alkanoyl, aminosulphonyl, alkylaminosulphonyl or dialkylaminosulphonyl groups, and the substituents may be identical or different,
the enantiomers thereof, the cis- and trans-isomers thereof (where R₄ and R₅ together denote a carbon-carbon bond) and the salts thereof.

2. Pyridyl derivatives of general formula I according to claim 1, wherein
n, A, X, Y, R₃ to R₆ and R₈ are defined as in claim 1 and
R₇ denotes a tetrazolyl group or a group of the formulae
- CO - OR',
- CO - O - (HCR'') - O - CO - R''' and
- CO - O - (HCR'') - O - CO - OR'''
wherein
R' denotes a straight-chained or branched C₁₋₆-alkyl group, a C₅₋₇-cycloalkyl group, a benzyl, 1-phenylethyl, 2-phenylethyl, 3-phenylpropyl, methoxymethyl or cinnamyl group,
R'' denotes a hydrogen atom or a methyl group and
R''' denotes a straight-chained or branched C₁₋₆-alkyl group, a C₅₋₇-cycloalkyl group, a phenyl, benzyl, 1-phenylethyl, 2-phenylethyl or 3-phenylpropyl group,
of if Y denotes a R₁NR₂- group, R''' may represent a carboxy group,
the enantiomers thereof, the cis- and trans-isomers thereof (if R₄ and R₅ together represent a carbon-carbon bond) and the salts thereof.

3. Pyridyl derivatives of general formula I according to claim 1, wherein
n denotes the number 2, 3, 4 or 5,
A is a bond or an ethylene group,
X is a nitromethylene group, a cyanomethylene group optionally substituted by an R₉ group, or a group of the formula =N-R₁₀, wherein R₉ has the meanings given for R₇ hereinafter with the exception of the tetrazolyl group and R₁₀ denotes a cyano, phenylsulphonyl or alkanesulphonyl group,
Y denotes a phenoxy or methylthio group or an R₁NR₂-group (wherein R₁ is a hydrogen atom, a straight-chained or branched C₁₋₈-alkyl group which may be substituted in the 2-, 3- or 4-position by a hydroxy or dimethylamino group, a C₁₋₄-alkyl group which is substituted by a phenyl or pyridyl group and may additionally be substituted in the 2-, 3- or 4-position by a hydroxy group, a C₃₋₈-cycloalkyl group, a methoxy, trimethylsilylmethyl or indan-2-yl group or a bicycloheptyl group optionally substituted by 1, 2 or 3 alkyl groups and
R₂ is a hydrogen atom or a methyl group or
R₁ and R₂ together with the nitrogen atom between them denote a piperidino group which may be substituted by one or two methyl groups or by a phenyl group and wherein additionally an ethylene bridge in the 3,4-position may be replaced by an o-phenylene group, or they denote a morpholino group or a piperazino group substituted in the 4-position by a phenyl group),
R₃ denotes a hydrogen atom or a methyl group,
R₄ and R₅ each denote a hydrogen atom or together represent another carbon-carbon bond,
R₆ denotes a 3-pyridyl or 4-pyridyl group and
R₇ denotes a cyano, tetrazolyl, alkoxycarbonyl, aminocarbonyl, alkylaminocarbonyl or dialkylaminocarbonyl group each having 1 to 3 carbon atoms in the alkoxy and alkyl moieties, or if Y denotes a R₁NR₂-group, R₇ may represent a carboxy group,
R₈ denotes a hydrogen, fluorine, chlorine or bromine atom or an alkyl, alkoxy or trifluoromethyl group,
whilst unless otherwise stated all the above-mentioned alkyl and alkoxy moieties may contain one to three carbon atoms,
the enantiomers thereof, the cis- and trans-isomers thereof (where R₄ and R₅ together denote a carbon-carbon bond) and the salts thereof.

4. Pyridyl derivatives of general formula I according to claim 1, wherein
n denotes the number 3,
A denotes a bond or an ethylene group,
X denotes a group of the formula =N-R₁₀ (wherein R₁₀ is a cyano or phenylsulphonyl group) or a dicyanomethylene group,
Y is an R₁NR₂- group (wherein R₁ is a straight-chained or branched C₁₋₈-alkyl group, a C₃₋₈-cycloalkyl group or an exo-norbornyl-(2) group and R₂ is a hydrogen atom),
R₃ is a hydrogen atom,
R₄ and R₅ each represent a hydrogen atom or together denote a carbon-carbon bond,
R₆ is a 3-pyridyl group and
R₇ denotes a carboxy or alkoxycarbonyl group having a total of 2 to 4 carbon atoms,
R₈ is a hydrogen, chlorine or bromine atom or a methyl or trifluoromethyl group,
the enantiomers thereof, the cis- and trans-isomers thereof and the salts thereof.

5. Compounds of formula I according to claim 1:
(a) 5E-6-(3-(2-cyano-3-cyclopropyl-guanidino)phenyl)-6-(3-pyridyl)hex-5-enoic acid,
(b) 5E-6-(3-(2-cyano-3-tert.butyl-guanidino)phenyl)-6-(3-pyridyl)hex-5-enoic acid,
(c) 5E-6-(3-(2-cyano-3-cyclopentyl-guanidino)phenyl)-6-(3-pyridyl)hex-5-enoic acid,
(d) 5E-6-(3-(2-cyano-3-isopropyl-guanidino)phenyl)-6-(3-pyridyl)hex-5-enoic acid,
(e) 5E-6-(3-(2-cyano-3-(exo-norborn-2-yl)guanidino)phenyl)-6-(3-pyridyl)hex-5-enoic acid,
(f) 5E-6-(3-(2-cyano-3-(2-methylpropyl)guanidino)phenyl)-6-(3-pyridyl)hex-5-enoic acid,
(g) 5E-6-(3-(2-cyano-3-neopentyl-guanidino)phenyl)-6-(3-pyridyl)hex-5-enoic acid,
(h) 5E-6-(3-(2-cyano-3-pentyl-guanidino)phenyl)-6-(3-pyridyl)hex-5-enoic acid,
(i) 5E-6-(3-(2-cyano-3-(3-methylbutyl)guanidino)phenyl)-6-(3-pyridyl)hex-5-enoic acid,
(j) 5E-6-(3-(2,2-dicyano-1-(2-methylpropylamino)ethyleneamino)phenyl)-6-(3-pyridyl)hex-5-enoic acid,
(k) 5E-6-(3-(2,2-dicyano-1-isopropylaminoethyleneamino)phenyl)-6-(3-pyridyl)hex-5-enoic acid,
(l) 5E-6-(3-(2,2-dicyano-1-(3-methylbutylamino)ethyleneamino)phenyl)-6-(3-pyridyl)hex-5-enoic acid,
(m) 5E-6-(3-(2,2-dicyano-1-cyclopentylaminoethyleneamino)phenyl)-6-(3-pyridyl)hex-5-enoic acid,
(n) 5E-6-(3-(2,2-dicyano-1-neopentylaminoethyleneamino)phenyl)-6-(3-pyridyl)hex-5-enoic acid,
(o) 5E-6-(3-(2,2-dicyano-1-cyclopropylaminoethyleneamino)-phenyl)-6-(3-pyridyl)hex-5-enoic acid,
(p) 5E-6-(3-(2,2-dicyano-1-propylamino-ethyleneamino)phenyl)-6-(3-pyridyl)hex-5-enoic acid,
(q) 5E-6-(3-(2,2-dicyano-1-tert.butylaminoethyleneamino)phenyl)-6-(3-pyridyl)hex-5-enoic acid,
(r) 5E-6-(4-(2-cyano-3-cyclohexyl-guanidino)phenyl)-6-(3-pyridyl)hex-5-enoic acid,
(s) 6-(3-(2-cyano-3-tert.butyl-guanidino)phenyl)-6-(3-pyridyl)hexanoic acid
(t) 5E-6-(3-(1-neopentylamino-2-nitro-ethyleneamino)phenyl)-6-(3-pyridyl)hex-5-enoic acid,
(u) E/Z-6-(4-(2-(2-cyano-3-tert.butyl-guanidino)ethyl)phenyl)-6-(3-pyridyl)hex-5-enoic acid,
(v) 5E-6-(3-(3-tert.butyl-2-phenylsulphonylguanidino)phenyl)-6-(3-pyridyl)hex-5-enoic acid,
(w) 5E-6-(3-(2-amidosulphonyl-3-(2-methylpropyl)guanidino)phenyl)-6-(3-pyridyl)hex-5-enoic acid,
(x) 5E-6-(3-(2-carbamoyl-2-cyano-1-(2-methylpropylamino)ethyleneamino)phenyl)-6-(3-pyridyl)hex-5-enoic acid and
(y) 4E-1-(5-(3-(2-cyano-3-cyclopentyl-guanidino)phenyl)-5-(3-pyridyl)-pent-4-enyl)tetrazole,
the cis- and trans-isomers thereof and the salts thereof.

6. 5E-6-(3-(2,2-Dicyano-1-cyclopentylaminoethyleneamino)phenyl)-6-(3-pyridyl)hex-5-enoic acid,
the cis- and trans-isomers thereof and the salts thereof.

7. Physiologically acceptable salts of the compounds according to claims 1 to 6 with inorganic or organic acids or bases.

8. Pharmaceutical compositions containing as active substance a compound according to claims 1 to 6 or a physiologically acceptable salt thereof according to claim 7 optionally together with one or more inert carriers and/or diluents.

9. Pharmaceutical composition according to claim 8, suitable for the treatment and prevention of thromboembolic disorders, for the prophylaxis of arteriosclerosis and for the prevention of metastases and for treating ischaemia, asthma and allergies.

10. Pharmaceutical composition according to claim 8, suitable for treating and preventing diseases in which thromboxane-mediated constriction or PGE₂-mediated dilation of the capillaries are involved, for reducing the severity of transplant rejection, for reducing the renal toxicity of substances such as cyclosporin, for treating kidney diseases and for treating cases of shock.

11. Pharmaceutical composition according to claim 8, 9 or 10, characterised in that it additionally contains as active substance a PDE-inhibitor or a lysing agent.

12. Process for preparing a pharmaceutical composition according to claims 8 to 11, characterised in that a compound according to claims 1 to 6 or a physiologically acceptable addition salt thereof according to claim 7, optionally together with a PDE-inhibitor or a lysing agent, is incorporated into one or more inert conventional carriers and/or diluents by a non-chemical method.

13. Process for preparing the compounds according to claims 1 to 7, characterised in that
a) in order to prepare compounds of general formula I wherein Y denotes an alkoxy, phenoxy, alkylthio or phenylthio group, a compound of general formula 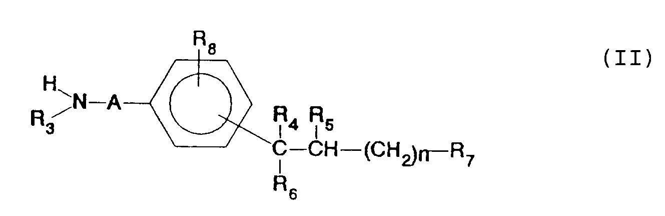 wherein
A, n and R₃ to R₈ are defined as in claims 1 to 6, is reacted with a compound of general formula
(Y')₂C=X (III)
wherein
X is defined as in claims 1 to 6 and
Y' denotes an alkoxy, phenoxy, alkylthio or phenylthio group, or
b) in order to prepare compounds of general formula I wherein Y is an R₁NR₂- group, a compound of general formula 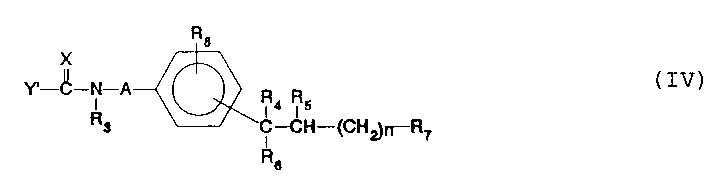 wherein
A, X, n and R₃ to R₈ are defined as in claims 1 to 6 and
Y' denotes an alkoxy, phenoxy, alkylthio or phenylthio group, is reacted with an amine of general formula 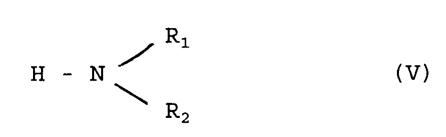 wherein
R₁ and R₂ are defined as in claims 1 to 6, or
c) in order to prepare compounds of general formula I wherein Y denotes an R₁NR₂- group and R₇ denotes a carboxy group, a protective group is cleaved from a compound of general formula 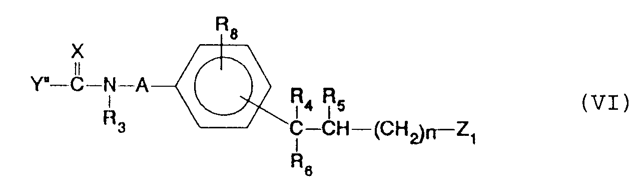 wherein
R₃ to R₆, R₈, A, X and n are defined as in claims 1 to 6, Y'' denotes an R₁NR₂- group wherein R₁ and R₂ are defined as in claims 1 to 6, and
Z₁ denotes a group which may be converted into a carboxy group by hydrolysis, thermolysis or hydrogenolysis, or
d) in order to prepare compounds of general formula I wherein R₄ and R₅ each represent a hydrogen atom, a compound of general formula 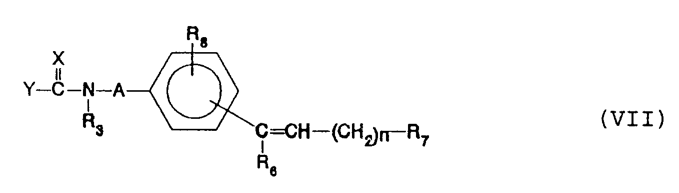 wherein
A, X, Y, n, R₃ and R₆ to R₈ are defined as in claims 1 to 6 is hydrogenated, or
e) in order to prepare a compound of general formula I wherein X has the meanings given for X in claims 1 to 6, with the exception of the cyano-containing groups, and R₇ denotes a tetrazolyl group, a compound of general formula 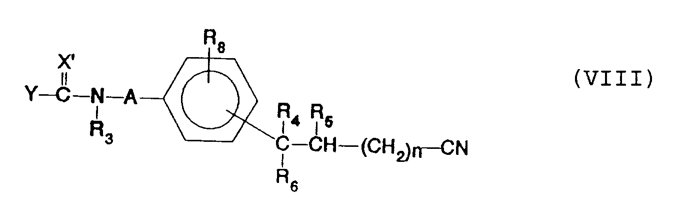 wherein
A, n, Y, R₃ to R₆ and R₈ are defined as in claims 1 to 6 and
X' has the meanings given for X in claims 1 to 6 with the exception of the cyano-containing groups, is reacted with hydrazoic acid or the salts thereof and
if desired a compound of general formula I thus obtained wherein X denotes a group of the formula =N-CN is converted by saponification into a corresponding compound of formula I wherein X denotes a group of the formula =N-CONH₂, or
subsequently, if desired, a compound of formula I thus obtained wherein R₇ denotes a carboxy group is converted by esterification or amidation into a corresponding compound of formula I wherein R₇ denotes a group which can be converted into a carboxy group metabolically in vivo, an aminocarbonyl, alkylaminocarbonyl or dialkylaminocarbonyl group, or
a compound of formula I thus obtained wherein R₇ denotes an aminocarbonyl group, is converted by dehydration into a corresponding compound of formula I wherein R₇ denotes a cyano group, or
a compound of formula I thus obtained wherein R₄ and R₅ together denote a carbon-carbon bond is resolved into the cis- and trans-isomers thereof or
a compound of formula I thus obtained is resolved into the enantiomers thereof or
a compound of formula I thus obtained is converted into the salts thereof, particularly the physiologically acceptable salts thereof with inorganic or organic acids or bases.

## Revendications

1. Dérivés pyridyliques de formule générale 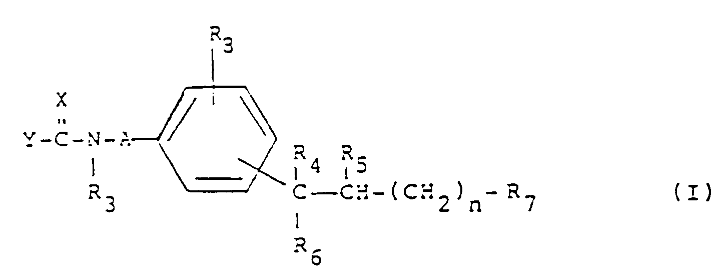 dans laquelle
n représente le nombre 2, 3, 4 ou 5,
A représente une liaison carbone-azote ou un groupe alkylène linéaire de 1 à 4 atomes de carbone éventuellement substitué par un ou deux groupes alkyle,
X représente un groupe nitrométhylène, un groupe cyanométhylène éventuellement substitué par un groupe R₉ ou un groupe de formule =N-R₁₀, R₉ possédant les significations citées dans la suite pour R₇, à l'exception du groupe tétrazolyle, et R₁₀ représentant un groupe cyano, alcanesulfonyle, phénylsulfonyle, phénylalcanesulfonyle, aminosulfonyle, alkylaminosulfonyle, dialkylaminosulfonyle, phénylcarbonyle, aminocarbonyle, alkylaminocarbonyle ou dialkylaminocarbonyle,
Y représente un groupe alcoxy, phénoxy, alkylthio ou phénylthio ou un groupe de formule -R₁NR₂ où R₁ représente un atome d'hydrogène, un groupe alkyle ramifié ou non ramifié de 1 à 10 atomes de carbone, qui peut être substitué en position 2, 3 ou 4 par un groupe hydroxyle, amino, alkylamino ou dialkylamino, un groupe alkyle de 1 à 4 atomes de carbone qui est substitué par un groupe phényle ou pyridyle et qui peut être substitué en outre en position 2, 3 ou 4 par un groupe hydroxyle, un groupe cycloalkyle de 3 ou 4 atomes de carbone, un groupe cycloalkyle de 5 à 8 atomes de carbone dans lequel un pont éthylène peut être remplacé par un groupe o-phénylène, un groupe bicycloalkyle de 6 à 8 atomes de carbone éventuellement substitué par 1, 2 ou 3 groupes alkyle, un groupe adamantyle, alcoxy ou triméthylsilylalkyle,
R₂ représente un atome d'hydrogène ou un groupe alkyle linéaire, ou bien
R₁ et R₂ forment avec l'atome d'azote situé entre eux un groupe alkylénimino cyclique de 4 à 6 atomes de carbone qui peut être substitué par un ou deux groupes alkyle ou par un groupe phényle et dans lequel en outre un pont éthylène peut être remplacé en position 3, 4 par un groupe o-phénylène, un groupe morpholino ou un groupe pipérazino éventuellement substitué en position 4 par un groupe alkyle de 1 à 3 atomes de carbone ou par un groupe phényle,
R₃ représente un atome d'hydrogène ou un groupe alkyle de 1 à 3 atomes de carbone,
R₄ et R₅ représentent chacun un atome d'hydrogène ou ensemble une liaison carbone-carbone,
R₆ représente un groupe pyridyle éventuellement substitué en position 3 ou 4 par un groupe alkyle,
R₇ représente un groupe cyano, tétrazolyle, aminocarbonyle, alkylaminocarbonyle ou dialkylaminocarbonyle, un groupe convertible in vivo par voie métabolique en un groupe carboxyle ou encore, lorsque Y représente un groupe R₁NR₂, un groupe carboxyle,
R₈ représente un atome d'hydrogène, de fluor, de chlore, de brome ou d'iode, un groupe alkyle, alcoxy ou trifluorométhyle,
toutes les parties alkyle et alcoxy citées ci-dessus pouvant contenir un à trois atomes de carbone, sauf indication contraire,
et, sauf indication contraire, tous les noyaux phényle cités ci-dessus pouvant être mono- ou disubstitués par un atome de fluor, de chlore ou de brome, par un groupe alkyle, hydroxyle, alcoxy, phényle, nitro, amino, alkylamino, dialkylamino, alcanoylamino, cyano, carboxyle, alcoxycarbonyle, aminocarbonyle, alkylaminocarbonyle, dialkylaminocarbonyle, trifluoro-méthyle, alcanoyle, aminosulfonyle, alkylaminosulfonyle ou dialkylaminosulfonyle et les substituants pouvant être identiques ou différents, leurs énantiomères, leurs isomères cis et trans, dans la mesure où R₄ et R₅ représentent ensemble une liaison carbone-carbone, et leurs sels.

2. Dérivés pyridyliques de formule générale I selon la revendication 1, dans laquelle
n, A, X, Y, R₃ à R₆ et R₈ sont définis comme dans la revendication 1 et
R₇ représente un groupe tétrazolyle ou un groupe selon les formules
-CO-OR',
-CO-O-(HCR'')-O-CO-R''' et
-CO-O-(HCR'')-O-CO-OR''',
dans lesquelles
R' représente un groupe alkyle linéaire ou ramifié de 1 à 6 atomes de carbone, un groupe cycloalkyle de 5 à 7 atomes de carbone, un groupe benzyle, 1-phényléthyle, 2-phényléthyle, 3-phénylpropyle, méthoxyméthyle ou cinnamyle,
R'' représente un atome d'hydrogène ou un groupe méthyle et
R''' représente un groupe alkyle linéaire ou ramifié de 1 à 6 atomes de carbone, un groupe cycloalkyle de 5 à 7 atomes de carbone, un groupe phényle, benzyle, 1-phényléthyle, 2-phényléthyle ou 3-phénylpropyle,
ou encore, lorsque Y représente un groupe R₁NR₂, un groupe carboxyle,
leurs énantiomères, leurs isomères cis et trans, dans la mesure où R₄ et R₅ représentent ensemble une liaison carbone-carbone, et leurs sels.

3. Dérivés pyridyliques de formule générale I selon la revendication 1, dans laquelle
n représente le nombre 2, 3, 4 ou 5,
A représente une liaison ou un groupe éthylène,
X représente un groupe nitrométhylène, un groupe cyanométhylène éventuellement substitué par un groupe R₉ ou un groupe de formule =N-R₁₀, R₉ possédant les significations citées dans la suite pour R₇, à l'exception du groupe tétrazolyle, et R₁₀ représentant un groupe cyano, un groupe phénylsulfonyle ou alcanesulfonyle,
Y représente un groupe phénoxy ou méthylthio ou un groupe R₁NR₂, R₁ représentant un atome d'hydrogène, un groupe alkyle linéaire ou ramifié de 1 à 8 atomes de carbone, qui peut être substitué en position 2, 3 ou 4 par un groupe hydroxyle ou diméthylamino, un groupe alkyle de 1 à 4 atomes de carbone qui est substitué par un groupe phényle ou pyridyle et qui peut être substitué en outre en position 2, 3 ou 4 par un groupe hydroxyle, un groupe cycloalkyle de 3 à 8 atomes de carbone, un groupe méthoxy, triméthylsilylméthyle, indan-2-yle ou un groupe bicycloheptyle éventuellement substitué par 1, 2 ou 3 groupes alkyle et
R₂ représente un atome d'hydrogène ou un groupe méthyle ou R₁ et R₂ forment avec l'atome d'azote situé entre eux un groupe pipéridino qui peut être substitué par un ou deux groupes méthyle ou par un groupe phényle et dans lequel en outre un pont éthylène peut être remplacé en position 3, 4 par un groupe o-phénylène, un groupe morpholino ou un groupe pipérazino substitué en position 4 par un groupe phényle,
R₃ représente un atome d'hydrogène ou un groupe méthyle,
R₄ et R₅ représentent chacun un atome d'hydrogène ou ensemble une liaison carbone-carbone supplémentaire,
R₆ représente un groupe 3-pyridyle ou 4-pyridyle,
R₇ représente un groupe cyano, tétrazolyle, alcoxycarbonyle, aminocarbonyle, alkylaminocarbonyle ou dialkylaminocarbonyle ayant dans chaque cas 1 à 3 atomes de carbone dans les parties alcoxy et alkyle, ou encore, lorsque Y représente un groupe R₁NR₂, un groupe carboxyle,
R₈ représente un atome d'hydrogène, de fluor, de chlore ou de brome, un groupe alkyle, alcoxy ou trifluorométhyle, toutes les parties alkyle et alcoxy citée ci-dessus pouvant contenir un à trois atomes de carbone, sauf indication contraire, leurs énantiomères, leurs isomères cis et trans, dans la mesure où R₄ et R₅ représentent ensemble une liaison carbone-carbone, et leurs sels.

4. Dérivés pyridyliques de formule générale I selon la revendication 1, dans laquelle
n représente le nombre 3,
A représente une liaison ou un groupe éthylène,
X représente un groupe de formule =N-R₁₀, R₁₀ représentant un groupe cyano ou phénylsulfonyle, ou un groupe dicyanométhylène,
Y représente un groupe R₁NR₂, R₁ représentant un groupe alkyle linéaire ou ramifié de 1 à 8 atomes de carbone, un groupe cycloalkyle de 3 à 8 atomes de carbone ou un groupe exo-norbornyle-(2) et
R₂ représente un atome d'hydrogène,
R₃ représente un atome d'hydrogène,
R₄ et R₅ représentent chacun un atome d'hydrogène ou ensemble une liaison carbone-carbone,
R₆ représente un groupe 3-pyridyle et
R₇ représente un groupe carboxyle ou alcoxycarbonyle ayant au total 2 à 4 atomes de carbone,
R₈ représente un atome d'hydrogène, de chlore ou de brome, un groupe méthyle ou trifluorométhyle, leurs énantiomères, leurs isomères cis et trans et leurs sels.

5. Composés de formule I selon la revendication 1:
(a) acide 5E-6-(3-(2-cyano-3-cyclopropyl-guanidino)phényl)-6-(3-pyridyl)hex-5-énoïque,
(b) acide 5E-6-(3-(2-cyano-3-tert.butyl-guanidino)phényl)-6-(3-pyridyl)hex-5-énoïque,
(c) acide 5E-6-(3-(2-cyano-3-cyclopentyl-guanidino)phényl)-6-(3-pyridyl)hex-5-énoïque,
(d) acide 5E-6-(3-(2-cyano-3-isopropyl-guanidino)phényl)-6-(3-pyridyl)hex-5-énoïque,
(e) acide 5E-6-(3-(2-cyano-3-(exo-norborn-2-yl)guanidino)phényl)-6-(3-pyridyl)hex-5-énoïque,
(f) acide 5E-6-(3-(2-cyano-3-(2-méthylpropyl)guanidino)phényl)-6-(3-pyridyl)hex-5-énoïque,
(g) acide 5E-6-(3-(2-cyano-3-néopentyl-guanidino)phényl)-6-(3-pyridyl)hex-5-énoïque,
(h) acide 5E-6-(3-(2-cyano-3-pentyl-guanidino)phényl)-6-(3-pyridyl)hex-5-énoïque,
(i) acide 5E-6-(3-(2-cyano-3-(3-méthylbutyl)guanidino)phényl)-6-(3-pyridyl)hex-5-énoïque,
(j) acide 5E-6-(3-(2,2-dicyano-1-(2-méthylpropylamino)éthylénamino)phényl)-6-(3-pyridyl)hex-5-énoïque,
(k) acide 5E-6-(3-(2,2-dicyano-1-isopropylaminoéthylénamino)phényl)-6-(3-pyridyl)hex-5-énoïque,
(l) acide 5E-6-(3-(2,2-dicyano-1-(3-méthylbutylamino)éthylénamino)phényl)-6-(3-pyridyl)hex-5-énoïque,
(m) acide 5E-6-(3-(2,2-dicyano-1-cyclopentylaminoéthylénamino)phényl)-6-(3-pyridyl)hex-5-énoïque,
(n) acide 5E-6-(3-(2,2-dicyano-1-néopentylaminoéthylénamino)phényl)-6-(3-pyridyl)hex-5-énoïque,
(o) acide 5E-6-(3-(2,2-dicyano-1-cyclopropylaminoéthylénamino)phényl)-6-(3-pyridyl)hex-5-énoïque,
(p) acide 5E-6-(3-(2,2-dicyano-1-propylamino-éthylénamino)phényl)-6-(3-pyridyl)hex-5-énoïque et
(q) acide 5E-6-(3-(2,2-dicyano-1-tert.butylaminoéthylénamino)phényl)-6-(3-pyridyl)hex-5-énoïque,
(r) acide 5E-6-(4-(2-cyano-3-cyclohexyl-guanidino)phényl)-6-(3-pyridyl)hex-5-énoïque,
(s) acide 6-(3-(2-cyano-3-tert.butyl-guanidino)phényl)-6-(3-pyridyl)hexanoïque,
(t) acide 5E-6-(3-(1-néopentylamino-2-nitro-éthylénamino)phényl)-6-(3-pyridyl)hex-5-énoïque,
(u) acide E/Z-6-(4-(2-(2-cyano-3-tert.butyl-quanidino)éthyl)phényl)-6-(3-pyridyl)hex-5-énoïque,
(v) acide 5E-6-(3-(3-tert.butyl-2-phénylsulfonyl-guanidino)phényl)-6-(3-pyridyl)hex-5-énoïque,
(w) acide 5E-6-(3-(2-amidosulfonyl-3-(2-méthylpropyl)guanidino)phényl)-6-(3-pyridyl)hex-5-énoïque,
(x) acide 5E-6-(3-(2-carbamoyl-2-cyano-1-(2-méthylpropylamino)éthylénamino)phényl)-6-(3-pyridyl)hex-5-énoïque et
(y) 4E-1-(5-(3-(2-cyano-3-cyclopentyl-guanidino)phényl)-5-(3-pyridyl)-4-pentényl)tétrazole, leurs isomères cis et trans et leurs sels.

6. Acide 5E-6-(3-(2,2-dicyano-1-cyclopentylaminoéthylénamino)phényl)-6-(3-pyridyl)hex-5-énoïque, ses isomères cis et trans et ses sels.

7. Sels physiologiquement acceptables des composés selon les revendications 1 à 6 avec des acides ou des bases inorganiques ou organiques.

8. Médicament contenant comme principe actif un composé selon les revendications 1 à 6 ou son sel physiologiquement acceptable selon la revendication 7 et éventuellement un ou plusieurs véhicules et/ou diluants inertes.

9. Médicament selon la revendication 8, convenant pour le traitement et pour la prophylaxie des maladies thromboemboliques, pour la prophylaxie de l'artériosclérose et pour la prophylaxie des métastases ainsi que pour le traitement de l'ischémie, de l'asthme et des allergies.

10. Médicament selon la revendication 8, convenant pour le traitement et pour la prophylaxie des maladies dans lesquelles une constriction des capillaires provoquée par le thromboxane ou une dilatation des capillaires provoquée par PGE₂ joue un rôle, pour réduire le degré de gravité d'un rejet de greffe, pour réduire la toxicité rénale de substances comme la cyclosporine, pour le traitement des maladies rénales et pour le traitement des états de choc.

11. Médicament selon la revendication 8, 9 ou 10, caractérisé en ce que celui-ci contient en outre comme principe actif un inhibiteur de PDE ou un agent de lyse.

12. Procédé de préparation d'un médicament selon les revendications 8 à 11, caractérisé en ce que, par voie non chimique, un composé selon les revendications 1 à 6 ou son sel d'addition physiologiquement acceptable selon la revendication 7, éventuellement en combinaison avec un inhibiteur de PDE ou un agent de lyse, est incorporé dans un ou plusieurs véhicules et/ou diluants inertes habituels.

13. Procédé de préparation des composés selon les revendications 1 à 7, caractérisé en ce que
(a) pour la préparation de composés de formule générale I dans laquelle Y représente un groupe alcoxy, phénoxy, alkylthio ou phénylthio, un composé de formule générale 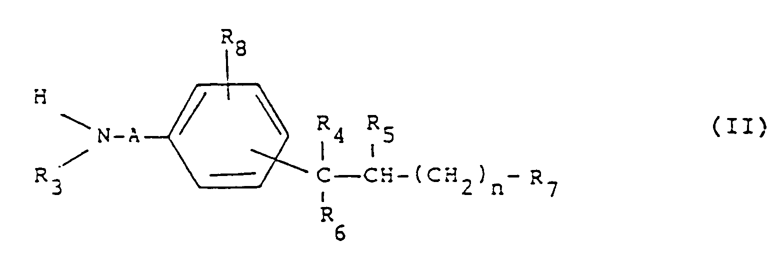 dans laquelle
A, n et R₃ à R₈ sont définis comme dans les revendications 1 à 6, est mis à réagir avec un composé de formule générale
(Y')₂C=X (III)
dans laquelle
X est défini comme dans les revendications 1 à 6 et
Y' représente un groupe alcoxy, phénoxy, alkylthio ou phénylthio, ou bien
b) pour la préparation de composés de formule générale I dans laquelle Y représente un groupe R₁NR₂, un composé de formule générale 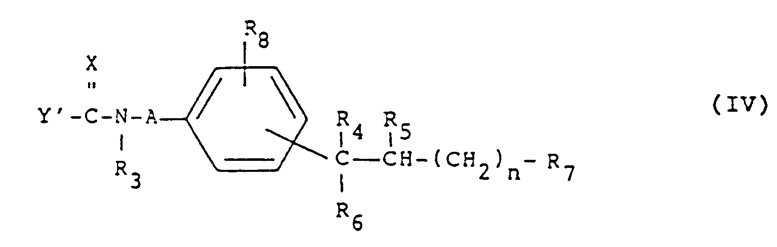 dans laquelle
A, X, n et R₃ à R₈ sont définis comme dans les revendications 1 à 6, et
Y' représente un groupe alcoxy, phénoxy, alkylthio ou phénylthio, est mis à réagir avec une amine de formule générale 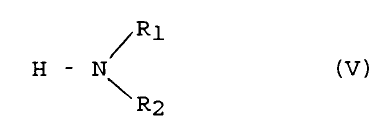 dans laquelle
R₁ et R₂ sont définis comme dans les revendications 1 à 6, ou bien
c) pour la préparation de composés de formule générale I dans laquelle Y représente un groupe R₁NR₂ et R₇ représente un groupe carboxyle, un reste protecteur d'un composé de formule générale 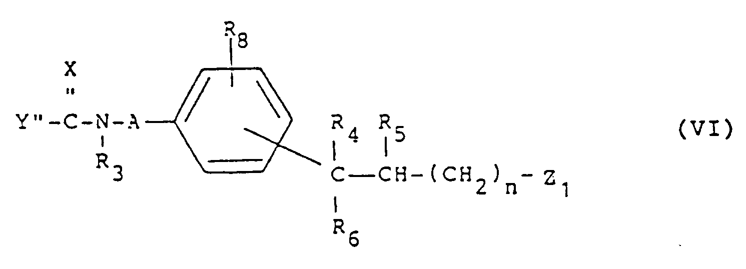 dans laquelle
R₃ à R₆, R₈, A, X et n sont définis comme dans les revendications 1 à 6,
Y'' représente un groupe R₁NR₂, R₁ et R₂ étant définis comme dans les revendications 1 à 6, et
Z₁ représente un groupe convertible en un groupe carboxyle par hydrolyse, thermolyse ou hydrogénolyse, est clivé ou bien
d) pour la préparation de composés de formule générale I dans laquelle R₄ et R₅ représentent chacun un atome d'hydrogène, un composé de formule générale 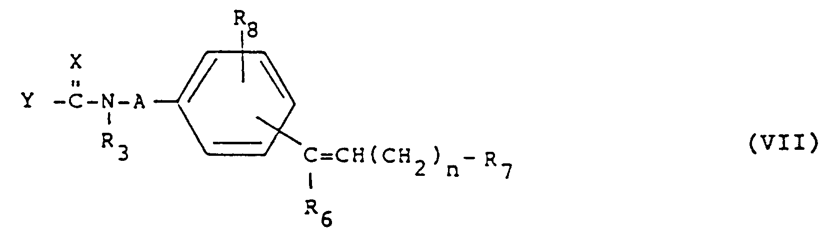 dans laquelle
A, X, Y, n, R₃ et R₆ à R₈ sont définis comme dans les revendications 1 à 6 est hydrogéné ou bien
e) pour la préparation d'un composé de formule générale I dans laquelle X possède les significations indiquées pour X dans les revendications 1 à 6 à l'exception des restes contenant un groupe cyano et R₇ représente un groupe tétrazolyle, un composé de formule générale 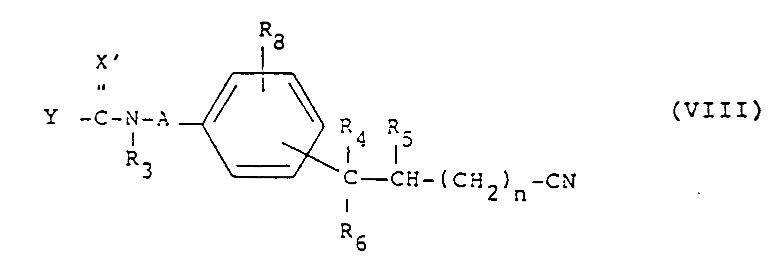 dans laquelle
A, n, Y, R₃ à R₆ et R₈ sont définis comme dans les revendications 1 à 6 et
X' possède les significations indiquées pour X dans les revendications 1 à 6 à l'exception des restes contenant un groupe cyano, est mis à réagir avec l'acide azothydrique ou ses sels et
si on le souhaite, un composé ainsi obtenu de formule générale I dans laquelle X représente un groupe de formule =N-CN, est converti par saponification en un composé correspondant de formule I dans laquelle X représente un groupe de formule =N-CONH₂ ou bien
si on le souhaite, un composé ainsi obtenu de formule I dans laquelle R₇ représente un groupe carboxyle est ensuite converti par estérification ou amidification en un composé correspondant de formule I dans laquelle R₇ représente un groupe convertible in vivo par voie métabolique en un groupe carboxyle, un groupe aminocarbonyle, alkylaminocarbonyle ou dialkylaminocarbonyle, ou bien
un composé ainsi obtenu de formule I dans laquelle R₇ représente un groupe aminocarbonyle est converti par déshydratation en un composé correspondant de formule I dans laquelle R₇ représente un groupe cyano, ou bien
un composé ainsi obtenu de formule I dans laquelle R₄ et R₅ représentent ensemble une liaison carbone-carbone est résolu en ses isomères cis et trans ou bien
un composé ainsi obtenu de formule I est résolu en ses énantiomères ou bien
un composé ainsi obtenu de formule I est converti en ses sels, en particulier en ses sels physiologiquement acceptables avec des acides ou des bases inorganiques ou organiques.
